# EUROPEAN PATENT SPECIFICATION

(11) **EP 4 164 594 B1**
(45) Date of publication and mention of the grant of the patent: **19.11.2025**
(21) Application number: 21730969.9
(22) Date of filing: 14.06.2021
(51) Int. Cl.: A61K 9/00, A61K 9/08, A61K 38/00

(54) **ANTIBODY FORMULATION DILUENT**
VERDÜNNUNGSMITTEL FÜR ANTIKÖRPERFORMULIERUNG
DILUANT DE FORMULATION D'ANTICORPS

(30) Priority: 12.06.2020 EP 20179607
(43) Date of publication of application: 19.04.2023
(73) Proprietor: IGI Therapeutics SA, 2300 La Chaux-de-Fonds (CH)
(72) Inventor: DUBEY, Sachin, 2300 La Chaux-de-Fonds (CH); GASSIAT, Brice, 2300 La Chaux-de-Fonds (CH)
(74) Representative: Paparelli, Laura
(86) International application number: PCT/EP2021/065933
(87) International publication number: WO 2021/250275

(56) References cited:
- WO-A1-2013/163519
- WO-A1-2018/204907
- WO-A1-2019/126133
- WO-A1-97/04801

## Description

### TECHNICAL FIELD

The present invention relates to a diluent for diluting a drug product, which comprises a multispecific hetero-dimeric immunoglobulin, to obtain a drug product ready for administration to a patient by infusion. In particular the diluent of the present invention is such that the loss of antibody material is minimized.

### BACKGROUND

The pharmaceutical formulation of a therapeutic product commonly comprises, besides an active pharmaceutical ingredient (API) with therapeutic properties, also inactive ingredients, or excipients that contribute to the stability, bioavailability and effectiveness of the API.

Example of excipients include solvents, diluents, buffering agents, pH-adjusting agents, surfactants, preservatives, tonicifying agents, antioxidants etc. Appropriate excipients are chosen based on the drug product presentation, route of administration and administration dosage, as well as based on their effect on the stability of the API.

Biological molecules, like proteins, e.g. antibodies, are nowadays developed as therapeutics to treat a variety of diseases spanning from cancer to autoimmune diseases. Therapeutic antibodies are normally stored in adequate formulations that keep the antibody physically and chemically stable at different temperatures and for periods of various months. Formulations comprising monospecific monoclonal antibodies, such as anti-CD22 antibodies and anti-CD200 antibodies, are known, see WO2013163519 and WO2019126133 respectively. Antibody formulations may be of different kinds, for instance liquid or lyophilized, in the latter case, the lyophilized formulation needs to be reconstituted for instance using a diluent such as bacteriostatic water for injection (BWFI), as in WO9704801. Considering the low concentrations at which certain therapeutic antibodies may need to be administrated, it is important to make sure that the drug product has the appropriate formulation and that it is diluted with diluents that would maintain the protein stable and at the same time limit their adsorption on the surfaces of the containers used for storage and for administration, such as vials, syringes, infusion systems (bags, lines, filters etc.). In certain cases, for instance for BiTE^{®} bispecific antibodies, the pharmaceutical compositions that allows high protein recovery from administration containers, comprises a preservative, such as benzyl alcohol, which has the function of inhibiting microbial growth (WO2018204907). Nevertheless, despite the benefit of preservatives in avoiding microbial contamination, it has been shown that they may cause physical instability of protein-based drugs due to protein structural alterations and aggregation (Correlating the Effects of Antimicrobial Preservatives on Conformational Stability, Aggregation Propensity, and Backbone Flexibility of an IgG1 mAb. Jayant Arora et al. Journal of Pharmaceutical Sciences 106 (2017) 1508-1518). Moreover, additional studies have shown the toxicity of certain preservatives to human neurons, also in relation to their concentrations (The relative toxicity of compounds used as preservatives in vaccines and biologics. David A. Geier et al. Med Sci Monit, 2010; 16(5): SR21-27).

Therefore, for the production of therapeutic proteins, such as antibodies, the development of diluents that assure stability and a high recovery of the therapeutic antibodies so as to administer to patients the necessary amount of a stable therapeutic drug, remains still a challenge.

### DESCRIPTION

The invention is set out in the appended set of claims.

As used herein, the following terms have the following meanings: "a", "an", and "the" as used herein refers to both singular and plural unless the context clearly dictates otherwise. Unless otherwise defined, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of cell and tissue culture, molecular biology, and protein and oligo- or polynucleotide chemistry, laboratory procedures and techniques of analytical chemistry, synthetic organic chemistry, and medicinal and pharmaceutical chemistry described herein are those well-known and commonly used in the art.

Temperatures above 0°C are expressed in the present invention either by preceding the temperature value by "+" or not, e.g. 25°C and +25°C can be used interchangeably according to the present invention.

According to the present invention the loss of antibody material is measured by measuring the antibody concentration before and after incubation in an infusion system. The antibody concentration is measured herein by ELISA. The term "ELISA" refers to an enzyme-linked immunosorbent assay as described herein or as otherwise known in the art. Traditional ELISA assays typically involve use of primary or secondary antibodies that are linked (conjugated) to an enzyme, which acts on a substrate to produce a detectable signal (e.g., production of a colored product) to indicate the presence of antigen or another analyte. As used herein, the term "ELISA" also encompasses use of non-enzymatic reporters such as fluorogenic, electrochemiluminescent, or real-time PCR reporters that generate quantifiable signals. It will be appreciated that the term "ELISA" encompasses a number of variations including but not limited to "direct", "indirect", "sandwich", "competitive", and "reverse" ELISA.

In an ELISA assay, generally an amount of antigen is immobilized to a solid surface, and then a specific antibody that recognizes the antigen is added onto the surface so as to allow the formation of an antigen-antibody complex. In the direct ELISA a labelled primary antibody (e.g. an antibody-enzyme conjugated) is used to detect the antigen, while in the indirect ELISA the antigen is bound by a primary antibody which then is detected by a labelled secondary antibody. A sandwich ELISA (also known as Capture ELISA) refers to immobilizing a capture antibody (specific for the antigen) onto a solid support followed by addition of an amount of antigen. The bound antigen is then detected by a second antibody (i.e., detection antibody) which recognizes a region on the antigen that is different from that of the capture antibody. The captured antigen is detected by the detection antibody which can be covalently linked to an enzyme, or can itself be detected by addition of a secondary antibody-enzyme conjugated. Direct, indirect and sandwich ELISA assays can be adapted to be performed in a competitive format. In the competitive ELISA the concentration of an antigen or antibody in a sample is measured as a variation of an expected signal output from a labelled antibody or antigen, respectively, with known concentration. For example, a competitive ELISA may be run by immobilizing the capture antibody specific for the antigen onto a surface and incubating it with a sample containing the target antigen and a known amount of enzyme-labelled target antigen. The more antigen is present in the sample, the less conjugated antigen will bind to the capture antibody. When the enzyme substrate is added, the signal produced is inversely proportional to the amount of antigen present in the sample. Differently from the techniques above, the reverse ELISA leaves the antigens suspended in a fluid. In particular, antibodies and antigens are incubated together, the incubated fluid is moved to a scavenger container to remove unbound antibody, and the remaining antibody and antigen complexes are measured via flow cytometry.

Other equivalent techniques to measure the loss of antibody material at low concentration include but are not limited to mesoscale discovery, and mass spectrometry analysis.

According to the present invention the terms "drug product" relates to a product containing an Active Pharmaceutical Ingredient (API), also known as Drug Substance (DS), in the finished dosage form ready for administration to a patient. For instance, a drug product can be obtained by the purification of a DS, such as a protein like an antibody or an antibody fragment, followed by the addition of excipients to the drug substance to obtain a stable pharmaceutical formulation, which can be liquid, or lyophilized or reconstituted. The drug product can be a "diluted" if a diluent is added to be ready for administration, for instance via an infusion system.

The "physical stability" of a protein in a pharmaceutical formulation is retained if it shows no signs of aggregation, precipitation and/or denaturation upon visual examination of color and/or clarity, or as measured by UV light scattering or by size exclusion chromatography. The "chemical stability" of a protein can be assessed by detecting and quantifying chemically altered forms of the protein. Chemical alteration may involve size modification (e.g. clipping) which can be evaluated using size exclusion chromatography, SDS-PAGE and/or matrix assisted laser desorption ionization/time-of- flight mass spectrometry (MALDI/TOF MS), for example. Other types of chemical alteration include charge alteration (e.g. occurring as a result of deamidation) which can be evaluated by ion exchange chromatography, for example.

A "liquid" formulation is one that has been prepared in a liquid format. Such a formulation may be suitable for direct administration to a subject or, alternatively, can be packaged for storage either in a liquid form, in a frozen state or in a dried form (e.g. lyophilized) for later reconstitution into a liquid form or other forms suitable for administration to a subject.

A "lyophilized" formulation is one that has been prepared by freeze-drying a liquid or pre- lyophilization formulation. Freeze-drying may be performed by freezing the formulation and then subliming ice from the frozen content at a temperature suitable for primary drying. Under this condition the product temperature is below the collapse temperature of the formulation. A secondary drying stage may then be carried out, which produces a suitable lyophilized cake.

A "reconstituted" formulation is one that has been prepared by dissolving a lyophilized protein formulation in a reconstitution media such that the protein is dispersed in the reconstituted formulation. The reconstituted formulation should be suitable for administration (e.g. parenteral administration) to a subject to be treated with the protein of interest.

Suitable "reconstitution media" useful for the preparation of a reconstituted formulation include ones which are pharmaceutically acceptable (safe and non-toxic for administration to a human). Examples of suitable reconstitution media include sterile water for injection (SWFI), bacteriostatic water for injection (BWFI). Water for injection (WFI), a pH buffered solution e.g. phosphate-buffered saline (PBS), sterile saline solution, Ringer's solution or dextrose solution and the buffer used to prepare the pharmaceutical formulation.

The drug product ready for administration of the present invention is ready for administration to a patient via one or more routes of administration using one or more of a variety of methods known in the art.

A "patient" for the purposes of the present invention includes both humans and other animals, preferably mammals and most preferably humans. Thus, the antibodies of the present invention have both human therapy and veterinary applications.

As will be appreciated by the skilled artisan, the route and/or mode of administration will vary depending upon the desired results. Preferred routes of administration include intravenous, intramuscular, intradermal, intraperitoneal, subcutaneous, spinal or other parenteral routes of administration, for example by injection or infusion. More preferred routes of administration are intravenous or subcutaneous. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, an antibody of the invention can be administered via a non- parenteral route, such as a topical, epidermal or mucosal route of administration, for example, intranasally, orally, vaginally, rectally, sublingually or topically.

In a particular embodiment the drug product ready for administration of the present invention is administrated by infusion, e.g. intravenously or subcutaneously, via an infusion system or infusion set comprising one or more elements selected from the group comprising an infusion bag, an infusion set, a line, a filter, a needle, a drip chamber, a syringe, a pump, a stopcock, a valve, a cannula, a tube, a cap , a spike, a coil, a chamber, a connector, a port, a Luer lock.

According to the present invention, the diluent is such that it is minimized loss of antibody material in the containers used for storage and/or for administration, such as vials, syringes, tubes, Falcon tubes, infusion systems comprising one or more elements selected from the group comprising an infusion bag, an infusion set, a line, a filter, a needle, a drip chamber, a syringe, a pump, a stopcock, a valve, a cannula, a tube, a cap, a spike, a coil, a chamber, a connector, a port, a Luer lock, wherein such containers can be made of different materials, including plastic and glass,. In particular according to the present invention, the drug product ready for administration is administered to a patient by infusion and the diluent is such that loss of antibody material in the infusion system is minimized. More specifically, the loss of antibody material is less than about 50%, or less than about 40%, or less than about 30%, or less than about 29%, or about 28%, or about 27%, or about 26%, or about 25%, or about 24%, or about 23%, or about 22%, or about 21%, or about 20%, about 19%, or about 18%, or about 17%, or about 16%, or about 15%, or about 14%, or about 13%, or about 12%, or about 11%, or about 10%, about 9%, or about 8%, or about 7%, or about 6%, or about 5%, or about 4%, or about 3%, or about 2%, or about 1%. Preferably the loss of antibody material is less than about 30%, more preferably less than about 20%, even more preferably less than about 10%, most preferably less than 5%.

According to the present invention, the diluent comprises a buffer and one or more stabilizing or tonicity agents.

The term "buffer" as used herein refers to a buffered solution that resists changes in pH by the action of its acid-base conjugate components. A buffer may have a pH in the range from about 5.0 to about 8.0; for instance pH selected from the group comprising pH equal to about 5, about 5.5, about 6, about 6.5, about 6.9, about 7, about 7.4, about 7.5, about 8. The diluent of the present invention has a pH between 5.5 and 7.5.

Examples of buffers that can control the pH in this range include citric acid, acetate (e.g. sodium acetate), succinate (such as sodium succinate), gluconate, amino acids, such as histidine (e.g. histidine-HCl), citrate, phosphate, citrate phosphate, Tris, other organic acid buffer, their salts and combinations of buffers. The buffer comprised in the diluent of the present invention is selected from the group comprising Histidine, Tris, Citrate, Phosphate and Citrate phosphate. In one embodiment of the present invention the buffer is present within the drug product ready for administration at concentration equal to or less than about 30 mM. In certain embodiments the concentration of the buffer is comprised between about 1 mM and about 30 mM; preferably the buffer is present within the drug product ready for administration at concentration selected from the group comprising about 1 mM, about 3 mM, about 5 mM, about 7 mM, about 10 mM, about 15 mM, about 20 mM, about 25 mM, and about 30 mM. In a preferred embodiment the buffer is present within the drug product ready for administration of the present invention at a concentration of about 25 mM. In another preferred embodiment the buffer is present within the drug product ready for administration of the present invention at a concentration of about 1 mM. The present invention also includes a buffer with a concentration at any intermediate value of the above said values.

Examples of stabilizing or tonicity agents include: sodium acetate, sodium bicarbonate, sodium carbonate, sodium chloride (NaCl), potassium acetate, potassium bicarbonate, potassium carbonate, potassium chloride, calcium chloride (CaCl2), glutamate, sugars such as sucrose, glucose and trehalose, polyols such as mannitol, maltitol, sorbitol, xylitol, erythritol, and isomalt, polyethylene glycol, such as PEG400, Ethylenediaminetetraacetic acid (EDTA), amino acids such as histidine (e.g. histidine-HCl), arginine (e.g. arginine hydrochloride) and glycine, methionine, proline, lysine (e.g. lysine-HCl), glutamic acid, glutamine, cysteine, amines, glutathione, cyclodextrin, such as such as Hydroxypropyl β-cyclodextrin (HPBCD), Hydroxypropyl-sulfobutyl β-cyclodextrin (HPSBCD), Sulfobutylether β-cyclodextrin (SBECD), β-cyclodextrin (BetaCD), α-cyclodextrin (Alpha CD) and γ-cyclodextrin (Gamm CD) and surfactants. Non limiting examples of a typical surfactant include: non- ionic surfactants (HLB 6 to 18) such as sorbitan fatty acid esters (e.g. sorbitan monocaprylate, sorbitan monolaurate, sorbitan monopalmitate), glycerine fatty acid esters (e.g. glycerine monocaprylate, glycerine monomyristate, glycerine monostearate), poly glycerine fatty acid esters (e.g. decaglyceryl monostearate, decaglyceryl distearate, decaglyceryl monolinoleate), polyoxyethylene sorbitan fatty acid esters (e.g. polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, polyoxyethylene sorbitan tristearate), polyoxyethylene sorbitol fatty acid esters (e.g. polyoxyethylene sorbitol tetrastearate, polyoxyethylene sorbitol tetraoleate), polyoxyethylene glycerine fatty acid esters (e.g. polyoxyethylene glyceryl monostearate), polyethylene glycol fatty acid esters (e.g. polyethylene glycol distearate), polyoxyethylene alkyl ethers (e.g. polyoxyethylene lauryl ether), polyoxy ethylene polyoxypropylene alkyl ethers (e.g. polyoxyethylene polyoxypropylene glycol ether, polyoxyethylene polyoxypropylene propyl ether, polyoxyethylene polyoxypropylene cetyl ether), polyoxyethylene alkylphenyl ethers (e.g. polyoxyethylene nonylphenyl ether), polyoxyethylene hydrogenated castor oils (e.g. polyoxyethylene castor oil, polyoxyethylene hydrogenated castor oil), polyoxyethylene beeswax derivatives (e.g. polyoxyethylene sorbitol beeswax), polyoxyethylene lanolin derivatives (e.g. polyoxyethylene lanolin), and polyoxyethylene fatty acid amides (e.g. polyoxyethylene stearyl amide); anionic surfactants such as Cio-Cis alkyl sulfates salts (e.g. sodium cetyl sulfate, sodium lauryl sulfate, sodium oleyl sulfate), polyoxyethylene Cio-Cis alkyl ether sulfates salts with an average of 2 - 4 moles of ethylene oxide (e.g. sodium polyoxyethylene lauryl sulfate), and Cs-Cis alkyl sulfosuccmate ester salts (e.g. sodium lauryl sulfosuccmate ester); natural surfactants such as lecithin, glycerophospho lipid, sphingophospho lipids (e.g. sphingomyelin) and sucrose esters of C 12-C 18f atty acids; Poloxamers such as Poloxamer 188, Poloxamer 407, Poloxamer 124, Poloxamer 237, Poloxamer 338, polyvinylpyrrolidone, salts and combinations of the above cited components. Preferably, the surfactant is selected from polyoxyethylene sorbitan fatty acid esters. Preferably, the surfactant is selected from polyoxyethylene sorbitan fatty acid esters. Particularly preferably the surfactant is Polysorbate 20, 21, 40, 60, 65, 80, 81 and 85, most preferably Polysorbate 80. Polysorbate 80 is also known by the brand name Tween 80^{™} (ICI Americas, Inc.).

The stabilizing or tonicity agent present in the diluent of the present invention is selected from the group comprising polyols such as Polysorbate 20, Polysorbate 40, Polysorbate 80, amino acids such as histidine, arginine, glycine, methionine, proline, lysine, salts such as sodium chloride.. In particular, Arginine or Arginine HCl is present within the drug product ready for administration at a concentration between about 50 mM and about 800 mM, preferably at a concentration selected from the group comprising about 50 mM, about 100 mM, about 150 mM, about 200 mM, about 250 mM, about 300 mM, about 350 mM, about 400 mM, about 450 mM, about 500 mM, about 550 mM, about 600 mM, about 650 mM, about 700 mM, abour 750 mM and about 800 mM, more preferably the concentration of Arginine or Arginine HCl is about 100 mM or about 500 mM; Lysine or Lysine HCl present within the drug product ready for administration at a concentration between about 20 mM and about 800 mM, preferably at a concentration selected from the group comprising about 20 mM, about 25 mM, about 30 mM, about 35 mM, about 40 mM, about 45 mM, about 50 mM, about 100 mM, about 150 mM, about 200 mM, about 250 mM, about 300 mM, about 350 mM, about 400 mM, about 450 mM, about 500 mM, about 550 mM, about 600 mM, about 650 mM, about 700 mM, abour 750 mM and about 800 mM, more preferably the concentration of Lysine HCl is about 500 mM or about 62 mM; NaCl is present within the drug product ready for administration at a concentration between about 20 mM and about 400 mM, preferably at a concentration selected from the group comprising about 20 mM, about 25 mM, about 30 mM, about 35 mM, about 40 mM, about 45 mM, about 50 mM, about 100 mM, about 150 mM, about 200 mM, about 250 mM, about 300 mM, about 350 mM, about 400 mM, more preferably the concentration of NaCl is about 154 mM; Polysorbate 80 is present within said drug product ready for administration at a concentration comprised between about 0.001% (w/v) and about 0.1%, preferably at a concentration selected from the group comprising about 0.001% (w/v); about 0.002% (w/v), about 0.004% (w/v), about 0.006% (w/v), about 0.008% (w/v), about 0.01% (w/v), about 0.02% (w/v), about 0.04% (w/v), about 0.05% (w/v), about 0.06% (w/v), about 0.08% (w/v), about 0.1% (w/v), more preferably at a concentration of about 0.004% (w/v), or about 0.04% (w/v), about 0.05% (w/v).The present invention also includes stabilizing or tonicity agents with a concentration at any intermediate value of the above said values.

In one embodiment, the diluent according to the present invention comprises a buffer selected from the group comprising Histidine, Tris and Phosphate, present within said diluent at a concentration of about 25 mM, and stabilizing or tonicity agents comprising Arginine, present within said diluent at a concentration of about 100 mM, and Polysorbate 80, present within said diluent at a concentration of about 0.05% (w/v) or at a concentration of about 0.004% (w/v), and the diluent has a pH of about 7 or of about 7.4.

In another embodiment of the present invention, the diluent of the current invention comprises Citrate buffer, present within said diluent at a concentration of about 1.1 mM, and stabilizing or tonicity agents comprising Lysine-HCl, present within said diluent at a concentration of about 62 mM, sodium chloride, present within said diluent at a concentration of about 154 mM, and Polysorbate 80, present within said diluent at a concentration of about 0.004% (w/v), and the diluent has a pH from about 6.6 to about 7. Specifically, a diluent of the present invention comprises citric acid present within said diluent at a concentration of about 2.1 g/L, NaCl present within said diluent at a concentration of about 9.0 g/L, Lysine HCl present within said diluent at a concentration of about 91.0 g/L and Polysorbate 80, present within said diluent at a concentration of about 0.004% (w/v), and the diluent has a pH from about 6.6 to about 7, specifically a pH selected from the group comprising about 6.6, about 6.7, about 6.8, about 6.9, about 7.

In other embodiments the diluent of the current invention comprises a buffer selected from the group comprising Histidine, Phosphate, Citrate and Citrate-Phosphate, present within said diluent at a concentration of about 25 mM, and stabilizing or tonicity agents comprising Arginine-HCl or Lysine-HCl, present within said diluent at a concentration of about 500 mM, and Polysorbate 80, present within said diluent at a concentration of about 0.04% (w/v), and wherein said diluent has a pH selected from the group comprising about 6, about 6.5 and about 7.

In a preferred embodiment, the diluent disclosed herein comprises Phosphate buffer present within said diluent at a concentration of about 25 mM, a stabilizing or tonicity agents consisting of Arginine-HCl present within said diluent at a concentration of about 500 mM, and Polysorbate 80, present within said diluent at a concentration of about 0.04% (w/v), and the diluent has a pH of about 6.5. In another preferred embodiment, the diluent disclosed herein comprises Citrate-Phosphate buffer present within said diluent at a concentration of about 25 mM, a stabilizing or tonicity agents consisting of Lysine-HCl present within said diluent at a concentration of about 500 mM, and Polysorbate 80, present within said diluent at a concentration of about 0.04% (w/v), and the diluent has a pH of about 6.0. In a more preferred embodiment, the diluent disclosed herein comprises Phosphate buffer present within said diluent at a concentration of about 25 mM, a stabilizing or tonicity agents consisting of Arginine-HCl present within said diluent at a concentration of about 500 mM, and Polysorbate 80, present within said diluent at a concentration of about 0.04% (w/v), and the diluent has a pH of about 6.

In certain more specific embodiments, the diluent of the present invention comprises a buffer selected from the group comprising Phosphate, Citrate and Citrate-Phosphate, present within said diluent at a concentration of about 25 mM, and stabilizing or tonicity agents comprising Arginine-HCl or Lysine-HCl, present within said diluent at a concentration of about 500 mM, and Polysorbate 80, present within said diluent at a concentration of about 0.04% (w/v), and said diluent has a pH of about 6.5 or of about 7 and it is stable at about 5°C and/or about 25°C and/or at about 40°C for at least 3 months.

In other more specific embodiments, the diluent of the present invention comprises a buffer selected from Phosphate or Citrate-Phosphate, present within said diluent at a concentration of about 25 mM, and stabilizing or tonicity agents comprising Arginine-HCl or Lysine-HCl, present within said diluent at a concentration of about 500 mM, and Polysorbate 80, present within said diluent at a concentration of about 0.04% (w/v), and the diluent has a pH of about 6 or of about 6.5 and it is stable at about 5°C for at least 29 months, at about 25°C for at least 12 months, and at about 40°C for at least 3 months.

In a particularly specific embodiment, the diluent disclosed inhere comprises Phosphate buffer present within said diluent at a concentration of about 25 mM, a stabilizing or tonicity agents consisting of Arginine-HCl present within said diluent at a concentration of about 500 mM, and Polysorbate 80, present within said diluent at a concentration of about 0.04% (w/v) and the diluent has a pH of about 6 and it is stable at about 5°C for at least 29 months, at about 25°C for at least 12 months, and at about 40°C for at least 3 months.

In one aspect of the present invention, an isotonic solution selected from the group comprising saline solution, dextrose solution, and combination of saline and dextrose solution is mixed with the diluent disclosed herein at a ratio selected from the group comprising about 1:2, about 1:5, about 1:10, about 1:15, about 1:20, preferably at a ratio of about 1:10.

The drug product ready for administration according to the present invention comprises a multispecific antibody or an antibody fragment thereof.

The term "antibody" or "immunoglobulin" are herein used interchangeably and referred to whole antibodies and any antigen binding fragments or single chains thereof. An "antibody" refers to a glycoprotein comprising at least two heavy (H) chains and two light (L) chains inter-connected by disulfide bonds, or an antigen binding fragment thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR) with are hypervariable in sequence and/or involved in antigen recognition and/or usually form structurally defined loops, interspersed with regions that are more conserved, termed framework regions (FR or FW). Each VH and VL is composed of three CDRs and four FWs, arranged from amino- terminus to carboxy- terminus in the following order: FW1, CDR1, FW2, CDR2, FW3, CDR3 and FW4. The amino acid sequences of FW1, FW2, FW3, and FW4 all together constitute the "non-CDR region" or "non-extended CDR region" of VH or VL as referred to herein. Antibodies are grouped into classes, also referred to as isotypes, as determined genetically by the constant region. Human constant light chains are classified as kappa (CK) and lambda (CX) light chains. Heavy chains are classified as mu (µ), delta (δ), gamma (γ), alpha (a), or epsilon (ε), and define the antibody's isotype as IgM, IgD, IgG, IgA, and IgE, respectively. Thus, "isotype" as used herein is meant any of the classes and/or subclasses of immunoglobulins defined by the chemical and antigenic characteristics of their constant regions. The known human immunoglobulin isotypes are IgG1 (IGHG1), IgG2 (IGHG2), IgG3 (IGHG3), IgG4 (IGHG4), IgA1 (IGHA1), IgA2 (IGHA2), IgM (IGHM), IgD (IGHD), and IgE (IGHE). The IgG class is the most commonly used for therapeutic purposes. In humans this class comprises subclasses IgG1, IgG2, IgG3 and IgG4.

Antibody fragments include, but are not limited to, (i) the Fab fragment consisting of VL, VH, CL and CHI domains, including Fab' and Fab'-SH, (ii) the Fd fragment consisting of the VH and CHI domains, (iii) the Fv fragment consisting of the VL and VH domains of a single antibody; (iv) the dAb fragment (Ward ES et al., (1989) Nature, 341 : 544-546) which consists of a single variable, (v) F(ab')2 fragments, a bivalent fragment comprising two linked Fab fragments (vi) single chain Fv molecules (scFv), wherein a VH domain and a VL domain are linked by a peptide linker which allows the two domains to associate to form an antigen binding site (Bird RE et al, (1988) Science 242: 423-426; Huston JS et al, (1988) Proc. Natl. Acad. Sci. USA, 85: 5879-83), (vii) bispecific single chain Fv dimers (PCT/US92/09965), (viii) "diabodies" or "triabodies", multivalent or multispecific fragments constructed by gene fusion (Tomlinson I & Hollinger P (2000) Methods Enzymol. 326: 461-79; WO94/13804; Holliger P et al, (1993) Proc. Natl. Acad. Sci. USA, 90: 6444-48) and (ix) scFv genetically fused to the same or a different antibody (Coloma MJ & Morrison SL (1997) Nature Biotechnology, 15(2): 159-163).

In one embodiment of this invention, the disclosed antibody is monoclonal.

The term "monoclonal antibody" as used herein, refers to antibodies that are produced by clone cells all deriving from the same single cell, and that specifically bind the same epitope of the target antigen. When therapeutic antibodies are produced, the generation of monoclonal antibodies is preferred over polyclonal antibodies. In fact, while monoclonal antibodies are produced by cells originating from a single clone and bind all the same epitope, polyclonal antibodies are produced by different immune cells and recognize multiple epitopes of a certain antigen. Monoclonal antibodies assure batch to batch homogeneity, reduced cross-reactivity and high specificity toward the target. Monoclonal antibodies can be expressed, for instance in host cells, using recombinant DNA, giving rise to a recombinant antibody.

In a certain embodiment of this invention the antibody is a recombinant antibody.

The term "recombinant antibody" as used herein, refers to an antibody that has been produced by any process involving the use of recombinant DNA. A recombinant antibody can be engineered in such a way to improve characteristics such as immunogenicity, binding affinity, molecular size, specificity, half-life, and format. Examples of recombinant antibodies include, but are not limited to engineered antibodies, chimeric antibodies, CDRs grafted antibodies (such as humanized antibodies), fully human antibodies, antibody fragments, Fc-engineered antibodies, multispecific antibody (such as bispecific, trispecific, tetraspecific antibody), monomeric and multimeric antibodies (such as homo-dimeric and hetero-dimeric antibodies).

In a more specific embodiment of the antibody of the present invention is a hetero-dimeric antibody, in particular the multispecific antibody or antibody fragment thereof is a hetero-dimeric immunoglobulin or hetero-dimeric fragment thereof comprising a first and a second engineered CH3 domain.

The term "hetero-dimeric antibody" or "hetero-dimeric fragment" or "hetero-dimer" as used herein includes an immunoglobulin molecule or part of comprising at least a first and a second polypeptide, like a first and a second domain, wherein the second polypeptide differs in amino acid sequence from the first polypeptide. Preferably, a hetero-dimeric immunoglobulin comprises two polypeptide chains, wherein the first chain has at least one non identical domain to the second chain, and wherein both chains assemble, i.e. interact through their non-identical domains. Specifically, a hetero-dimeric immunoglobulin comprises at least two domains, wherein the first domain is non identical to the second domain, and wherein both domains assemble, i.e. interact through their protein-protein interfaces. More preferably the hetero-dimeric immunoglobulin, has binding specificity for at least two different ligands, antigens or binding sites, i.e. is bispecific.

The term "multispecific antibody" refers to any antibody having two or more binding sites that can bind two or more different epitopes of the same antigen, or two or more different antigens. Non limiting examples of multispecific antibodies are bispecific antibodies, trispecific antibodies, tetraspecific antibodies. Preferably the antibody of the present invention is bispecific or trispecific or tetraspecific, more preferably it is bispecific or trispecific. In certain embodiments of the present invention the multispecific antibody or fragment thereof is bispecific.

The term "hetero-dimeric immunoglobulin" or "hetero-dimeric fragment" or "hetero-dimer" as used herein includes an immunoglobulin molecule or part of comprising at least a first and a second polypeptide, like a first and a second domain, wherein the second polypeptide differs in amino acid sequence from the first polypeptide. Preferably, a hetero-dimeric immunoglobulin comprises two polypeptide chains, wherein the first chain has at least one non identical domain to the second chain, and wherein both chains assemble, i.e. interact through their non identical domains. Specifically, a hetero-dimeric immunoglobulin comprises at least two domains, wherein the first domain is non identical to the second domain, and wherein both domains assemble, i.e. interact through their protein-protein interfaces. More preferably the hetero-dimeric immunoglobulin, has binding specificity for at least two different ligands, antigens or binding sites, i.e. is bispecific. Hetero-dimeric immunoglobulin as used herein includes but is not limited to full length bispecific antibodies, bispecifc Fab, bispecifc F(ab')2 bispecific scFv fused to an Fc region, diabody fused to an Fc region and domain antibody fused to an Fc region. Preferably, a hetero-dimeric immunoglobulin fragment comprises at least two domains, wherein the first domain is non identical to the second domain, and wherein both domains assemble, i.e. interact through their protein-protein interfaces. More preferably, a hetero-dimeric immunoglobulin fragment comprises at least two engineered domains, wherein the first engineered domain is non identical to the second engineered domain i.e. the first engineered domain differs in amino acid sequence from the second engineered domain, and wherein both engineered domains assemble by interaction through their protein-protein interfaces.

The term "domain" as used herein includes any region of a polypeptide that is responsible for selectively assembling with a protein partner (i.e., another protein (or region of) or another domain) and/or can perform a complete biological function or part of like binding a receptor, or a substrate, independently or within a multidomain entity. Usually a domain as referred to herein is not a hinge region and/or does not contain a hinge region. The domain can exist independently of the rest of a protein chain. A domain forms a compact three-dimensional structure and is independently stable and folded. Domains vary in length from between about 25 amino acids up to 500 amino acids in length. Preferably the domains as used herein vary in length from between about 70 amino acids up to about 120 amino acids in length. The shortest domains such as zinc fingers are stabilized by metal ions or disulfide bridges. Domains often form functional units, such as the calcium-binding EF hand domain of calmodulin. Because they are self-stable, domains can be "swapped" by genetic engineering between one protein and another to make chimeric proteins. Immunoglobulins are made of variable and constant domains belonging to the immunoglobulin superfamily (Williams AF and Barclay AN, Annu Rev Immunol, 6:381-405 (1988); Bork P et al, J Mol Biol, 242(4):309-20 (1994)). Domains, which are included herein are CHI and CH3, specifically naturally occurring CHI and CH3, from IGHA1, IGHA2, IGHD, IGHE, IGHG1, IGHG2, IGHG3, IGHG4, IGHGP and IGHM; CH4, specifically naturally occurring CH4, from IGHE and IGHM; IGKC, IGLCI, IGLC2, IGLC3, IGLC6, and IGLC7, specifically naturally occurring IGKC, IGLCI, IGLC2, IGLC3, IGLC6, and IGLC7. Further domains which are included herein are CH2, specifically naturally occurring CH2, from IGHA1, IGHA2, IGHD, IGHE, IGHG1, IGHG2, IGHG3, IGHG4, IGHGP and IGHM. Preferably unglycosylated CH2 domains are used herein which in their unglycosylated form are homo-dimers as referred herein. The CH2 domains in an unglycosylated Fc fragment approach each other much more closely compared to the CH2 domains in a naturally glycosylated Fc fragment. The crystal structure of the murine unglycosylated IgGI Fc fragment has shown that a fully unglycosylated Fc fragment can adopt a "closed" structure with the distance between the Pro 332 from the CH2 domain of the first unglycosylated immunoglobulin chain and the Pro 332 from the CH2 domain of the second unglycosylated immunoglobulin chain is only 1 1.6 A (Feige MJ et al. , J Mol Biol, 391(3):599-608 (2009)). Further domains which are included herein are VH and VL domains which do not have an engineered protein-protein interface according to the invention, i.e. which are not specifically engineered to modify its naturally occurring protein-protein interface except for backmutations arising out of the humanization process.

The term "engineered immunoglobulin chain" as used herein includes an immunoglobulin chain comprising at least one engineered domain with a protein-protein interface which differs from the parent domain. The term "engineered domain" as used herein includes a domain engineered from a parent domain and a donor domain.

The present invention utilises the BEAT^{®} technology described method (PCT publication No: WO2012/131555), which is based on a unique concept of bio-mimicry that exhibit superior hetero-dimerisation over prior art methods. The BEAT technology is based on an interface exchange between naturally occurring homo or hetero-dimeric immunoglobulin domain pairs to create new hetero-dimers which can be used as building blocks for Fc-based bispecific antibodies.

In one aspect, the present invention provides a hetero-dimeric immunoglobulin or fragment thereof comprising first and second polypeptides comprising an engineered immunoglobulin constant region with a modified CH3 domain having a protein-protein interface, wherein the protein-protein interface of the first polypeptide comprises an amino acid substitution at a position selected from the group consisting of: 3, 5, 7, 20, 22, 26, 27, 79, 81, 84, 84.2, 85.1, 86, 88 and 90 (IMGT^{®} numbering), and wherein the protein-protein interface of the second polypeptide comprises an amino acid substitution at position 84.4 and at a position selected from the group consisting of 3, 5, 7, 20, 22, 26, 27, 79, 81, 84, 84.2, 85.1, 86, 88 and 90 (IMGT^{®} numbering).

In a further embodiment, the multispecific antibody or antibody fragment thereof according to the present invention is a hetero-dimeric immunoglobulin or hetero-dimeric fragment thereof wherein the first and second polypeptides comprise an engineered immunoglobulin constant region with a modified CH3 domain having a protein-protein interface, wherein the protein-protein interface of the first polypeptide comprises an amino acid substitution at position 88 and at a position selected from the group consisting of: 3, 5, 7, 20, 22, 26, 27, 79, 81, 84, 84.2, 85.1, 86 and 90 (IMGT^{®} numbering), and wherein the protein-protein interface of the second polypeptide comprises an amino acid substitution at position 85.1 and/or 86 and at a position selected from the group consisting of 3, 5, 7, 20, 22, 26, 27, 79, 81, 84, 84.2, 84.4, 88 and 90 (IMGT^{®} numbering), wherein the amino acid residue substituted at position 88 in the first engineered immunoglobulin constant region is interacting with the amino acid residue substituted at position 85.1 and/or 86 in the second engineered immunoglobulin constant region, wherein the amino acid position of each group member is indicated according to the IMGT^{®} numbering.

Preferably the amino acid residue which is substituted in the protein-protein interface of the first engineered immunoglobulin constant region at position 88 is 88 W and conservative amino acid substitutions thereof, wherein the amino acid position is indicated according to IMGT^{®} numbering. More preferably, the amino acid residue which is substituted in the protein-protein interface of the first engineered immunoglobulin constant region at position 88 is 88 W and wherein the further amino acid residue substituted in the protein-protein interface of the first engineered immunoglobulin constant region is selected from the group consisting of: 3A, 20V, 20T, 20A, 20N, 20Q, 20E, 20S, 20K, 20W, 22A, 22G, 22T, 22L, 221, 22V, 26R, 26Q, 26T, 26K, 26V, 26S, 26N, 26E, 79Y, 85. IT, 85.1M, 85.1A, 85. IS, 85.1R, 85.1H, 85. IK, 85. IF, 85.1C, 85. IN, 85.1W, 86S, 861, 86T, 86H, 86Q, 86V, 86W, 86Y, 86F and 90N, wherein the amino acid position is indicated according to the IMGT^{®} numbering.

Preferably the amino acid residue which is substituted at position 85 and 86 in the protein- protein interface of the second engineered immunoglobulin constant region is selected from the group consisting of: 85.1 A, 85. IS, 85.1C and 86S and conservative amino acid substitutions thereof (IMGT^{®} numbering). More preferably the amino acid residue which is substituted in the protein-protein interface of the second engineered immunoglobulin constant region is selected from the group consisting of: 85.1 A, 85. IS, 85.1C and 86S and wherein the further amino acid residue substituted in the protein-protein interface of the second engineered immunoglobulin constant region is selected from the group consisting of: 3E, 5A, 7F, 20T, 22V, 26T, 8 ID, 84L, 84.2E, 88R and 90R and conservative amino acid substitutions thereof (IMGT^{®} numbering).

In a preferred embodiment the amino acid residue which is substituted in the protein-protein interface of the first engineered immunoglobulin constant region at position 88 is 88 W and wherein the further amino acid residue substituted in the protein-protein interface of the first engineered immunoglobulin constant region is: 3A, 20K, 22V, 26T, 79Y, 85. IS, 86V and 90N and, wherein the amino acid residues which are substituted in the protein-protein interface of the second engineered immunoglobulin constant region at positions 85.1 and 86 are 85.1 A, 85. IS or 85.1 A and 86S and wherein the further amino acid residue substituted in the protein-protein interface of the second engineered immunoglobulin constant region is: 3E, 5A, 7F, 20T, 22V, 26T, 8 ID, 84L, 84.2E, 84.4Q, 88R and 90R (IMGT^{®} numbering).

In an alternative embodiment, the present invention provides a hetero-dimeric immunoglobulin or fragment thereof, wherein the first and second polypeptides comprise an engineered immunoglobulin constant region with a modified CH3 domain having a protein- protein interface, wherein the protein-protein interface of the first polypeptide comprises an amino acid substitution at position 20, and at a position selected from the group consisting of: 3, 5, 7, 22, 26, 27, 79, 81, 84, 84.2, 85.1, 86, 88 and 90 and, wherein the protein-protein interface of the second polypeptide comprises an amino acid substitution at position 26 and at a position selected from the group consisting of: 3, 22, 27, 79, 81, 84, 85.1, 86, and 88, wherein the amino acid residue substituted at position 20 in the first engineered immunoglobulin constant region is interacting with the amino acid residue substituted at position 26 in the second engineered immunoglobulin constant region, wherein the amino acid position of each group member is indicated according to the IMGT numbering.

Preferably the amino acid residues which are substituted in the protein-protein interface of the first engineered immunoglobulin chain comprise the amino acid residues at positions 20 and 22, and optionally a further amino acid residue at a position selected from the group consisting of: 3, 5, 7, 26, 27, 79, 81, 84, 84.2, 84.4, 85.1, 86, 88 and 90 and, wherein the amino acid residues which are substituted in the protein-protein interface of the second engineered immunoglobulin chain comprise the amino acid residues at positions 26 and at a further position selected from the group consisting of: 3, 5, 7, 20, 22, 27, 79, 81, 84, 84.2, 84.4, 85.1, 86, 88 and 90, wherein the amino acid position of each group member is indicated according to the IMGT^{®} numbering.

Preferably the amino acid residues which are substituted in the protein-protein interface of the first engineered immunoglobulin chain comprise the amino acid residues at positions 20 and 22, and optionally a further amino acid residue at a position selected from the group consisting of: 3, 5, 7, 26, 27, 79, 81, 84, 84.2, 84.4, 85.1, 86, 88 and 90 and, wherein the amino acid residues which are substituted in the protein-protein interface of the second engineered immunoglobulin chain comprise the amino acid residues at positions 26 and 86 and optionally at a further position selected from the group consisting of 3, 5, 7, 20, 22, 27, 79, 81, 84, 84.2, 84.4, 85.1, 88 and 90, wherein the amino acid position of each group member is indicated according to the IMGT^{®} numbering.

In a most preferred embodiment the amino acid residue which is substituted in the protein- protein interface of the first engineered immunoglobulin constant region at position 20 is 20K and wherein the further amino acid residue substituted in the protein-protein interface of the first engineered immunoglobulin constant region is 3 A, 22V, 26T, 79Y, 85. IS, 86V, 88W and 90N and, wherein the amino acid residues which are substituted in the protein-protein interface of the second engineered immunoglobulin constant region at position 26 is 26T and wherein the further amino acid residue substituted in the protein-protein interface of the second engineered immunoglobulin constant region is 3E, 5A, 7F, 20T, 22V, 8 ID, 84L, 84.2E, 84.4Q, 85.1C/S/A, 86S, 88R and 90R (IMGT^{®} numbering).

In one aspect, the multispecific antibody or antibody fragment thereof according to the present invention is a hetero-dimeric immunoglobulin or hetero-dimeric fragment thereof, wherein the hetero-dimeric immunoglobulin or hetero-dimeric fragment thereof comprises a first and a second engineered immunoglobulin chain, wherein the first and second immunoglobulin chains are engineered with substitutions in amino acids in a protein interface that promote dimerization between the first and second immunoglobulin chains, wherein the first engineered immunoglobulin chain and the second engineered immunoglobulin chain are not identical and, wherein the first and second engineered domain each comprise a CH3 domain, wherein the substitutions in the engineered domains are from an equivalent position in a naturally-occurring TCR constant domain hetero-dimer or homo-dimer, wherein the amino acid residues which are substituted in the protein-protein interface of the first and second engineered immunoglobulin chains comprise the following amino acid residues: (20, 22, 26, 79, 85.1, 86, 88, 90) in the first engineered immunoglobulin chain and (3, 5, 7, 20, 22, 26, 81, 84, 84.2, 85.1, 86, 88, 90) in the second engineered immunoglobulin chain; or (7, 20, 22, 27, 79, 81, 84.2, 85.1, 86, 88, 90) in the first engineered immunoglobulin chain, and (3, 5, 20, 22, 26, 27, 81, 84, 85.1, 86, 88) in the second engineered immunoglobulin chain; wherein the amino acid position of each group member is indicated according to the IMGT^{®} numbering.

In a more specific embodiment, the first engineered CH3 domain comprises substitutions at one or more of the following residues 20, 22, 26, 79, 85.1, 86, 88, 90, according to the IMGT numbering and wherein said second engineered CH3 domain comprises substitutions in at least one residue selected from the group 3, 5, 20, 22, 26, 27, 81, 84, 85.1, 86, 88, according to the IMGT numbering, and wherein the hetero-dimeric immunoglobulin or hetero-dimeric fragment heterodimerize through said first and second engineered CH3 domains; more specifically, the first engineered CH3 domain comprises the substitutions of the group consisting of: S20K, T22V, K26T, K79Y, F85.1S, Y86V, K88W, T90N, and the second engineered CH3 domain comprises the substitutions of the group consisting of: Q3E, Y5A, L7F, S20T, T22V, K26T, T81D, V84L, D84.2E, F85.1A, Y86S, K88R, T90R. In a further embodiment, the second engineered CH3 domain also comprises the substitution D84.4Q according to the IMGT numbering.

According to one aspect of the present invention, the multispecific hetero-dimeric immunoglobulin is present within the drug product ready for administration of the current invention at a concentration between about 1 ng/mL and about 5 mg/mL, or about 1 ng/mL and about 4 mg/mL, or about 1 ng/mL and about 2 mg/mL, or about 1 ng/mL and about 1 mg/mL, or about 2 ng/mL and about 5 mg/mL, or about 5 ng/mL and about 5 mg/mL, or about 10 ng/mL and about 5 mg/mL, or about 5 ng/mL and about 4 mg/mL, or about 10 ng/mL and about 2 mg/mL; for instance the concentration of the drug product ready for administration is selected from the group comprising about 1 ng/mL, about 5 ng/mL, about 10 ng/mL, about 50 ng/mL, about 100 ng/mL, about 150 ng/mL, about 200 ng/mL, about 250 ng/mL, about 300 ng/mL, about 350 ng/mL, about 400 ng/mL, about 450 ng/mL, about 500 ng/mL, about 550 ng/mL, about 600 ng/mL, about 650 ng/mL, about 700 ng/mL, about 750 ng/mL, about 800 ng/mL, about 850 ng/mL, about 900 ng/mL, about 950 ng/mL, about 1 mg/mL, about 1.2 mg/mL, about 1.4 mg/mL, about 1.6 mg/mL, about 1.8 mg/mL, about 2 mg/mL, about 2.2 mg/mL, about 2.4 mg/mL, about 2.6 mg/mL, about 2.8 mg/mL, about 3 mg/mL, about 3.2 mg/mL, about 3.4 mg/mL, about 3.6 mg/mL, about 3.8 mg/mL, about 4 mg/mL, about 4.2 mg/mL, about 4.4 mg/mL, about 4.6 mg/mL, about 4.8 mg/mL, about 5 mg/mL. In a more particular embodiment, the antibody or antibody fragment thereof is present within the drug product ready for administration of the current invention at a concentration comprised between about 5 ng/mL and about 20 ng/mL, more in particular the concentration selected from the group comprising about 5 ng/mL, about 8 ng/mL, about 10 ng/mL, about 12 ng/mL, about 15 ng/mL, about 18 ng/mL, about 20 ng/mL. The present invention also includes antibody or antibody fragment thereof present within the disclosed drug product ready for administration at a concentration at any intermediate value of the above said values.

In further aspects of the present invention, the multispecific hetero-dimeric immunoglobulin or hetero-dimeric immunoglobulin thereof of the present invention binds to a first target selected from the group comprising CD3, HER2, CD38, EGFR, CD20, OX40, CD19, CD47, IgE, IL1RAP, BMCA, NKp30, CD16, CD16a and CD16b, a second target different from the first target selected from the group comprising CD3, HER2, CD38, EGFR, CD20, OX40, CD19, CD47, IgE, IL1RAP, BMCA, NKp30, CD16, CD16a and CD16b, a third target different from the first target or different from the first and the second target selected from the group comprising CD3, HER2, CD38, EGFR, CD20, OX40, CD19, CD47, IgE, IL1RAP, BMCA, NKp30, CD16, CD16a and CD16b, and a fourth target different from the first target or different from the first and the second target, or different from the first, the second and the third target selected from the group comprising CD3, HER2, CD38, EGFR, CD20, OX40, CD19, CD47, IgE, IL1RAP, BMCA, NKp30, CD16, CD16a and CD16b.

In other aspects of the present invention, the multispecific hetero-dimeric immunoglobulin or hetero-dimeric immunoglobulin thereof of the present invention binds to a first target selected from the group comprising CD3, HER2, CD38, EGFR, CD20, OX40, CD19, CD47, IgE, IL1RAP, BMCA, NKp30, CD16, CD16a and CD16b, a second target different from the first target selected from the group comprising CD3, HER2, CD38, EGFR, CD20, OX40, CD19, CD47, IgE, IL1RAP, BMCA, NKp30, CD16, CD16a and CD16b, and a third target different from the first target or different from the first and the second target selected from the group comprising CD3, HER2, CD38, EGFR, CD20, OX40, CD19, CD47, IgE, IL1RAP, BMCA, NKp30, CD16, CD16a and CD16b.

In certain aspects of the present invention the multispecific hetero-dimeric immunoglobulin or hetero-dimeric immunoglobulin thereof of the present invention binds to a first target selected from the group comprising CD3, HER2, CD38, EGFR, CD20, OX40, CD19, CD47, IgE, IL1RAP, BMCA, NKp30, CD16, CD16a and CD16b, and a second target different from the first target selected from the group comprising CD3, HER2, CD38, EGFR, CD20, OX40, CD19, CD47, IgE, IL1RAP, BMCA, NKp30, CD16, CD16a and CD16b,.

In a more specific aspect, the multispecific hetero-dimeric antibody or hetero-dimeric antibody fragment thereof binds to CD3 and at least to a second target selected from the group comprising HER2, CD38, EGFR, CD20, OX40, CD19, CD47, IL1RAP, BMCA, NKp30, CD16, CD16a and CD16b,.

In another specific aspect, the multispecific hetero-dimeric antibody or hetero-dimeric antibody fragment thereof binds to HER2 and at least to a second target selected from the group comprising CD3, CD38, EGFR, CD20, OX40, CD19, CD47, IL1RAP, BMCA, NKp30, CD16, CD16a and CD16b,.

In another specific aspect, the multispecific hetero-dimeric antibody or hetero-dimeric antibody fragment thereof binds to CD38 and at least to a second target selected from the group comprising HER2, CD3, EGFR, CD20, OX40, CD19, CD47, IL1RAP, BMCA, NKp30, CD16, CD16a and CD16b,.

In another specific aspect, the multispecific hetero-dimeric antibody or hetero-dimeric antibody fragment thereof binds to EFGR and at least to a second target selected from the group comprising HER2, CD38, CD3, CD20, OX40, CD19, CD47, IL1RAP, BMCA, NKp30, CD16, CD16a and CD16b,.

In another specific aspect, the multispecific hetero-dimeric antibody or hetero-dimeric antibody fragment thereof binds to CD20 and at least to a second target selected from the group comprising HER2, CD38, EGFR, CD3, OX40, CD19, CD47, IL1RAP, BMCA, NKp30, CD16, CD16a and CD16b,.

In another specific aspect, the multispecific hetero-dimeric antibody or hetero-dimeric antibody fragment thereof binds to OX40 and at least to a second target selected from the group comprising HER2, CD38, EGFR, CD20, CD3, CD19, CD47, IL1RAP, BMCA, NKp30, CD16, CD16a and CD16b,.

In another specific aspect, the multispecific hetero-dimeric antibody or hetero-dimeric antibody fragment thereof binds to CD19 and at least to a second target selected from the group comprising HER2, CD38, EGFR, CD20, OX40, CD3, CD47, IL1RAP, BMCA, NKp30, CD16, CD16a and CD16b,.

In another specific aspect, the multispecific hetero-dimeric antibody or hetero-dimeric antibody fragment thereof binds to CD47 and at least to a second target selected from the group comprising HER2, CD38, EGFR, CD20, OX40, CD19, CD3, IL1RAP, BMCA, NKp30, CD16, CD16a and CD16b,.

In another specific aspect, the multispecific hetero-dimeric antibody or hetero-dimeric antibody fragment thereof binds to IL1RAP and at least to a second target selected from the group comprising HER2, CD38, EGFR, CD20, OX40, CD19, CD47, CD3, BMCA, NKp30, CD16, CD16a and CD16b,.

In another specific aspect, the multispecific hetero-dimeric antibody or hetero-dimeric antibody fragment thereof binds to BMCA and at least to a second target selected from the group comprising HER2, CD38, EGFR, CD20, OX40, CD19, CD47, IL1RAP, CD3, NKp30, CD16, CD16a and CD16b,.

In another specific aspect, the multispecific hetero-dimeric antibody or hetero-dimeric antibody fragment thereof binds to NKp30and at least to a second target selected from the group comprising HER2, CD38, EGFR, CD20, OX40, CD19, CD47, IL1RAP, BMCA, CD3, CD16, CD16a and CD16b.

In another specific aspect, the multispecific hetero-dimeric antibody or hetero-dimeric antibody fragment thereof binds to CD16 and at least to a second target selected from the group comprising HER2, CD38, EGFR, CD20, OX40, CD19, CD47, IL1RAP, BMCA, NKp30, CD3, CD16a and CD16b,
In another specific aspect, the multispecific hetero-dimeric antibody or hetero-dimeric antibody fragment thereof binds to CD16a and at least to a second target selected from the group comprising HER2, CD38, EGFR, CD20, OX40, CD19, CD47, IL1RAP, BMCA, NKp30, CD3, CD16 and CD16b,
In another specific aspect, the multispecific hetero-dimeric antibody or hetero-dimeric antibody fragment thereof binds to CD16b and at least to a second target selected from the group comprising HER2, CD38, EGFR, CD20, OX40, CD19, CD47, IL1RAP, BMCA, NKp30, CD3, CD16a and CD16,
In specific embodiments, the multispecific hetero-dimeric antibody or hetero-dimeric antibody fragment thereof binds to CD3 and to a second target selected from the group comprising HER2, CD38, EGFR

For instance the multispecific antibody according to the current invention may be generated by BEAT^{®} technology (WO2012131555).

For instance the present invention provides a drug product comprising a bispecific hetero-dimeric immunoglobulin binding to:
i) CD3 and HER2, comprising the amino acid sequences of SEQ ID NOs: 1, 2 and 3; or the amino acid sequences of SEQ ID NOs: 10, 11 and 12; or the amino acid sequences of SEQ ID NOs: 13, 14 and 15; or the amino acid sequences of SEQ ID NOs: 16, 17 and 12; or the amino acid sequences of SEQ ID NOs: 18, 19 and 20; or the amino acid sequences of SEQ ID NOs: 20, 21 and 22; or the amino acid sequences of SEQ ID NOs: 23, 24 and 15; or
ii) CD3 and CD38, comprising the amino acid sequences of SEQ ID NOs: 7, 8 and 9; or the amino acid sequences of SEQ ID NOs: 25, 14 and 26; or the amino acid sequences of SEQ ID NOs: 27, 28 and 29; or the amino acid sequences of SEQ ID NOs: 30, 31 and 26; or the amino acid sequences of SEQ ID NOs: 32, 33 and 34; or the amino acid sequences of SEQ ID NOs: 24, 35 and 36; or the amino acid sequences of SEQ ID NOs: 24, 29 and 37;or
iii) CD3 and OX40, comprising the amino acid sequences of SEQ ID NOs: 38, 39 ad 14; or the amino acid sequences 40, 41 and 24; or the amino acid sequences 42, 43 and 24; or the amino acid sequences 44, 38 and 24; or
iv) CD3 and EGFR, comprising the amino acid sequences of SEQ ID NOs: 4, 5, and 6; or the amino acid sequences of SEQ ID NOs: 45, 46 and 28; or the amino acid sequences of SEQ ID NOs: 47, 48 and 24; or the amino acid sequences of SEQ ID NOs: 49, 50 and 24; or the amino acid sequences of SEQ ID NOs: 51, 52 and 24; or the amino acid sequences of SEQ ID NOs: 53, 54 and 24; or the amino acid sequences of SEQ ID NOs: 55, 46 and 24; or the amino acid sequences of SEQ ID NOs: 56, 57 and 24; or
v) CD3 and CD20, comprising the amino acid sequences of SEQ ID NOs: 58, 59 and 31; or the amino acid sequences of SEQ ID NOs: 60, 61 and 24; or the amino acid sequences of SEQ ID NOs: 62, 63 and 24 ; or the amino acid sequences of SEQ ID NOs: 64, 59 and 24; or
vi) CD3 and CD19, comprising the amino acid sequences of SEQ ID NOs: 65, 66 and 24; or
vii)CD3 and IgE, comprising the amino acid sequences of SEQ ID NOs: 67, 68 and 24; or the amino acid sequences of SEQ ID NOs: 69, 70 and 24; or the amino acid sequences of SEQ ID NOs: 71, 72 and 24; or the amino acid sequences of SEQ ID NOs: 73, 74 and 24; or the amino acid sequences of SEQ ID NOs: 75, 76 and 24; or the amino acid sequences of SEQ ID NOs: 77, 78 and 24.

In certain preferred embodiments, the bispecific antibody or an antibody fragment thereof binds a first target CD3 and a second target selected from the group comprising HER2, CD38 and EGFR. In one embodiment of this invention, the bispecific antibody, referred to as Ab1, binds the cluster of differentiation 3 (CD3) expressed by T-cells and the human epidermal growth factor receptor 2 (HER2), often overexpressed in cancer cells such as breast, ovarian, bladder, salivary gland, endometrial, pancreatic and non-small-cell lung cancer (NSCLC) cancer cells. Ab1 according to the present invention comprises the amino acid sequences of SEQ ID NOs: 1 to 3. In another embodiment, the monoclonal bispecific antibody is Ab2 (SEQ ID NOs: 4 to 6), which binds to CD3 and EGFR, known to be a target in different types of cancers, including colorectal cancer. In one embodiment, the bispecific antibody provided by the present invention binds to epitopes upon CD3 and CD38 (SEQ ID NOs: 7 to 9). In another embodiment, the monoclonal bispecific antibody is Ab3 designed to simultaneously engage the CD3 molecule on T cells and the CD38 antigen on multiple myeloma cells and thus bridge cytotoxic T cells to multiple myeloma tumor cells, thereby killing the bound target cells. This process is described as redirected killing or lysis.

The present invention also relates to a drug product ready for administration for use in the treatment of a patient in need thereof. According to another aspect of the present invention, there is provided a method of treating a patient in need thereof.

As used herein, the term "subject" is equivalent to the term "patient" and it includes any human or nonhuman animal. The term "nonhuman animal" includes all vertebrates, e.g., mammals and non-mammals, such as nonhuman primates, sheep, dogs, cats, horses, cows, chickens, amphibians, reptiles, etc. Preferably the subject is human.

The term "treatment" or "treating" in the present invention is meant to include therapeutic treatment, as well as prophylactic, or suppressive measures for a disease or disorder. Thus, for example, successful administration of an antibody prior to onset of the disease results in treatment of the disease. As another example, successful administration of an antibody after clinical manifestation of the disease to combat the symptoms of the disease comprises treatment of the disease. "Treatment" and "treating" also encompasses administration of an antibody after the appearance of the disease in order to eradicate the disease. Successful administration of an antibody after onset and after clinical symptoms have developed, with possible abatement of clinical symptoms and perhaps amelioration of the disease, comprises treatment of the disease. Those "in need of treatment" include mammals already having the disease or disorder, as well as those prone to having the disease or disorder, including those in which the disease or disorder is to be prevented.

The antibody of the present invention can be administered at a single or multiple doses. The term "dose" or "dosage" as used in the present invention are interchangeable and indicates an amount of drug substance administered per body weight of a subject or a total dose administered to a subject irrespective to their body weight.

In accordance with one aspect of the present invention, the drug product ready for administrations provided may be administered to individuals. Administration is preferably in a "therapeutically effective amount", this being sufficient to show benefit to a subject. Such benefit may be at least amelioration of at least one symptom. The actual amount administered, and rate and time-course of administration, will depend on the nature and severity of what is being treated. Prescription of treatment, e.g. decisions on dosage etc, is within the responsibility of medical doctors. Appropriate doses of antibody are well known in the art (Ledermann JA et al., (1999) Int J Cancer 47: 659-664; Bagshawe KD et a/., (1991) Antibody, Immunoconjugates and Radiopharmaceuticals, 4: 915-922). The precise dose will depend upon a number of factors, including the size and location of the area to be treated, body weight of the subject, the precise nature of the antibody (e.g. whole antibody or fragment) and any additional therapeutic agents administered before, at the time of or after administration of the antibody.

The present invention also relates a method of manufacturing the diluent of the present invention.

The present invention also discloses an article of manufacture comprising the diluent of the present invention.
Figure 1: Stability of Ab1 in diluents D1 to D10 at T0, and after 1 month at 5°C, 25°C and 40°C, measured by absorption at 280 nm.
Figure 2: Ab1 recovery (ng/mL) for diluents D1 to D10 after 24 hours of incubation in a glass vial and in a polyethylene lined administration set (giving set), first run. Theoretical concentration ("Exp Conc") reported.
Figure 3: Ab1 recovery (ng/mL) for diluents D5, D6, D7, D9 and D10 after 24 hours of incubation in a glass vial and in a polyethylene lined administration set (giving set), second run. Theoretical concentration ("Exp Conc") reported.
Figure 4: Stability of Ab1 in diluent D1 at T0, after three freeze - thaw (FT) cycles from -80°C to 25°C and after three FT cycles from -80°C to 25°C followed by 24h or 48h of incubation at 25°C, or followed by 24h or 48h of incubation at 5°C, measured by absorption at 280 nm.
Figure 5: Stability of Ab1 in diluent D1 at T0, after one, two or three FT cycles from -80°C to 25°C and after three FT cycles from -80°C to 25°C followed by 24h or 48h of incubation at 25°C, or followed by 24h or 48h of incubation at 5°C, measured by absorption at 280 nm
Figure 6: Stability of Ab1 in diluent D1 at T0, after three FT cycles from -20°C to 25°C and after three FT cycles from -20°C to 25°C followed by 24h or 48h of incubation at 25°C, or followed by 24h or 48h of incubation at 5°C, measured by absorption at 280 nm.
Figure 7: Stability of Ab1 in diluent D1 at T0, after one, two or three FT cycles from -20°C to 25°C and after three FT cycles from -20°C to 25°C followed by 24h or 48h of incubation at 25°C, or followed by 24h or 48h of incubation at 5°C, measured by absorption at 280 nm.
Figure 8: Stability of Ab1 in diluent D2 at T0, after one, two or three FT cycles from -80°C to 25°C and after three FT cycles from -80°C to 25°C followed by 24h or 48h of incubation at 25°C, or followed by 24h or 48h of incubation at 5°C, measured by absorption at 280 nm.
Figure 9: Stability of Ab1 in diluent D2 at T0, after one, two or three FT cycles from -20°C to 25°C and after three FT cycles from -20°C to 25°C followed by 24h or 48h of incubation at 25°C, or followed by 24h or 48h of incubation at 5°C, measured by absorption at 280 nm.
Figure 10: Stability of Ab1 in diluent D3 at T0, after one, two or three FT cycles from -80°C to 25°C and after three FT cycles from -80°C to 25°C followed by 24h or 48h of incubation at 25°C, or followed by 24h or 48h of incubation at 5°C, measured by absorption at 280 nm.
Figure 11: Stability of Ab1 in diluent D5 at T0, after one, two or three FT cycles from -80°C to 25°C and after three FT cycles from -80°C to 25°C followed by 24h or 48h of incubation at 25°C, or followed by 24h or 48h of incubation at 5°C, measured by absorption at 280 nm.
Figure 12: Stability of Ab1 in diluent D3 at T0, after one, two or three FT cycles from -20°C to 25°C and after three FT cycles from -20°C to 25°C followed by 24h or 48h of incubation at 25°C, or followed by 24h or 48h of incubation at 5°C, measured by absorption at 280 nm.
Figure 13: Stability of Ab1 in diluent D5 at T0, after one, two or three FT cycles from -20°C to 25°C and after three FT cycles from -20°C to 25°C followed by 24h or 48h of incubation at 25°C, or followed by 24h or 48h of incubation at 5°C, measured by absorption at 280 nm.
Figure 14: Stability of Ab1 in diluent D8 at T0, after one, two or three FT cycles from -80°C to 25°C and after three FT cycles from -80°C to 25°C followed by 24h or 48h of incubation at 25°C, or followed by 24h or 48h of incubation at 5°C, measured by absorption at 280 nm.
Figure 15: Stability of Ab1 in diluent D8 at T0, after one, two or three FT cycles from -20°C to 25°C and after three FT cycles from -20°C to 25°C followed by 24h or 48h of incubation at 25°C, or followed by 24h or 48h of incubation at 5°C, measured by absorption at 280 nm.
Figure 16: Stability of Ab1 in diluent D1 comprising Tween 80-1 (Tween 1), or Tween 80-2 (Tween 2) or Tween 80-3 (Tween 3), at T0, after one FT cycles from -80°C to 25°C, measured by absorption at 280 nm.
Figure 17: Stability of Ab1 in diluent D1 comprising Tween 80-1 (Tween 1), or Tween 80-2 (Tween 2) or Tween 80-3 (Tween 3), at T0 and after one FT cycles from -20°C to 25°C, measured by absorption at 280 nm.
Figure 18: Stability of Ab1 in diluent D1 comprising Tween 80-1 (Tween 1), or Tween 80-2 (Tween 2) or Tween 80-3 (Tween 3), at T0 and after 24h or 1 week (1W) of incubation at 5°C, measured by absorption at 280 nm.
Figure 19: Stability of Ab1 in diluent D1 comprising Tween 80-1 (Tween 1), or Tween 80-2 (Tween 2) or Tween 80-3 (Tween 3), at T0 and after 24h or 1 week of incubation at 25°C in darkness conditions, measured by absorption at 280 nm.
Figure 20: Stability of Ab1 in diluent D1 comprising Tween 80-1 (Tween 1), or Tween 80-2 (Tween 2) or Tween 80-3 (Tween 3), at T0 and after 24h or 1 week of incubation at 25°C in light condition, measured by absorption at 280 nm.
Figure 21: Stability of Ab1 in diluent D1 comprising Tween 80-1 (Tween 1), or Tween 80-2 (Tween 2) or Tween 80-3 (Tween 3), after 1 week of incubation at 5°C in a 100 mL glass vial filled up to the 80% of the volume where stainless-steel needle are placed in the middle ("80% Fill + needles"), measured by absorption at 280 nm.
Figure 22: Stability of Ab1 in diluent D1 comprising Tween 80-1 (Tween 1), or Tween 80-2 (Tween 2) or Tween 80-3 (Tween 3), after 1 week of incubation at 25°C in a 100 mL glass vial filled up to the 80% of the volume where stainless-steel needle are placed in the middle ("80% Fill + needles") in darkness conditions, measured by absorption at 280 nm.
Figure 23: Stability of Ab1 in diluent D1 comprising Tween 80-1 (Tween 1), or Tween 80-2 (Tween 2) or Tween 80-3 (Tween 3), after 1 week of incubation at 25°C in a 100 mL glass vial filled up to the 80% of the volume where stainless-steel needle are placed in the middle ("80% Fill + needles") in light conditions,, measured by absorption at 280 nm.
Figure 24: Stability of Ab1 in diluent D1 comprising Tween 80-1 (Tween 1), or Tween 80-2 (Tween 2) or Tween 80-3 (Tween 3), after 1 week of incubation at 5°C in a 100 mL glass vial fully filled or filled up to 80%, measured by absorption at 280 nm.
Figure 25: Stability of Ab1 in diluent D1 comprising Tween 80-1 (Tween 1), or Tween 80-2 (Tween 2) or Tween 80-3 (Tween 3), after 1 week of incubation at 25°C in a 100 mL glass vial fully filled or filled up to 80%, in darkness conditions, measured by absorption at 280 nm.
Figure 26: Stability of Ab1 in diluent D1 comprising Tween 80-1 (Tween 1), or Tween 80-2 (Tween 2) or Tween 80-3 (Tween 3), after 1 week of incubation at 25°C in a syringe a 100 mL glass vial or filled up to 80%, in light conditions, measured by absorption at 280 nm.
Figure 27: Stability of Ab1 in diluent D1 comprising Tween 80-1 (Tween 1), or Tween 80-2 (Tween 2) or Tween 80-3 (Tween 3), after 1 week of incubation at 25°C in dark conditions and in room light, measured by absorption at 280 nm.
Figure 28: ELISA Quantification results (Trial 1)
Figure 29: ELISA Quantification results (Trial 2)
Figure 30: ELISA Quantification results (Trial 3)

### Example 1: Ab1 stability study in diluents D1 to D10

### Materials and methods

First the stability of Ab1, a CD3xHER2 bispecific antibody, in diluents D1 to D10 (shown in Table 1) under different stress conditions was investigated. Ab1 was present within said diluents at a concentration of 10 ng/ml.

**Table 1. Composition of diluents D1 to D10**

| **Diluent** | **Composition** |
|---|---|
| D1 | 25 mM histidine - 100 mM Arginine - 0.05% (w/v) Polysorbate 80 - pH 7.4 |
| D2 | 25 mM histidine - 100 mM Arginine - 0.05% (w/v) Polysorbate 80 - pH 7.0 |
| D3 | 25 mM Tris - 100 mM Arginine - 0.05% (w/v) Polysorbate 80 - pH 7.4 |
| D4 | 25 mM Tris - 100 mM Arginine - 0.05% (w/v) Polysorbate 80 - pH 7.0 |
| D5 | 25 mM phosphate - 100 mM Arginine - 0.05% (w/v) Polysorbate 80 - pH 7.4 |
| D6 | 25 mM phosphate - 100 mM Arginine - 0.05% (w/v) Polysorbate 80 - pH 7.0 |
| D7 | Citric acid 210 mg/L - NaCl 9.0 g/L - Lysine HCl 9.1 g/L - Polysorbate 80 0.04g/L (0.004%) - 6.6 ≤pH≤ 7 |
| D8 | 25 mM histidine - 100 mM Arginine - 0.004% (w/v) Polysorbate 80 - pH 7.0 |
| D9 | 25 mM Tris - 100 mM Arginine - 0.004% (w/v) Polysorbate 80 - pH 7.0 |
| D10 | 25 mM phosphate - 100 mM Arginine - 0.004% (w/v) Polysorbate 80 - pH 7.0 |

Unstressed samples were directly analyzed at time point T0 upon receiving the material in order to have a control to compare to stressed samples. The investigated stress conditions include:
- Temperature stress: the goal of the temperature stress is to evaluate the impact of each temperature on Ab1 molecular properties. In this case 1 month (1M) of incubation at 5 ± 3°C, 25 ± 2°C and 40 ± 2°C has been investigated.
- Freeze/thaw (FT) stress: FT and hold time studies were performed in different conditions including 1, 2 or 3 cycles of FT from -20°C or from -80°C to 25°C, and 1 or 3 cycles of FT from -20°C or from -80°C to 25°C, followed by incubation at 5°C or at 25°C for 24 or 48 hours.

For these experiments Tween 80 has been used in D1 to D10 as Polysorbate 80. The effect of different types of Polysorbate 80, in particular of different types of Tween 80, on the stability of Ab1 has been investigated, as well as the effect of the contact to metal needles, of a glass vial fill volume and of the light/darkness conditions.

To establish the stability of the molecule in the described conditions the total protein concentration was measured by UV-Visible spectrophotometer using Nanodrop 2000 equipment (Thermo scientific).

### Results and conclusions

### One month stability study

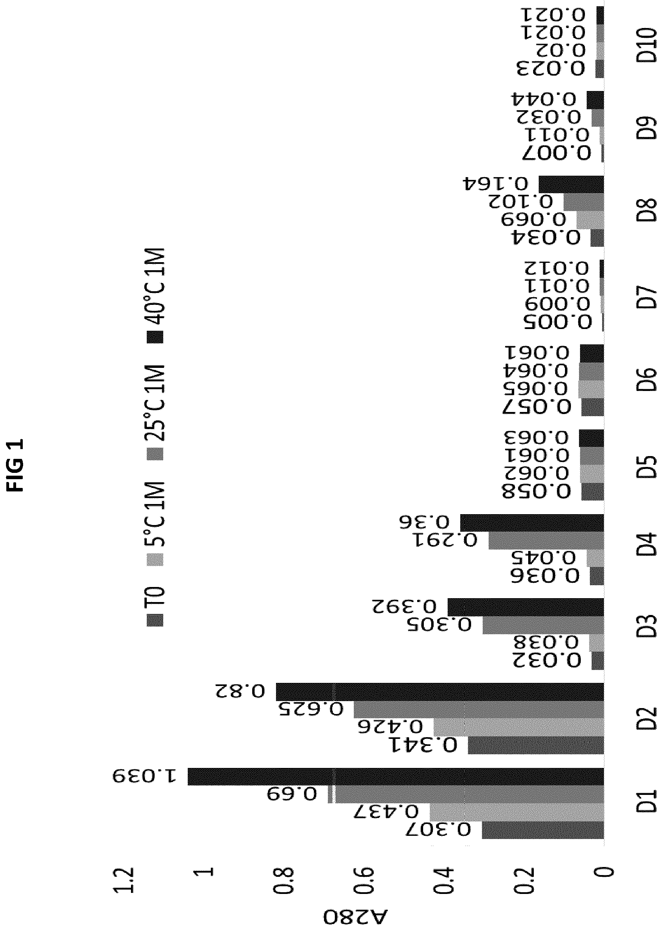

First the stability of Ab1 in diluents D1 to D10 was investigated before storage (T0), and after 1 month of storage at 5°C, at 25°C and at 40°C. From the results obtained by A280 measurement, diluents D5 to D10 resulted to be the most stable, with A280 lower than the limit set by the specification of 0.15 (a measured value of 0.15 is set as a cut off value as ≥0.15 vales start interfering with actual protein content measurement) for all the tested temperatures, except D8 storage at 40°C which resulted to have A280 slightly higher than the specification limit (data shown in Figure 1).

### Recovery study

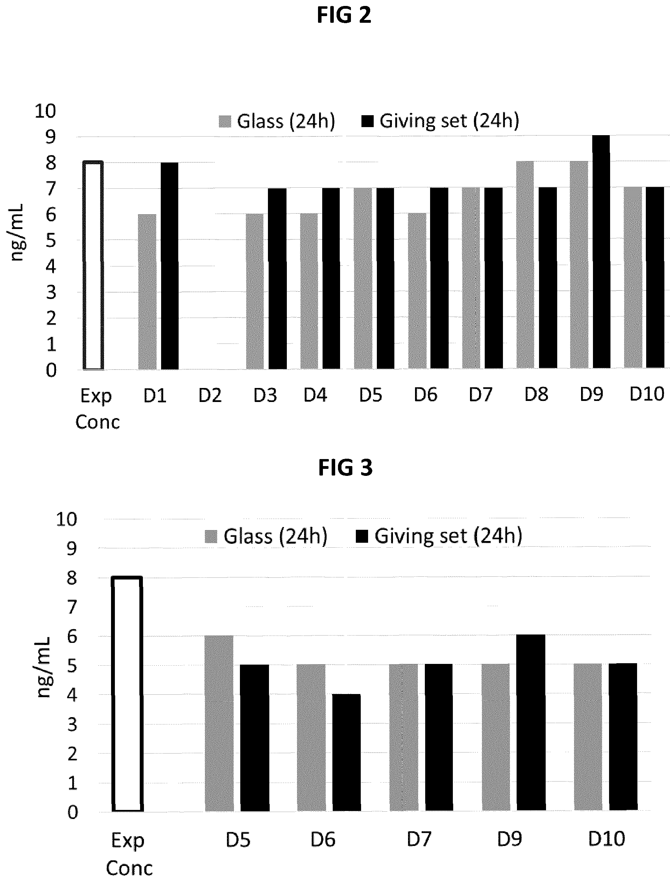

Recovery of Ab1 at dilution 8 ng/mL in diluents D1 to D10 was studied after its storage in a glass vial and in a polyethylene lined administration set for 24 hours. The results, shown in Figure 2 and Figure 3, indicate that that recovery is similar in most of the buffers tested, nevertheless D7 and D10 result most stable among the different buffers. Diluents were considered acceptable if in agreement with the limits of ±40%/50% of the target value at the lowest concentration.

### Freeze-thaw stress

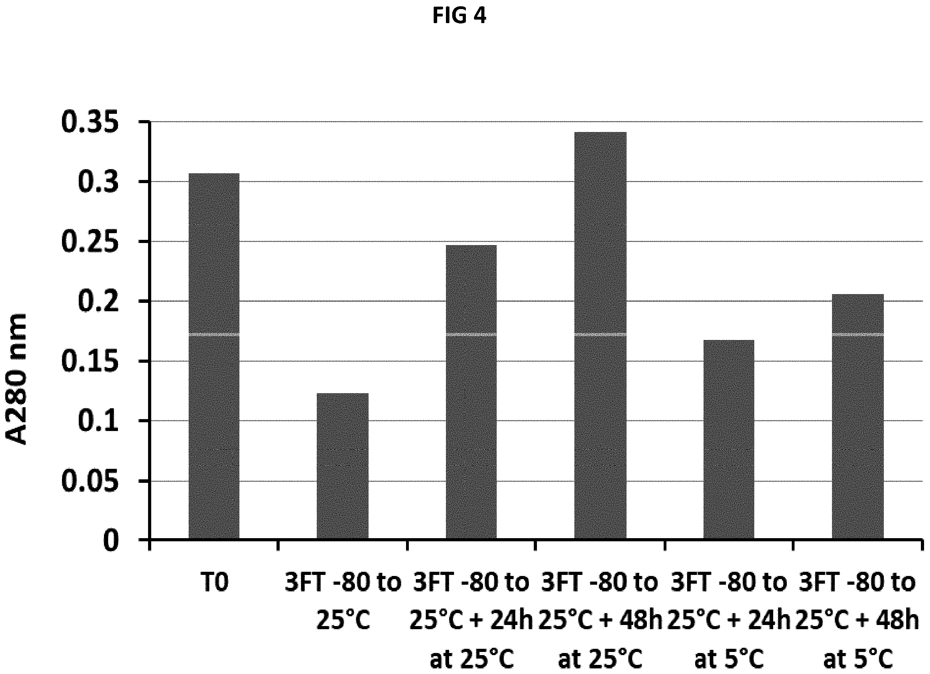

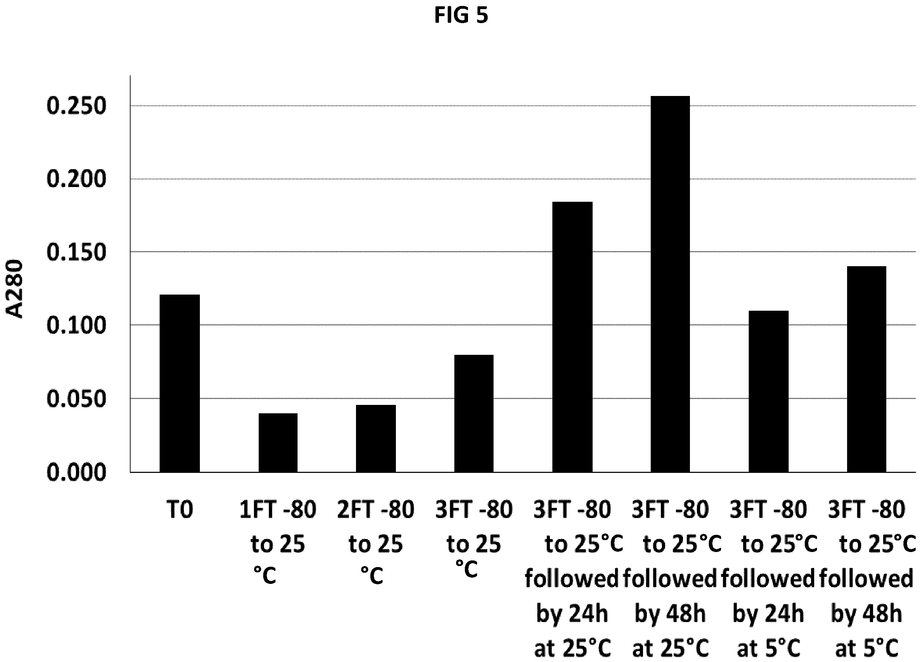

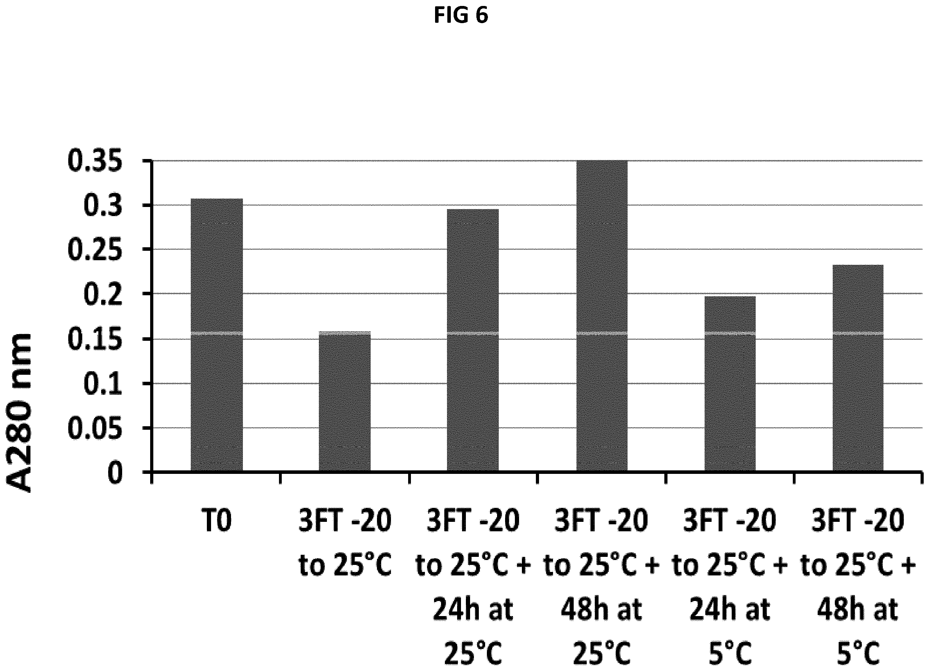

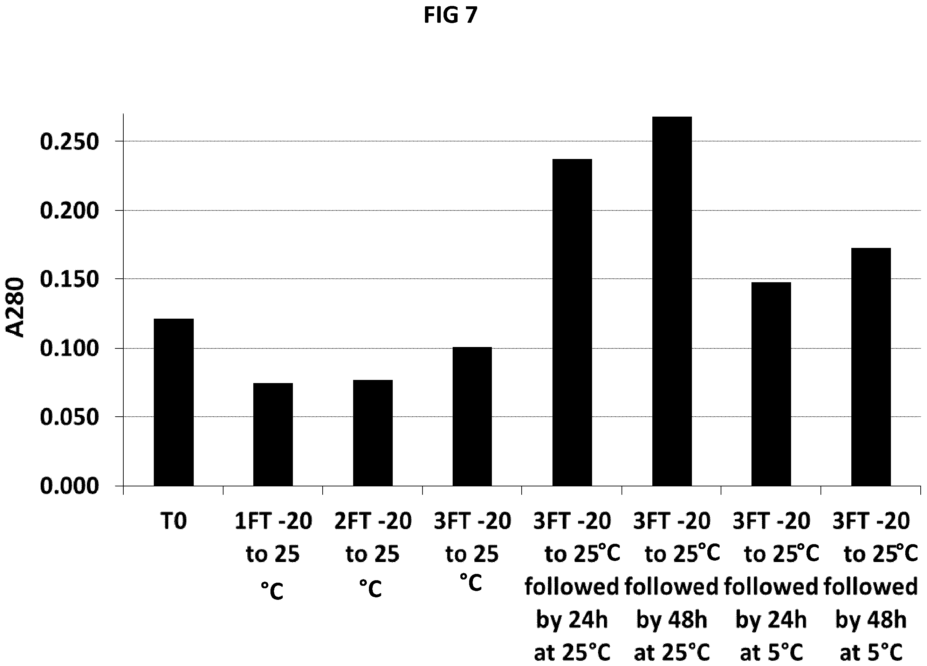

The results of Ab1 stability analysis obtained after application of freeze-thaw (FT) stress are given in Figure 4 to Figure 15. To investigate the effect of FT temperature, FT from -80°C to 25°C and from -20°C to 25°C were performed for diluents D1 and D7. In particular, Figure 4 to Figure 7 show the results obtained for D1. More specifically Figure 4 shows the stability of Ab1 in diluent D1 at T0, after three FT cycles from -80°C to 25°C and after three FT cycles from -80°C to 25°C followed by 24h or 48h of incubation at 25°C, or followed by 24h or 48h of incubation at 5°C, measured by absorption at 280 nm on spectrophotometer. Figure 5 shows the stability of Ab1 in diluent D1 before (T0) and after one, two or three FT cycles from - 80°C to 25°C, as well as after three FT cycles from -80°C to 25°C followed by 24h or 48h of incubation at 25°C or at 5°C, measures by Nanodrop. Figure 6 shows the stability of Ab1 in diluent D1 at T0, after three FT cycles from -20°C to 25°C and after three FT cycles from -20°C to 25°C followed by 24h or 48h of incubation at 25°C, or followed by 24h or 48h of incubation at 5°C, measured by absorption at 280 nm on spectrophotometer. Figure 7 show the stability of Ab1 in diluent D1 at T0, and after one, two or three FT cycles from -20°C to 25°C, as well as after three FT cycles from -20°C to 25°C followed by 24h or 48h of incubation at 25°C or at 5°C, measures by A280 spectroscopy or Nanodrop.

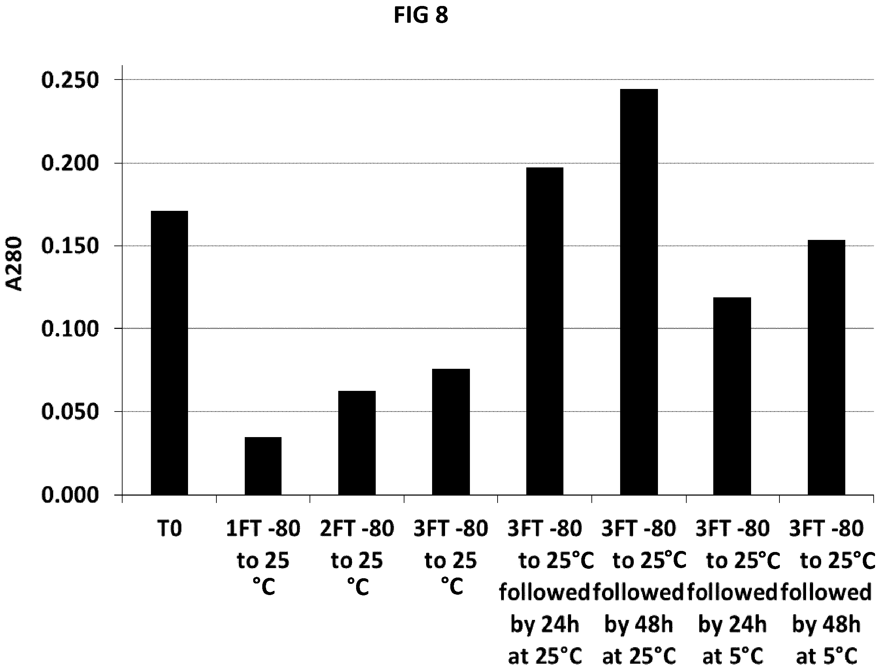

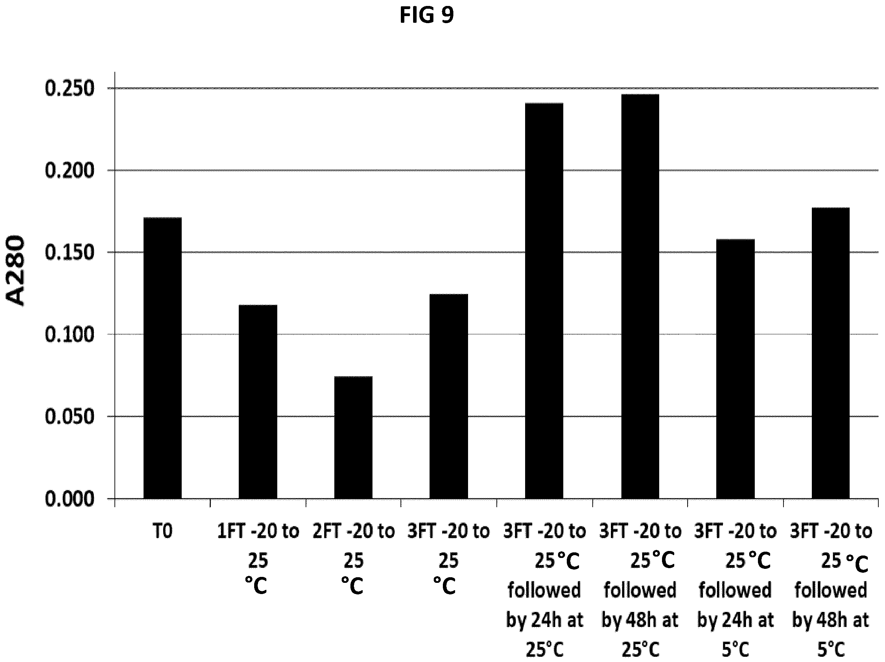

Next, the effect of pH on the stability of Ab1 was investigated by FT studies of D2. In Figure 8 and Figure 9 it is shown the stability of Ab1 in diluent D2 at T0 and after one, two or three FT cycles from -80°C to 25°C and from -20°C to 25°C, respectively, as well as after three FT cycles from -80°C (or -20°C) to 25°C followed by 24h or 48h of incubation at 25°C, or followed by 24h or 48h of incubation at 5°C.

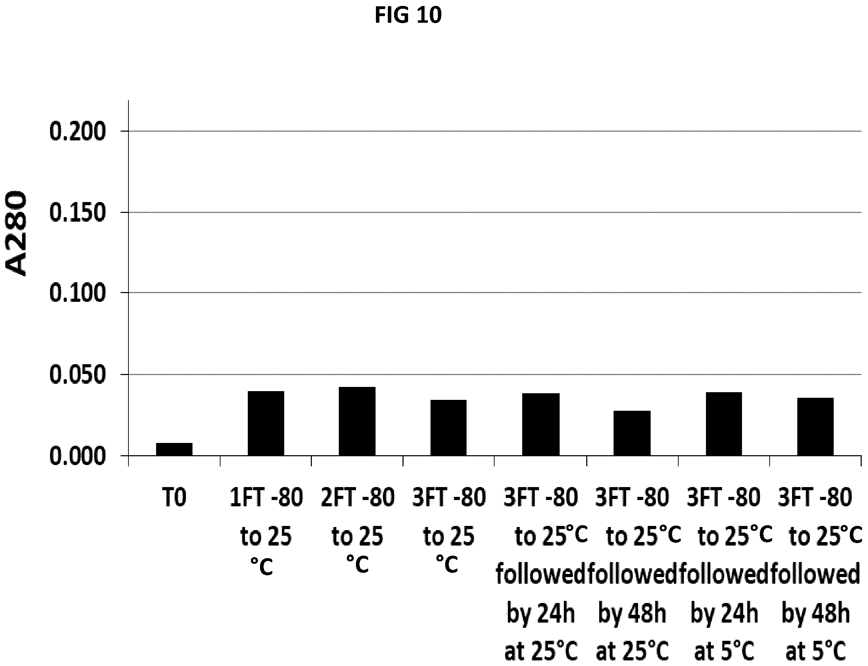

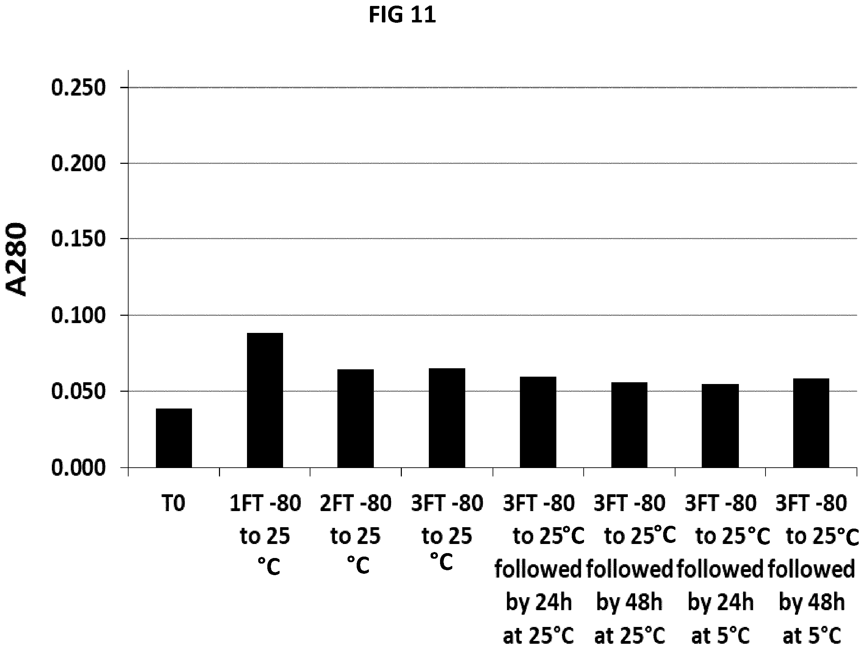

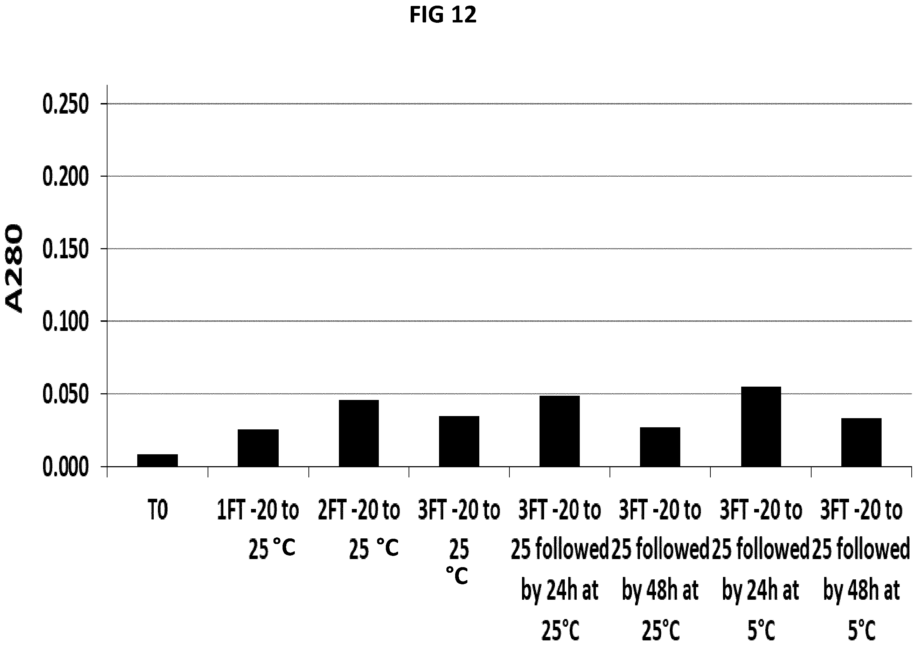

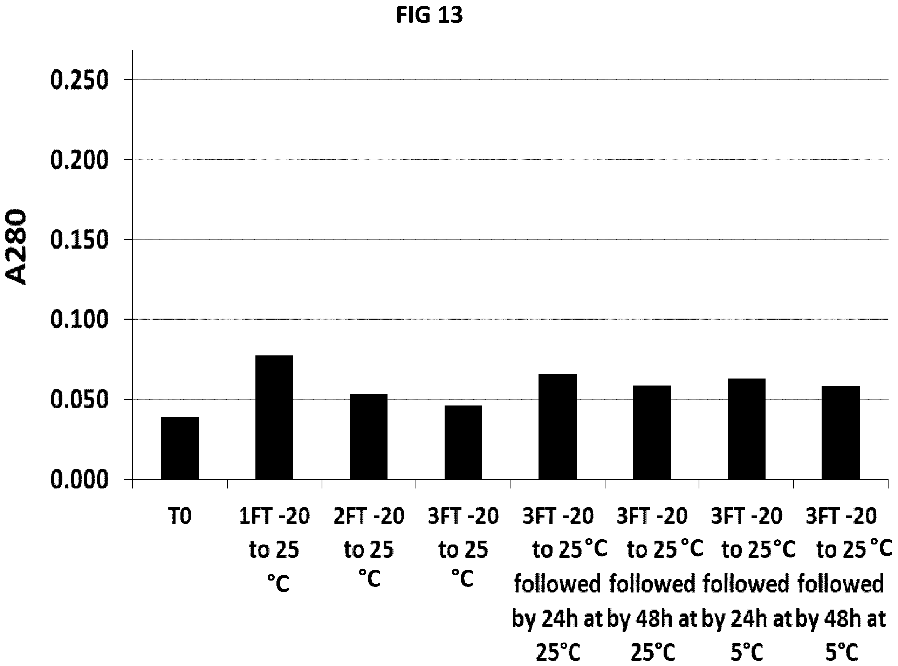

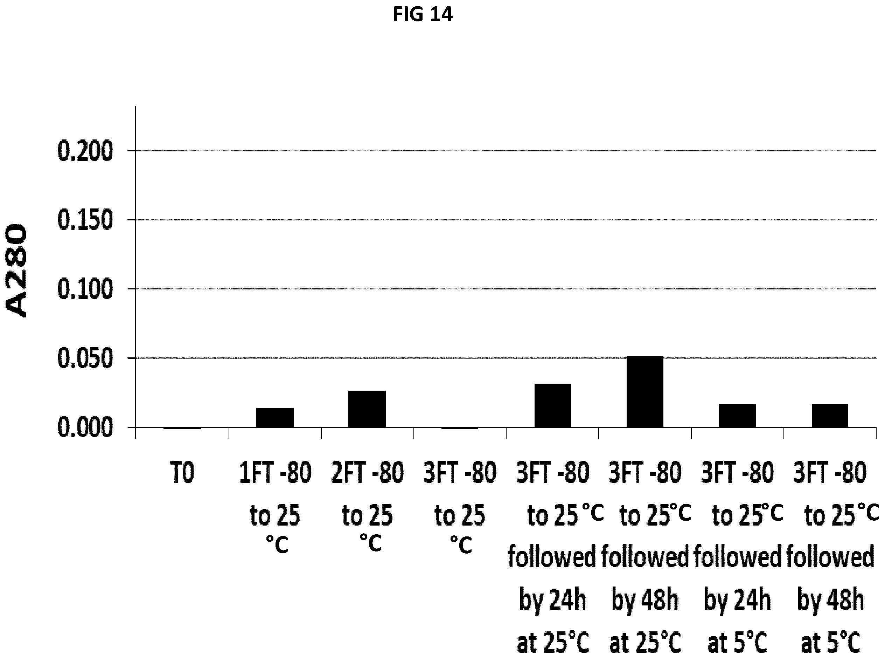

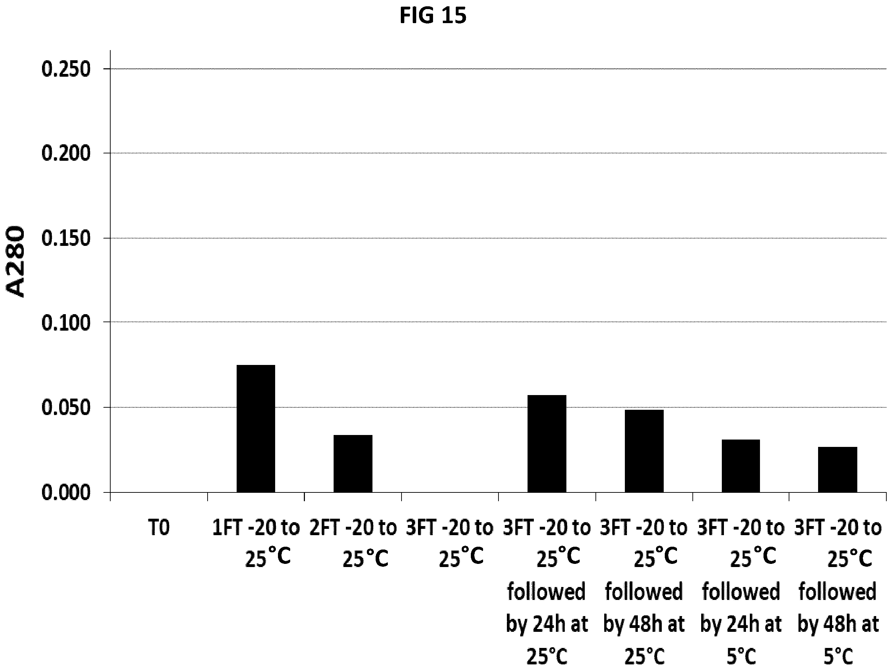

Similar experiment were performed on diluents D3 and D5 to investigate the effect of the buffer type (see Figure 10 and Figure 12 for the results obtained for D3 and Figure 11 and Figure 13 for the results obtained for D5). Figure 14 and Figure 15 show the evaluation of the effect of Polysorbate 80 concentration, the results were obtained for D8 at T0, after one, two or three FT cycles from -80°C to 25°C and from -20°C to 25°C, respectively, as well as and after three FT cycles from -80°C (or -20°C) to 25°C followed by 24h or 48h of incubation at 25°C, or at 5°C.

Based on the comparison between the FT studies performed for D1, D2, D3, D5, and D8 it can be concluded that the freeze temperature (i.e. -20°C or -80°C) has a minor influence in the degradation of the antibody. The type of buffer has an impact on the stability of the antibody, in fact when comparing the degradation profiles of D1 (buffer: histidine), D3 (buffer: Tris) and D5 (buffer: phosphate) it is clear that higher stability is obtained when phosphate is used. Differently the pH of the diluent does not affect the antibody degradation profile, as shown by the results obtained for D1, having pH 7.4 (Figure 5 and Figure 7) and for D2, having pH 7.0 (Figure 8 and Figure 9).

### Effect of Tween type

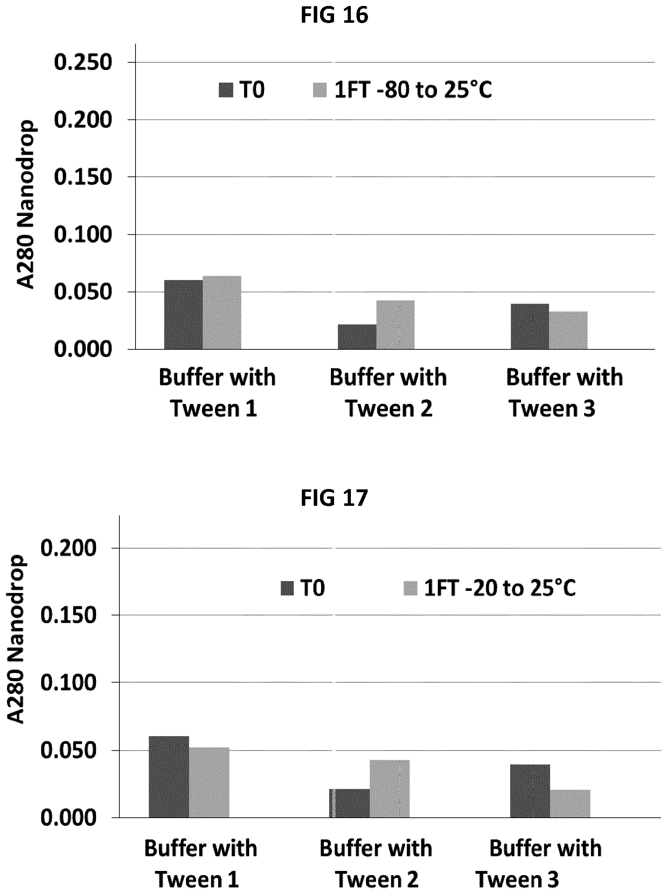

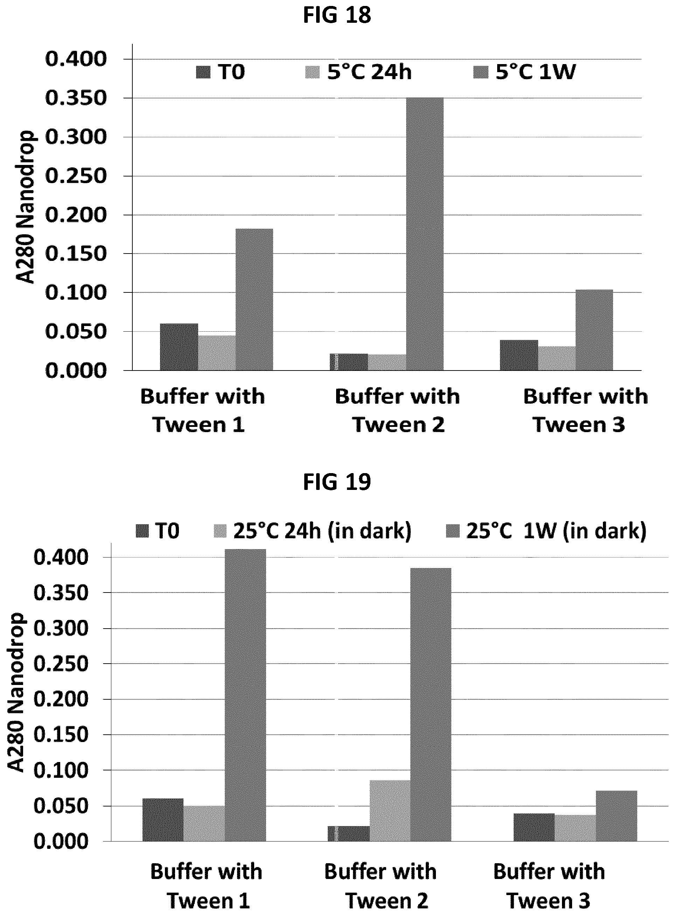

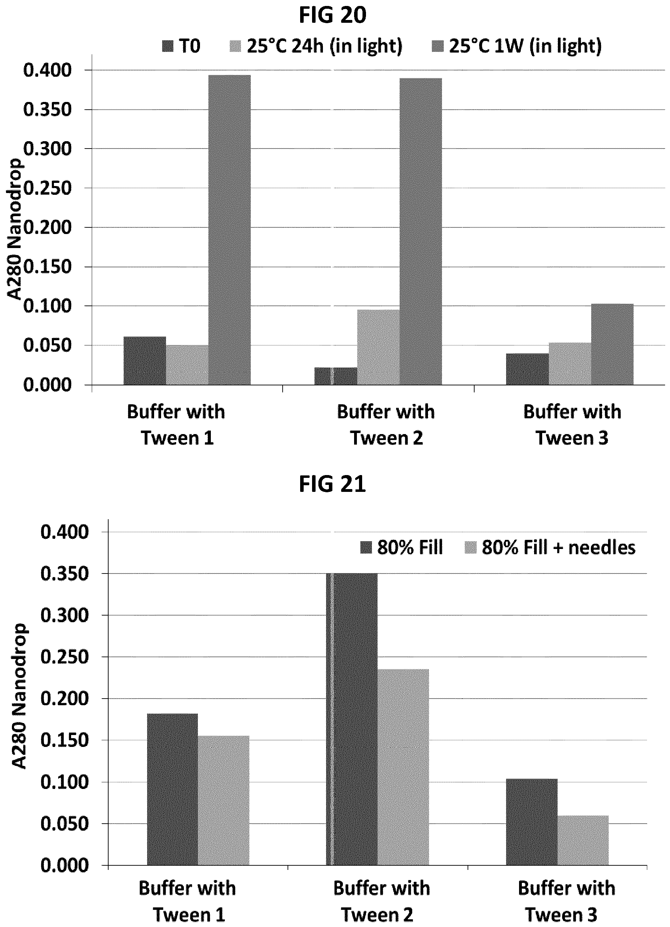

Next the effect of the Polysorbate 80 type on the stability of Ab1 diluted in D1 in a 100 ml glass vial (80% filled) was investigated. In particular Tween 80-1, Tween 80-2 and Tween 80-3 were tested. First the effect of the Polysorbate 80 type was tested before and after 1 cycle of FT from -80°C to 25°C (Figure 16) and after one cycle of FT from -20°C to 25°C (Figure 17) showing overall no significant difference among the tested Tween 80. Next the storage at 5°C for 24 hours and for 1 week (1W) was investigated (Figure 18) demonstrating the instability of the antibody after 1W of storage when Tween 1 and Tween 2 are used. Similar results were obtained after the incubation of the sample at 25°C for 24 hours and for 1 week (1W), both in conditions of darkness (Figure 19) and light (Figure 20).

### Effect of metal

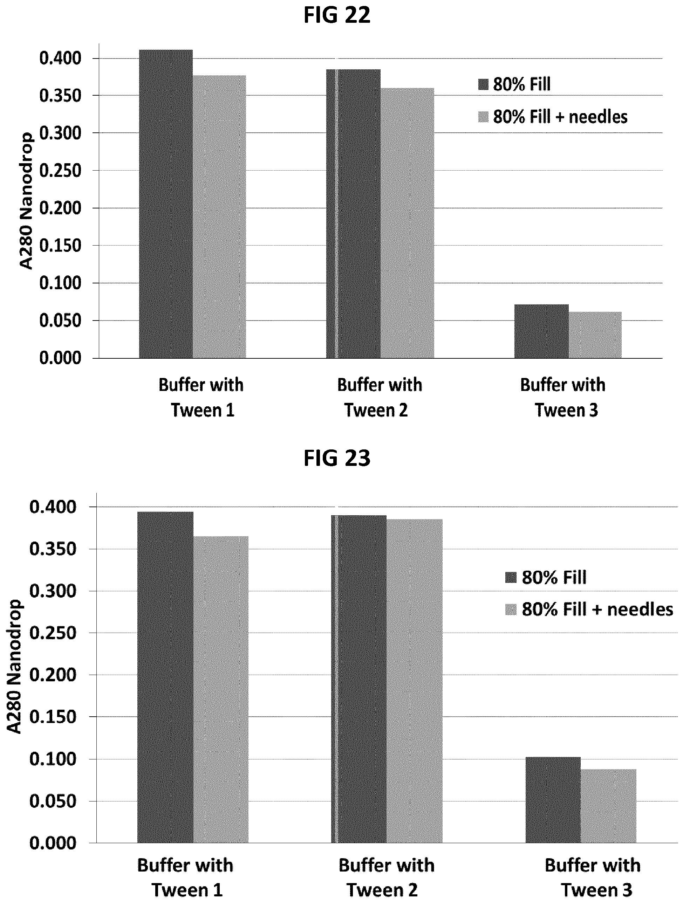

Additionally, it was studied the effect of a metal on the stability of Ab1 diluted in D1 comprising Tween 80-1, or Tween 80-2 or Tween 80-3. In particular, a 100 mL glass vial was filled up to the 80% of the volume, a set of five stainless-steel needles were placed in the middle of the vial, and the stability of the antibody was evaluated after one week of incubation at 5°C (Figure 21), and at 25°C in the dark (Figure 22) or in light conditions (Figure 23). As demonstrated by the results in Figure 21 to Figure 23, the stability of Ab1 is not affected by the presence of the needles.

### Effect of fill volume

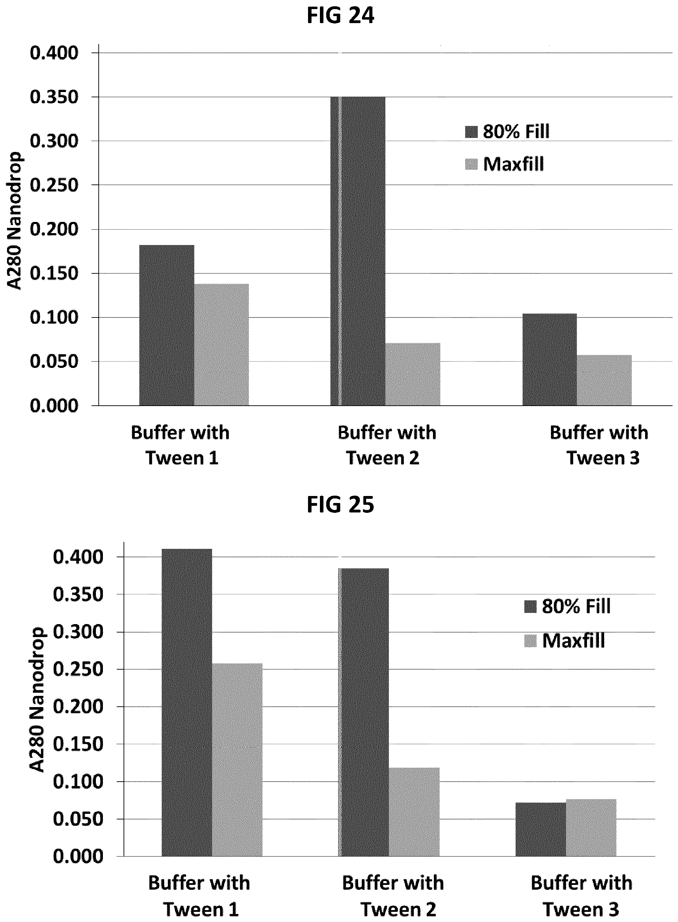

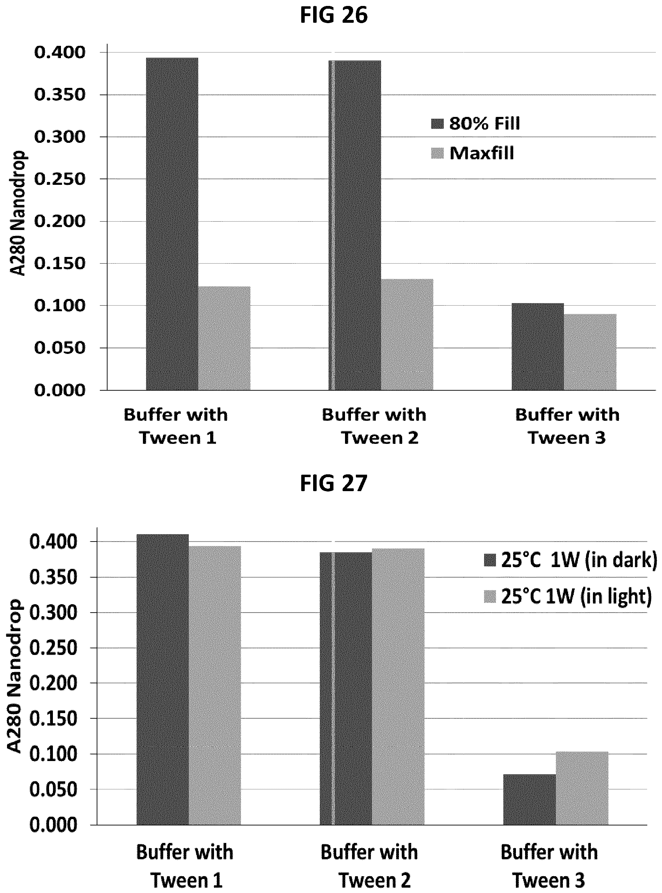

Also, the effect of the vial filling percentage on the stability of Ab1 in D1 comprising Tween 80-1, or Tween 80-2 or Tween 80-3 was investigated. In particular, a 100 mL glass vial was fully filled (Maxfill), therefore excluding the presence of air, or it was filled up to the 80% of the volume and the stability of the antibody was evaluated after one week of incubation at 5°C (Figure 24), and at 25°C in the dark (Figure 25) or in light conditions (Figure 26). The results of this study showed that the fill volume affects the stability of Ab1 just when Tween80-1 or Tween 80-2 are used as Polysorbate 80.

### Effect of light

The comparison of results obtained after the incubation of Ab1 at 25°C for 1 week under light or in darkness conditions demonstrates that light does not influence the stability of Ab1 (Figure 27).

### Example 2: Ab2 stability study in diluents C1 to C18

Ab2 is an EGFRxCD3 bispecific antibody that targets epidermal growth factor receptor (EGFR), a proven target in several cancers including squamous cell carcinoma of the head and neck and colorectal cancer. At low concentration, such as 10 ng/mL, proteins are prone to adsorption over various surfaces including the infusion system (Bag, line, filter etc.). A diluent composition with appropriate excipient is required to limit adsorption losses. In this development different compositions of diluents were tested for recovery of Ab2.

### Materials and Methods

The composition of the tested diluents (C1 to C18) is given in Table 2.

**Table 2. List of the tested custom diluents. ^{a}Prepared using Citric Acid and Trisodium Citrate Dihydrate ^{b}Prepared using L-Histidine and L-Histidine monohydrochloride. ^{c}Prepared using Sodium Phosphate monobasic and Sodium Phosphate dibasic. ^{d}Prepared using Citrate buffer and Phosphate buffer (2:1 ratio) ^{e}Prepared using Citrate buffer and Phosphate buffer (1:2 ratio).**

| **Diluent** | **Composition** |
|---|---|
| C1 | 25 mM Citrate^{a} - 500 mM L-Arginine HCl - 0.04% (w/v) Polysorbate 80 - pH 6.0 |
| C2 | 25 mM Citrate^{a} - 500 mM L-Lysine HCl - 0.04% (w/v) Polysorbate 80 - pH 6.0 |
| C3 | 25 mM Citrate^{a} - 500 mM L-Arginine HCl - 0.04% (w/v) Polysorbate 80 - pH 6.5 |
| C4 | 25 mM Citrate^{a} - 500 mM L-Arginine HCl - 0.04% (w/v) Polysorbate 80 - pH 7.0 |
| C5 | 25 mM Histidine^{b} - 500 mM L-Arginine HCl - 0.04% (w/v) Polysorbate 80 - pH 6.0 |
| C6 | 25 mM Histidine^{b} - 500 mM L-Lysine HCl - 0.04% (w/v) Polysorbate 80 - pH 6.0 |
| C7 | 25 mM Histidine^{b} - 500 mM L-Arginine HCl - 0.04% (w/v) Polysorbate 80 - pH 6.5 |
| C8 | 25 mM Histidine^{b} - 500 mM L-Lysine HCl - 0.04% (w/v) Polysorbate 80 - pH 6.5 |
| C9 | 25 mM Phosphate^{c} - 500 mM L-Arginine HCl - 0.04% (w/v) Polysorbate 80 - pH 6.0 |
| C10 | 25 mM Phosphate^{c} - 500 mM L-Lysine HCl - 0.04% (w/v) Polysorbate 80 - pH 6.0 |
| C11 | 25 mM Phosphate^{c} - 500 mM L-Arginine HCl - 0.04% (w/v) Polysorbate 80 - pH 6.5 |
| C12 | 25 mM Phosphate^{c} - 500 mM L-Lysine HCl - 0.04% (w/v) Polysorbate 80 - pH 6.5 |
| C13 | 25 mM Phosphate^{c} - 500 mM L-Arginine HCl - 0.04% (w/v) Polysorbate 80 - pH 7.0 |
| C14 | 25 mM Phosphate^{c} - 500 mM L-Lysine HCl - 0.04% (w/v) Polysorbate 80 - pH 7.0 |
| C15 | 25 mM Citrate-Phosphate^{d} - 500 mM L-Arginine HCl - 0.04% (w/v) Polysorbate 80 - pH 6.0 |
| C16 | 25 mM Citrate-Phosphate^{d} - 500 mM L-Lysine HCl - 0.04% (w/v) Polysorbate 80 - pH 6.0 |
| C17 | 25 mM Citrate-Phosphate^{e} - 500 mM L-Arginine HCl - 0.04% (w/v) Polysorbate 80 - pH 7.0 |
| C18 | 25 mM Citrate-Phosphate^{e} - 500 mM L-Lysine HCl - 0.04% (w/v) Polysorbate 80 - pH 7.0 |

Materials to be used in clinical trial for intravenous (IV) route include syringes (50 mL), needles (23G), infusion sets, three way stop cock and custom diluent (buffer). For IV compatibility study, different dilutions of Ab2 were prepared and filled either in 50 mL Falcon tubes (Trial 1 and 2), or in saline bags (Trial 3, 4 and 5); T0 samples were taken directly from the Falcon tubes (Trial 1 and 2) or from the saline bags (Trial 3, 4 and 5) using a fresh 3 mL syringe and 18G needle; these were kept at 25 ± 2°C for up to 24 h. After which the Falcon tube samples was used analysis (Trial 1 and 2) or the infusion line (with needle - 23G) was mounted and slow injection was made in an empty glass vial and used for analysis. A summary of trial information is given in Table 3 .

**Table 3. List of giving set material used for the Ab2 custom diluent development**

| **Item** | **Description** | **Trial** |
|---|---|---|
| **Injection bag** | Viaflex 500-mL Sodium Chloride 0.9% | 3 and 4 |
| | Ecobag^{®} 500-mL Natriumchloride 0.9% | 5 |
| **Infusion set** | Infusomat Space Pump IV set | 3 and 4 |
| | Alaris^{®} VP Volumetric Pump 15 µm filter | 5 |
| **3-Way Stopcock** | Discofix^{®} | 3 and 4 |
| | BD Connecta^{™} | 5 |
| **Needle** | BD Microlance^{™} 3 Needle 23G | 3, 4 and 5 |
| **Syringe** | BD 1-mL Luer-Lok^{™} syringe | 3, 4 and 5 |
| | BD 3-mL Luer-Lok^{™} syringe | 3, 4 and 5 |
| | BD PlastiPak^{™} 60-mL syringe | 3, 4 and 5 |

For the compatibility study in Falcon tubes (Trials 1 and 2), Ab2 was diluted in C1 to C18 diluents to the target concentration of 10 ng/mL. For the compatibility study in saline bags (Trials 3, 4 and 5), the following steps were performed sequentially to prepare the samples:
1. Ab2 bulk drug substance (BDS) was diluted to 0.10 mg/mL in vial and the saline bags were kept at room temperature for a minimum of 2h for equilibration to room temperature.
2. Vials were washed with high purified water (HPW) and dried at room temperature. These washed empty vials were autoclaved using steam sterilization and dried. Washed and autoclaved vials were used for collecting samples for ELISA Quantification.
3. Diluents were prepared according to the compositions of Table 2.
4. The prepared buffers were filtered using 0.22 µm filter under laminar air flow hood (LAF) in a sterile 1L glass vessel (Schott), labeled appropriately and stored at 5 ± 3°C until use.
5. For each diluent, an 18G needle and a 60 mL syringe were taken and 55 mL of saline was withdrawn from each bags. This 55 mL saline was then discarded.
6. Using another 18G needle and 60 mL syringe, 55 mL of custom diluent were added in the saline bag (from which 55 mL of saline have already been withdrawn).
7. To the vials containing Ab2 at 0.1 mg/mL, 0.9 mL of diluent was added, thus the volume increased from 1.1 mL to 2.0 mL with subsequent decrease in concentration from 0.1 mg/mL to 0.055 mg/mL.
8. A single dilution of Ab2 was prepared as summarized in Table 4.

**Table 4. Sample preparation (dilution) scheme (IV). *500 mL bags are typically overfilled with 5-10% overage to ensure priming etc. Calculation have been done considering a volume of 550 mL.**

| **Ab2 target concentration** | **Ab2 drug product concentration** | **Ab2concentration in vial after diluent addition** | **Dilution (in saline bag)** | |
|---|---|---|---|---|
| | | | **Ab2 from thawed diluted vial** | **Volume of prepared bag** |
| **ng/mL** | **mg/mL** | **µg/mL** | **mL** | **mL** |
| 10 | 0.1 | 55 | 0.1 | 550* |

9. During this study, different conditions were tested, as presented in Table 5.

**Table 5. Tested conditions summary: X: Tested; NT: Not tested; *Trial 5 was retested using 5 replicates of each condition, hence Ab2 was diluted in 5 bags.**

| | **Trial 3** | | | | **Trial 4** | | **Trial 5** | | | | **Trial 5 retest** | |
|---|---|---|---|---|---|---|---|---|---|---|---|---|
| **Diluent** | T0 25°C (bag) | T0 25°C (line) | T24 25°C (bag) | T24 25°C (line) | T0 25°C | T24 25°C | T0 5°C | T24 5°C | T0 25°C | T24 25°C | T0 25°C | T24 25°C |
| C8 | NT | NT | NT | NT | X | X | NT | NT | NT | NT | NT | NT |
| C9 | X | X | X | X | X | X | X | X | X | X | X* | X* |
| C11 | X | X | X | X | NT | NT | NT | NT | NT | NT | NT | NT |
| C12 | X | X | X | X | X | X | NT | NT | NT | NT | NT | NT |
| C17 | NT | NT | NT | NT | X | X | NT | NT | NT | NT | NT | NT |
| Citrate diluent (D7) | NT | NT | NT | NT | X | X | NT | NT | NT | NT | NT | NT |

10. After incubation, bags were removed and infusion line were connected. In trial 3, the line was connected also at T0 to study the adsorption after incubation at +25 ±2°C for 24 hours both in line and bag.
11. Infusion line was primed and the first 30 mL were discarded, following which sample was collected in glass vials. About 1 mL of sample was collected in glass vials and used for ELISA Quantification (see Table 6 and description below for details).
12. All the samples (T0 and post incubation) were analyzed at same time during each study.

**Table 6. Test method and specification used for Ab2 custom diluent development**

| **Type of assay** | **Parameter** | **Specifications** |
|---|---|---|
| ELISA Quantification | Protein Concentration | 5 - 15 ng/mL |

ELISA method was developed for the quantification of the test protein. Following steps are followed, 1) coating is performed by adding 100 µl of 10 µg/mL of antibody target (Human CD3 1-26 N-terminal peptide FC) on 96 well costar plate. Upon addition of the antibody target, the plate is incubated at 2-8°C overnight. 2) the next morning plate was washed with 200µL of PBS 0.01 % Tween 20 using a plate washer, this was followed by the addition of 200 µL of blocking buffer (2% BSA in PBS) and an incubation of 90 ±30 min at 37°C±2°C. 3) After incubation, the blocking buffer was removed and plate washed with 200µL of PBS 0.01 % Tween 20 using the plate washer. Samples and reference standard (known concentration) were then added (100 µL) to the plate. The plate was further incubated at 37°C ± 2°C for 60 ± 5 minutes. 4) After incubation, the plate was washed with 200µL of PBS 0.01 % Tween 20 and detection antibody was added to the plate. Plate was incubated at 37°C ± 2°C for an additional 60 ± 5 minutes. 5) Finally, the plate was washed with 200µL of PBS 0.01 % tween 20 and developed by the addition of TMB Peroxidase EIA substrate kit. The reaction was stopped after 10 min by the addition of 50 µL of H2SO4 1M solution and the plate was read by using a UV spectrophotometer.

### Results and Discussion

### Trial 1

The aim of the Trial 1 was to screen the diluents of Table 2 to select the most promising for the subsequent trials. In this trial, Ab2 was diluted to a target concentration of 10 ng/mL in the 18 buffer compositions listed in Table 2. Analysis of recovery was performed using ELISA Quantification as described above.

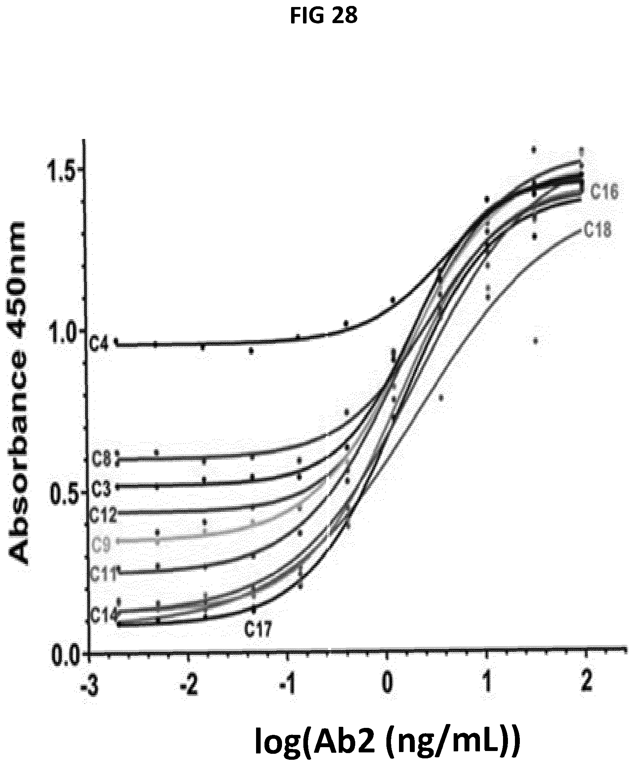

The 18 diluents were analyzed. Data showed a matrix incompatibility (originating from the absence of proper S curve with upper asymptote, lower asymptote and linear points - potential main reason for this is the difference in composition and buffer type leading to matrix effect. This matrix effect makes the data interpretation difficult for the samples) for 8 diluents (C1, C2, C5, C6, C7, C10, C13 and C15). For the 10 others, it seemed that the conditions were not optimal when compared to the blocking buffer, so they were repeated to 5 ng/mL and higher dilution of Strep-HRP. The results are presented in Figure 28.

Based on the data presented in Figure 28, a further selection of diluents to be tested for Trial 2 was made. The shortlisted diluents of Figure 28 were ranked based on three criteria:
- Noise during the analysis;
- Binding (or recovery) of Ab2;
- Accuracy (or closeness) of observed value with target value. In each case, the average of T0 and T24 was compared to target value of 10 ng/mL, then the ranking was applied (Table 7).

**Table 7. Ranking of selected diluents after Trial. NA: Not applicable as T24 values were higher than T0 and hence was ignored for calculation of average ranking value.**

| **Diluent** | **Background noise (AU)** | **Protein binding data (ng/mL)** | | | **Ranking** | | | |
|---|---|---|---|---|---|---|---|---|
| | | **T0** | **T24** | **%Change** | **Noise** | **Binding** | **Accuracy** | **Average** |
| C3 | 0.5 | 9.9 | 4.5 | 54.55 | 2 | 3 | 1 | 2.00 |
| C4 | 1 | 4.6 | 5.5 | -19.57 | 3 | NA | 1 | 2.00 |
| C8 | 0.6 | 11.4 | 11.6 | -1.75 | 3 | 1 | 1 | 1.67 |
| C9 | 0.3 | 9.2 | 13.8 | -50.00 | 2 | NA | 1 | 1.50 |
| C11 | 0.2 | 6.2 | 8.5 | -37.10 | 2 | NA | 1 | 1.50 |
| C12 | 0.4 | 10 | 10 | 0.00 | 2 | 1 | 1 | 1.33 |
| C14 | 0.12 | 22 | 23.8 | -8.18 | 2 | NA | 2 | 2.00 |
| C16 | 0.12 | 19.4 | 18.5 | 4.64 | 2 | 1 | 2 | 1.67 |
| C17 | 0.09 | 23.2 | 18.3 | 21.12 | 1 | 2 | 2 | 1.67 |
| C18 | 0.1 | 81.1 | 89.1 | -9.86 | 1 | NA | 3 | 2.00 |

Based on the above ranking, 4 diluents C9, C11, C12 and C14 were selected for further screening.

### Trial 2

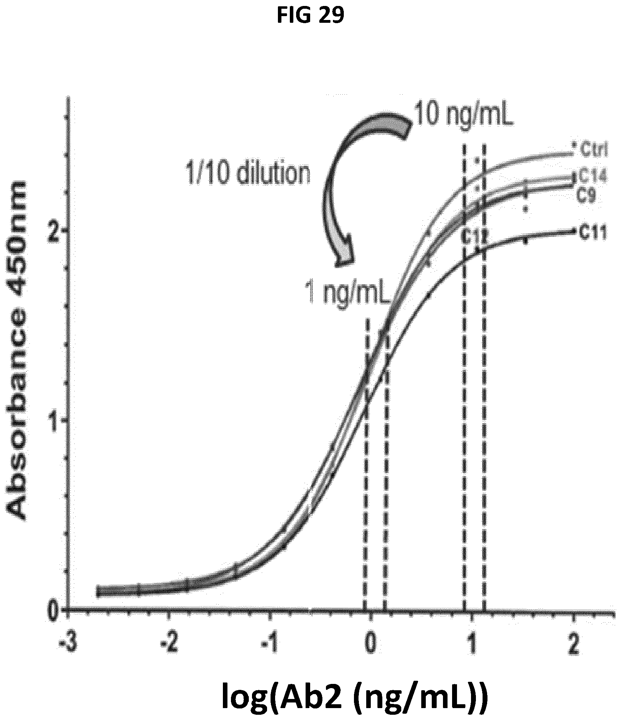

In this Trial, the four selected diluents (C9, C11, C12 and C14) and D7 (see Table 1), named as Control "Ctrl" in Figure 29 and Table 8 were tested. In first attempt of analysis, the samples appeared to be close to the curve plateau, which led to a high variability in the measured concentrations and therefore, the samples were diluted 10 times in order to appear in the center of linear part of curve.

**Table 8. Trial 2 ELISA quantification results**

| **Diluent** | **Specification** | **First attempt** | | **Final results after 1/10 dilution** | |
|---|---|---|---|---|---|
| | | **Target concentration: 10 ng/mL** | | | |
| | | **T0** | **24h at 25±2°C** | **T0** | **24h at 25±2°C** |
| C9 | 5 - 15 ng/mL | 13 | 12 | 11 | 10 |
| C11 | 5 - 15 ng/mL | 13 | 20 | 10 | 11 |
| C12 | 5 - 15 ng/mL | 11 | 16 | 11 | 9 |
| C14 | 5 - 15 ng/mL | 20 | 6 | 10 | 9 |
| D7 | 5 - 15 ng/mL | 7 | 6 | 10 | 10 |

Based on the available data, all recovery results were found to be within the predefined specifications (≥ 5 and ≤ 15 ng/mL). Ab2 was found to be compatible with the tested giving set when diluted to 10 ng/mL in C9, C11, C12, C14 and D7.

### Trial 3

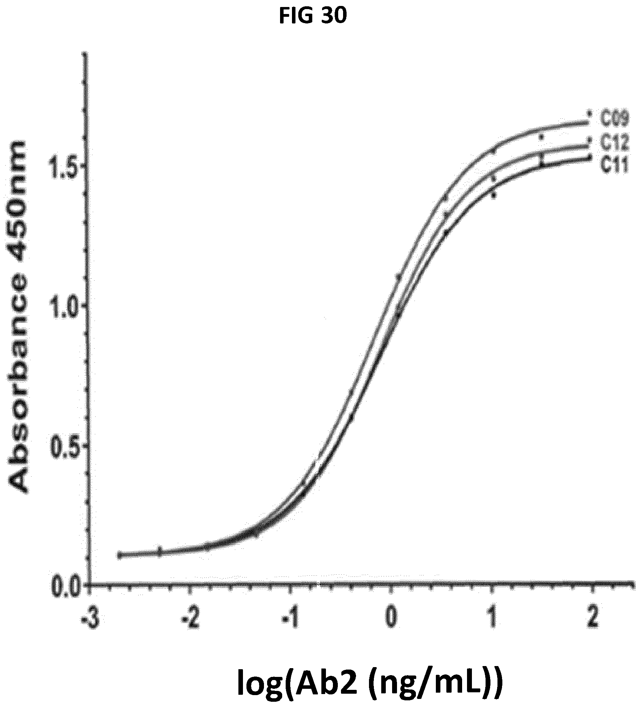

In this Trial, the goal was to test compatibility of Ab2 with a giving set (Table 3) when diluted to 10 ng/mL in C9, C11 and C12 diluents Table 9 and Figure 30.

**Table 9. Trial 3 ELISA quantification results**

| **Diluent** | **Specification** | **Incubation time** | | | |
|---|---|---|---|---|---|
| | | **Target concentration: 10 ng/mL** | | | |
| | | **T0 (from bag)** | **T0 (from line)** | **24h at 25±2°C (from bag)** | **24h at 25±2°C (from line)** |
| C9 | 5 - 15 ng/mL | 5 | 7 | 6 | 7 |
| C11 | 5 - 15 ng/mL | 13 | 13 | 13 | 13 |
| C12 | 5 - 15 ng/mL | 13 | 10 | 12 | 11 |

Based on the available data, all recovery results were found to be within the predefined specifications (≥ 5 and ≤ 15 ng/mL). The Ab2 was found to be compatible with the tested giving set when diluted to 10 ng/mL in C9, C11 and C12.

### Trial 4

In this Trial, the goal was to test compatibility of two diluents C9 and C12 selected from Trial 3 and two diluents C8 and C17 that were abandoned after Trial 1, due to their potential matrix incompatibility with the method. Also D7 was used as a control prior to analysis, all samples were diluted in the blocking buffer so that they reach the same absorbance at 450 nm (Y axis).

**Table 10. Trial 4 ELISA quantification results**

| **Diluent** | **Specification** | **Incubation time** | |
|---|---|---|---|
| | | **Target concentration: 10 ng/mL** | |
| | | **T0** | **24h at 25±2°C** |
| C8 | 5 - 15 ng/mL | 8 | 7 |
| C9 | 5 - 15 ng/mL | 7 | 7 |
| C12 | 5 - 15 ng/mL | 8 | 6 |
| C17 | 5 - 15 ng/mL | 8 | 7 |
| D7 | 5 - 15 ng/mL | 7 | 7 |

Based on the available data, all recovery results were found to be within the predefined specifications (≥ 5 and ≤ 15 ng/mL). Ab2 was found to be compatible with the tested giving set when diluted to 10 ng/mL in C8, C9, C12, C17 and Citrate diluent (D7).

### Trial 5

In this Trial, the goal was to test compatibility of Ab2 with a giving set (Table 3) when diluted to 10 ng/mL in the selected C9 diluent and in D7.

**Table 11. Trial 5 ELISA quantification results**

| **Diluent** | **Specification** | **Incubation time** | | | |
|---|---|---|---|---|---|
| | | **Target concentration: 10 ng/mL** | | | |
| | | **T0 (5 ± 3°C)** | **T24 (5 ± 3°C)** | **T0 (25 ± 2°C)** | **T24 (25 ± 2°C)** |
| C9 | 5 - 15 ng/mL | 2 | 3 | 2 | 2 |
| D7 | 5 - 15 ng/mL | 6 | 7 | 6 | 6 |

Based on the available data, the recovery results were found to be within the predefined specifications (target concentration ± 50%, i.e. ≥ 5 and ≤ 15 ng/mL) for D7, but not for C9 diluent. Hence, Ab2 was found to be compatible with the tested giving set (Table 3) when diluted to 10 ng/mL in Citrate diluent (D7) and seemed not to be compatible with the giving set when diluted to 10 ng/mL in C9 diluent. This observation was confirmed with retesting of C9 diluent as below section.

### Trial 5 (retest of +5 ± 3°C condition)

Based on the previous results, the +5 ± 3°C condition was retested. Ab2 was diluted to 10 ng/mL in 5 separate bags (Table 3) and samples at T0 and post-incubation (24 hours at +5 ± 3°C) were analyzed by ELISA Quantification. All results are presented in the Table 12.

**Table 12. Trial 5 (retest) ELISA quantification results**

| **Custom diluent Code** | **Specification** | **Incubation time** | |
|---|---|---|---|
| | | **Target concentration: 10 ng/mL** | |
| | | **T0 (5 ± 3°C)** | **T24 (5 ± 3°C)** |
| C9 - replicate#1 | 5 - 15 ng/mL | 8 | 7 |
| C9 - replicate#2 | 5 - 15 ng/mL | 13 | 7 |
| C9 - replicate#3 | 5 - 15 ng/mL | 9 | 8 |
| C9 - replicate#4 | 5 - 15 ng/mL | 9 | 10 |
| C9 - replicate#5 | 5 - 15 ng/mL | 9 | 10 |

Based on the available data, all recovery results were found to be within the predefined specifications (≥ 5 and ≤ 15 ng/mL). Ab2 was found to be compatible with the tested giving set (Table 3) when diluted to 10 ng/mL in C9.

In conclusion, based on the recovery data, it seemed that two diluents were optimal for Ab2 when diluted to 10 ng/mL and after 24 hours incubation at both +5 ± 3°C and +25 ± 2°C in infusion bags. The two optimal buffers are as follows:
- Citric acid 2.1 g/L, Sodium chloride 9.0 g/L, L-Lysine Monohydrochloride 91.0 g/L, Polysorbate 80 0.4 g/L (0.04%) pH 7.0 (D7).
- 25 mM Phosphate, 500 mM L-Arginine mono hydrochloride, Polysorbate 80 0.4 g/L (0.04%) pH 6.0 (C9).

D7 was found to be optimal in terms of protection against adsorption losses as well as stability of the buffer composition.

C9 was found to be optimal in terms of protection against adsorption losses and stability, as shown in Example 3.

### Example 3: Ab2 stability studies - long term storage

The objective of this experiments was to assess the stability of Ab2 in C3, C4, C7, C8, C9, C11, C12, C14, C16, C17, C18 diluents when stored at +5 ± 3°C, at +25 ± 2°C and at +40 ± 2°C. Based on the 3 months stability data, three diluents were selected for further stability up to 29 months.

### Materials and methods

Long term stability was assessed by monitoring variation in pH, by evaluating the appearance of the sample and by measuring protein content (by measuring absorbance at 280 nm wavelength (A280)), the sample osmolality, and by performing sub-visible particles analysis using light obscuration method as recommended by USP <787> or by using similar approach while using micro flow imaging instrument. In both the cases the basic principal used was that the presence of particles lead to blockage in light path which is used to measure particle size and count.

The obtained values were compared to the ones set by specification which are considered to ensure sample stability. Tight specifications (typically; target ± 0.1 unit) were established for pH and the intention was to use this assay as sensitive measure of any degradation of buffering components. Standard specification applicable in biopharma industry was applied for appearance testing. For sub visible particle specifications were employed from USP <787>, while the remaining test only "report results" was used as specification at this point in time as the tests require data based specifications - nonetheless they are still evaluated against their change as compared to T0.

### Results and conclusions

### C3 - 3 months of storage at +5±3°C, at +25 ± 2°C and at +40 ± 2°C

**Table 13: C3 stability study: 3 months of storage at +5±3°C, at +25 ± 2°C and at +40 ± 2°C**

| **Stability Tests** | **Specifications** | **Incubation time at +5±3°C** | | |
|---|---|---|---|---|
| | | **T0** | **T1M** | **T3M** |
| pH | pH at T0 ± 0.1 (i.e., ≥ 6.4 and ≤ 6.6) | 6.5 | 6.6 | 6.6 |
| Appearance | Colorless to slightly yellowish, clear to slightly opalescent liquid | Clear, colorless | Clear, colorless | Clear, colorless |
| Conductivity | Report results (in mS/cm) | 24.7 | 20.3 | 33.4 |
| A₂₈₀ | Report results (in AU) | 0.034 | 0.045 | 0.037 |
| Osmolality | Report results (in mOsm/kg) | 846 | 883 | 873 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm:115 | ≥ 10 µm:40 | ≥ 10 µm:160 |
| | | ≥ 25 µm: 20 | ≥ 25 µm: 5 | ≥ 25 µm: 13 |

| **Stability Tests** | **Specification** | **Incubation time at +25±2°C** | | |
|---|---|---|---|---|
| | | **T0** | **T1M** | **T3M** |
| pH | pH at T0 ± 0.1 (i.e., ≥ 6.4 and ≤ 6.6) | 6.5 | 6.6 | 6.6 |
| Appearance | Colorless to slightly yellowish, clear to slightly opalescent liquid | Clear, colorless | Clear, colorless | Clear, colorless |
| Conductivity | Report results (in mS/cm) | 24.7 | 25.8 | 32.6 |
| A₂₈₀ | Report results (in AU) | 0.034 | 0.043 | 0.035 |
| Osmolality | Report results (in mOsm/kg) | 846 | 881 | 872 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm:115 | ≥ 10 µm:55 | ≥ 10 µm:53 |
| | | ≥ 25 µm: 20 | ≥ 25 µm: 0 | ≥ 25 µm: 3 |

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.4 and ≤ 6.6) | 6.5 | 6.6 | 6.6 |
| Appearance | Colorless to slightly yellowish, clear to slightly opalescent liquid | Clear, colorless | Clear, colorless | Clear, colorless |
| Conductivity | Report results (in mS/cm) | 24.7 | 26.6 | 31.0 |
| A₂₈₀ | Report results (in AU) | 0.034 | 0.044 | 0.036 |
| Osmolality | Report results (in mOsm/kg) | 846 | 884 | 853 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm:115 | ≥ 10 µm:30 | ≥ 10 µm:237 |
| | | ≥ 25 µm: 20 | ≥ 25 µm: 0 | ≥ 25 µm: 13 |

Diluent C3 was found to be stable when stored at +5 ± 3°C, at +25 ± 2°C and at +40 ± 2°C for up to 3 months, as pH values, appearance of the liquid and sub-visible particles content were conform to the predefined specifications. No significant change was observed in terms of osmolality, absorbance (A₂₈₀) value and Polysorbate 80 content.

### C4 - 3 months of storage at +5±3°C, at +25 ± 2°C and at +40 ± 2°C

**Table 14: C4 stability study: 3 months of storage at +5±3°C, at +25 ± 2°C and at +40 ± 2°C**

| **Stability Tests** | **Specification** | **Incubation time at +5±3°C** | | |
|---|---|---|---|---|
| | | **T0** | **T1M** | **T3M** |
| pH | pH at T0 ± 0.1 (i.e., ≥ 6.9 and ≤ 7.1) | 7.0 | 7.1 | 7.0 |
| Appearance | Colorless to slightly yellowish, clear to slightly opalescent liquid | Clear, colorless | Clear, colorless | Clear, colorless |
| Conductivity | Report results (in mS/cm) | 15.1 | 7.4 | 33.5 |
| A₂₈₀ | Report results (in AU) | 0.035 | 0.039 | 0.036 |
| Osmolality | Report results (in mOsm/kg) | 861 | 890 | 870 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 85 | ≥ 10 µm:30 | ≥ 10 µm: 157 |
| | | ≥ 25 µm: 0 | ≥ 25 µm: 5 | ≥ 25 µm: 0 |

| **Stability Tests** | **Specification** | **Incubation time at +25±2°C** | | |
|---|---|---|---|---|
| | | **T0** | **T1M** | **T3M** |
| pH | pH at T0 ± 0.1 (i.e., ≥ 6.9 and ≤ 7.1) | 7.0 | 7.1 | 7.0 |
| Appearance | Colorless to slightly yellowish, clear to slightly opalescent liquid | Clear, colorless | Clear, colorless | Clear, colorless |
| Conductivity | Report results (in mS/cm) | 15.1 | 21.7 | 33.0 |
| A₂₈₀ | Report results (in AU) | 0.035 | 0.040 | 0.034 |
| Osmolality | Report results (in mOsm/kg) | 861 | 891 | 876 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 85 | ≥ 10 µm:60 | ≥ 10 µm:87 |
| | | ≥ 25 µm: 0 | ≥ 25 µm: 35 | ≥ 25 µm: 0 |

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.9 and ≤ 7.1) | 7.0 | 7.1 | 7.1 |
| Appearance | Colorless to slightly yellowish, clear to slightly opalescent liquid | Clear, colorless | Clear, colorless | Clear, colorless |
| Conductivity | Report results (in mS/cm) | 15.1 | 21.2 | 32.1 |
| A₂₈₀ | Report results (in AU) | 0.035 | 0.042 | 0.038 |
| Osmolality | Report results (in mOsm/kg) | 861 | 890 | 880 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 85 | ≥ 10 µm:10 | ≥ 10 µm:210 |
| | | ≥ 25 µm: 0 | ≥ 25 µm: 0 | ≥ 25 µm: 20 |

Diluent C4 was found to be stable when stored at +5 ± 3°C, at +25 ± 2°C and at +40 ± 2°C for up to 3 months, as pH value, appearance of the liquid and sub-visible particles content were conform to the predefined specifications. No significant change was observed in terms of osmolality, absorbance (A₂₈₀) value and Polysorbate 80 content.

### C7 - 3 months of storage at +5±3°C, at +25 ± 2°C and at +40 ± 2°C

**Table 15: C7 stability study: 3 months of storage at +5±3°C, at +25 ± 2°C and at +40 ± 2°C**

| **Stability Tests** | **Specification** | **Incubation time at +5±3°C** | | |
|---|---|---|---|---|
| | | **T0** | **T1M** | **T3M** |
| pH | pH at T0 ± 0.1 (i.e., ≥ 6.4 and ≤ 6.6) | 6.5 | 6.6 | 6.5 |
| Appearance | Colorless to slightly yellowish, clear to slightly opalescent liquid | Clear, colorless | Clear, colorless | Clear, colorless |
| Conductivity | Report results (in mS/cm) | 15.9 | 24.4 | 30.8 |
| A₂₈₀ | Report results (in AU) | 0.039 | 0.043 | 0.145 |
| Osmolality | Report results (in mOsm/kg) | 823 | 839 | 831 |
| Polysorbate 80 | Report result (in %w/v) | 0.04 | 0.04 | 0.03 |
| Subvisible particles (SVPs) | ≤ 6000 particles per container of size ≥ 10 µm and ≤ 600 particles per container of size ≥ 25 µm | ≥ 10 µm: 130 | ≥ 10 µm:25 | ≥ 10 µm: 110 |
| | | ≥ 25 µm: 0 | ≥ 25 µm: 0 | ≥ 25 µm: 3 |

| **Stability Tests** | **Specification** | **Incubation time at +25±2°C** | | |
|---|---|---|---|---|
| | | **T0** | **T1M** | **T3M** |
| pH | pH at T0 ± 0.1 (i.e., ≥ 6.4 and ≤ 6.6) | 6.5 | 6.5 | 6.5 |
| Appearance | Colorless to slightly yellowish, clear to slightly opalescent liquid | Clear, colorless | Clear, colorless | Clear, colorless |
| Conductivity | Report results (in mS/cm) | 15.9 | 18.5 | 30.8 |
| A₂₈₀ | Report results (in AU) | 0.039 | 0.199 | 0.232 |
| Osmolality | Report results (in mOsm/kg) | 823 | 831 | 856 |
| Polysorbate 80 | Report result (in %w/v) | 0.04 | 0.03 | 0.03 |
| Subvisible particles (SVPs) | ≤ 6000 particles per container of size ≥ 10 µm and ≤ 600 particles per container of size ≥ 25 µm | ≥ 10 µm: 130 | ≥ 10 µm:25 | ≥ 10 µm:103 |
| | | ≥ 25 µm: 0 | ≥ 25 µm: 10 | ≥ 25 µm: 7 |

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.4 and ≤ 6.6) | 6.5 | 6.5 | 6.5 |
| Appearance | Colorless to slightly yellowish, clear to slightly opalescent liquid | Clear, colorless | Clear, slightly yellowish | Clear, slightly yellowish |
| Conductivity | Report results (in mS/cm) | 15.9 | 19.7 | 29.3 |
| A₂₈₀ | Report results (in AU) | 0.039 | 0.231 | 0.232 |
| Osmolality | Report results (in mOsm/kg) | 823 | 844 | 824 |
| Polysorbate 80 | Report result (in %w/v) | 0.04 | 0.02 | 0.03 |
| Subvisible particles (SVPs) | ≤ 6000 particles per container of size ≥ 10 µm and ≤ 600 particles per container of size ≥ 25 µm | ≥ 10 µm: 130 | ≥ 10 µm: 50 | ≥ 10 µm:10 |
| | | ≥ 25 µm: 0 | ≥ 25 µm: 0 | ≥ 25 µm: 0 |

Diluent C7 was found to be unstable when stored at +5 ± 3°C for up to 3 months and at both +25 ± 2°C and +40 ± 2°C for up to 1 month, as not all the results were conform to the predefined specifications. Indeed, a decrease in Polysorbate 80 concentration was observed after 3 months at +5 ± 3°C and after 1 month at both +25 ± 2°C and +40 ± 2°C. It seems that there is an increasing trend in terms of absorbance value at all storage temperature condition, which may be related to a degradation in the sample. pH value and appearance of the liquid were conform to the predefined specifications.

### C8 - 3 months of storage at +5±3°C, at +25 ± 2°C and at +40 ± 2°C

**Table 16: C8 stability study: 3 months of storage at +5±3°C, at +25 ± 2°C and at +40 ± 2°C**

| **Stability Tests** | **Specification** | **Incubation time at +5±3°C** | | |
|---|---|---|---|---|
| | | **T0** | **T1M** | **T3M** |
| pH | pH at T0 ± 0.1 (i.e., ≥ 6.4 and ≤ 6.6) | 6.5 | 6.5 | 6.5 |
| Appearance | Colorless to slightly yellowish, clear to slightly opalescent liquid | Clear, colorless | Clear, colorless | Clear, colorless |
| Conductivity | Report results (in mS/cm) | 29.8 | 23.3 | 31.6 |
| A₂₈₀ | Report results (in AU) | 0.048 | 0.065 | 0.231 |
| Osmolality | Report results (in mOsm/kg) | 911 | 932 | 921 |
| Polysorbate 80 | Report result (in %w/v) | 0.04 | 0.04 | 0.02 |
| Subvisible particles (SVPs) | ≤ 6000 particles per container of size ≥ 10 µm and ≤ 600 particles per container of size ≥ 25 µm | ≥ 10 µm: 45 | ≥ 10 µm:20 | ≥ 10 µm:107 |
| | | ≥ 25 µm: 0 | ≥ 25 µm: 0 | ≥ 25 µm: 10 |

| **Stability Tests** | **Specification** | **Incubation time at +25±2°C** | | |
|---|---|---|---|---|
| | | **T0** | **T1M** | **T3M** |
| pH | pH at T0 ± 0.1 (i.e., ≥ 6.4 and ≤ 6.6) | 6.5 | 6.5 | 6.5 |
| Appearance | Colorless to slightly yellowish, clear to slightly opalescent liquid | Clear, colorless | Clear, colorless | Clear, slightly yellowish |
| Conductivity | Report results (in mS/cm) | 29.8 | 17.4 | 30.9 |
| A₂₈₀ | Report results (in AU) | 0.048 | 0.240 | 0.231 |
| Osmolality | Report results (in mOsm/kg) | 911 | 935 | 883 |
| Polysorbate 80 | Report result (in %w/v) | 0.04 | 0.03 | 0.03 |
| Subvisible particles (SVPs) | ≤ 6000 particles per container of size ≥ 10 µm and ≤ 600 particles per container of size ≥ 25 µm | ≥ 10 µm: 45 | ≥ 10 µm:55 | ≥ 10 µm: 63 |
| | | ≥ 25 µm: 0 | ≥ 25 µm: 0 | ≥ 25 µm: 7 |

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.4 and ≤ 6.6) | 6.5 | 6.5 | 6.4 |
| Appearance | Colorless to slightly yellowish, clear to slightly opalescent liquid | Clear, colorless | Clear, colorless | Clear, slightly yellowish |
| Conductivity | Report results (in mS/cm) | 29.8 | 22.9 | 30.7 |
| A₂₈₀ | Report results (in AU) | 0.048 | 0.238 | 0.235 |
| Osmolality | Report results (in mOsm/kg) | 911 | 936 | 925 |
| Polysorbate 80 | Report result (in %w/v) | 0.04 | 0.02 | 0.03 |
| Subvisible particles (SVPs) | ≤ 6000 particles per container of size ≥ 10 µm and ≤ 600 particles per container of size ≥ 25 µm | ≥ 10 µm: 45 | ≥ 10 µm:45 | ≥ 10 µm: 37 |
| | | ≥ 25 µm: 0 | ≥ 25 µm: 0 | ≥ 25 µm: 7 |

Diluent C8 was found to be unstable when stored at +5 ± 3°C for up to 3 months, and at both +25 ± 2°C and +40 ± 2°C for up to 1 month, as not all the results were conform to the predefined specifications. Indeed, a decrease in Polysorbate 80 concentration was observed after 3 month at +5 ± 3°C and after 1 month at both +25 ± 2°C and +40 ± 2°C. It seems that there is an increasing trend in terms of absorbance value at all storage temperature condition, which may be related to a degradation in the sample. pH value and appearance of the liquid were conform to the predefined specifications.

### C9 - 3 months of storage at +5±3°C, at +25 ± 2°C and at +40 ± 2°C

**Table 17: C9 stability study: 3 months of storage at +5±3°C, at +25 ± 2°C and at +40 ± 2°C**

| **Stability Tests** | **Specification** | **Incubation time at +5±3°C** | | |
|---|---|---|---|---|
| | | **T0** | **T1M** | **T3M** |
| pH | pH at T0 ± 0.1 (i.e., ≥ 6.0 and ≤ 6.2) | 6.1 | 6.1 | 6.1 |
| Appearance | Colorless to slightly yellowish, clear to slightly opalescent liquid | Clear, colorless | Clear, colorless | Clear, colorless |
| Conductivity | Report results (in mS/cm) | 31.5 | 15.0 | 31.5 |
| A₂₈₀ | Report results (in AU) | 0.037 | 0.042 | 0.037 |
| Osmolality | Report results (in mOsm/kg) | 841 | 855 | 846 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 30 | ≥ 10 µm:5 | ≥ 10 µm:157 |
| | | ≥ 25 µm: 0 | ≥ 25 µm: 0 | ≥ 25 µm: 0 |

| **Stability Tests** | **Specification** | **Incubation time at +25±2°C** | | |
|---|---|---|---|---|
| | | **T0** | **T1M** | **T3M** |
| pH | pH at T0 ± 0.1 (i.e., ≥ 6.0 and ≤ 6.2) | 6.1 | 6.1 | 6.1 |
| Appearance | Colorless to slightly yellowish, clear to slightly opalescent liquid | Clear, colorless | Clear, colorless | Clear, colorless |
| Conductivity | Report results (in mS/cm) | 31.5 | 18.9 | 31.0 |
| A₂₈₀ | Report results (in AU) | 0.037 | 0.042 | 0.035 |
| Osmolality | Report results (in mOsm/kg) | 841 | 856 | 854 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 30 | ≥ 10 µm:25 | ≥ 10 µm: 40 |
| | | ≥ 25 µm: 0 | ≥ 25 µm: 0 | ≥ 25 µm: 0 |

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.0 and ≤ 6.2) | 6.1 | 6.1 | 6.1 |
| Appearance | Colorless to slightly yellowish, clear to slightly opalescent liquid | Clear, colorless | Clear, colorless | Clear, colorless |
| Conductivity | Report results (in mS/cm) | 31.5 | 19.5 | 30.2 |
| A₂₈₀ | Report results (in AU) | 0.037 | 0.047 | 0.036 |
| Osmolality | Report results (in mOsm/kg) | 841 | 857 | 846 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 30 | ≥ 10 µm:5 | ≥ 10 µm: 110 |
| | | ≥ 25 µm: 0 | ≥ 25 µm: 0 | ≥ 25 µm: 7 |

Diluent C9 was found to be stable when stored at +5 ± 3°C, at +25 ± 2°C and at +40 ± 2°C for up to 3 months, as pH value, appearance of the liquid and sub-visible particles content were conform to the predefined specifications. No significant change was observed in terms of conductivity, osmolality, absorbance (A₂₈₀) value and Polysorbate 80 content.

### C11 - 3 months of storage at +5±3°C, at +25 ± 2°C and at +40 ± 2°C

**Table 18: C11 stability study: 3 months of storage at +5±3°C, at +25 ± 2°C and at +40 ± 2°C**

| **Stability Tests** | **Specification** | **Incubation time at +5±3°C** | | |
|---|---|---|---|---|
| | | **T0** | **T1M** | **T3M** |
| pH | pH at T0 ± 0.1 (i.e., ≥ 6.5 and ≤ 6.7) | 6.6 | 6.6 | 6.6 |
| Appearance | Colorless to slightly yellowish, clear to slightly opalescent liquid | Clear, colorless | Clear, colorless | Clear, colorless |
| Conductivity | Report results (in mS/cm) | 30.9 | 16.1 | 31.9 |
| A₂₈₀ | Report results (in AU) | 0.036 | 0.044 | 0.035 |
| Osmolality | Report results (in mOsm/kg) | 837 | 861 | 845 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 25 | ≥ 10 µm:15 | ≥ 10 µm: 407 |
| | | ≥ 25 µm: 0 | ≥ 25 µm: 0 | ≥ 25 µm: 13 |

| **Stability Tests** | **Specification** | **Incubation time at +25±2°C** | | |
|---|---|---|---|---|
| | | **T0** | **T1M** | **T3M** |
| pH | pH at T0 ± 0.1 (i.e., ≥ 6.5 and ≤ 6.7) | 6.6 | 6.6 | 6.6 |
| Appearance | Colorless to slightly yellowish, clear to slightly opalescent liquid | Clear, colorless | Clear, colorless | Clear, colorless |
| Conductivity | Report results (in mS/cm) | 30.9 | 19.8 | 31.2 |
| A₂₈₀ | Report results (in AU) | 0.036 | 0.044 | 0.034 |
| Osmolality | Report results (in mOSm/kg) | 837 | 856 | 861 |
| Polysorbate 80 | Report result (in %) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 25 | ≥ 10 µm:45 | ≥ 10 µm: 77 |
| | | ≥ 25 µm: 0 | ≥ 25 µm: 15 | ≥ 25 µm: 0 |

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.5 and ≤ 6.7) | 6.6 | 6.6 | 6.6 |
| Appearance | Colorless to slightly yellowish, clear to slightly opalescent liquid | Clear, colorless | Clear, colorless | Clear, colorless |
| Conductivity | Report results (in mS/cm) | 30.9 | 15.3 | 31.0 |
| A₂₈₀ | Report results (in AU) | 0.036 | 0.051 | 0.036 |
| Osmolality | Report results (in mOsm/kg) | 837 | 860 | 839 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 25 | ≥ 10 µm:30 | ≥ 10 µm: 173 |
| | | ≥ 25 µm: 0 | ≥ 25 µm: 10 | ≥ 25 µm: 17 |

Diluent C11 was found to be stable when stored at +5 ± 3°C, at +25 ± 2°C and at +40 ± 2°C for up to 3 months, as pH value, appearance of the liquid and sub-visible particles content were conform to the predefined specifications. No significant change was observed in terms of conductivity, osmolality, absorbance (A₂₈₀) value and Polysorbate 80 content.

### C12 - 3 months of storage at +5±3°C, at +25 ± 2°C and at +40 ± 2°C

**Table 19: C12 stability study: 3 months of storage at +5±3°C, at +25 ± 2°C and at +40 ± 2°C**

| **Stability Tests** | **Specification** | **Incubation time at +5±3°C** | | |
|---|---|---|---|---|
| | | **T0** | **T1M** | **T3M** |
| pH | pH at T0 ± 0.1 (i.e., ≥ 6.5 and ≤ 6.7) | 6.6 | 6.6 | 6.6 |
| Appearance | Colorless to slightly yellowish, clear to slightly opalescent liquid | Clear, colorless | Clear, colorless | Clear, colorless |
| Conductivit y | Report results (in mS/cm) | 26.3 | 11.5 | 33.7 |
| A₂₈₀ | Report results (in AU) | 0.037 | 0.044 | 0.035 |
| Osmolality | Report results (in mOsm/kg) | 942 | 953 | 933 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 30 | ≥10 µm:20 | ≥ 10 µm: 187 |
| | | ≥ 25 µm: 0 | ≥25 µm: 0 | ≥ 25 µm: 7 |

| **Stability Tests** | **Specification** | **Incubation time at +25±2°C** | | |
|---|---|---|---|---|
| | | **T0** | **T1M** | **T3M** |
| pH | pH at T0 ± 0.1 (i.e., ≥ 6.5 and ≤ 6.7) | 6.6 | 6.6 | 6.6 |
| Appearance | Colorless to slightly yellowish, clear to slightly opalescent liquid | Clear, colorless | Clear, colorless | Clear, colorless |
| Conductivit y | Report results (in mS/cm) | 26.3 | 18.1 | 32.6 |
| A₂₈₀ | Report results (in AU) | 0.037 | 0.050 | 0.039 |
| Osmolality | Report results (in mOsm/kg) | 942 | 953 | 929 |
| Polysorbate 80 | Report result (in %w/v) | 0.04 | 0.05 | 0.04 |
| Subvisible particles (SVPs) | ≤ 6000 particles per container of size ≥ 10 µm and ≤ 600 particles per container of size ≥ 25 µm | ≥ 10 µm: 30 | ≥ 10 µm:15 | ≥ 10 µm: 110 |
| | | ≥ 25 µm: 0 | ≥ 25 µm: 0 | ≥ 25 µm: 3 |

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.5 and ≤ 6.7) | 6.6 | 6.6 | 6.6 |
| Appearance | Colorless to slightly yellowish, clear to slightly opalescent liquid | Clear, colorless | Clear, colorless | Clear, colorless |
| Conductivit y | Report results (in mS/cm) | 26.3 | 24.2 | 32.1 |
| A₂₈₀ | Report results (in AU) | 0.037 | 0.051 | 0.125 |
| Osmolality | Report results (in mOsm/kg) | 942 | 957 | 938 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 30 | ≥10 µm:0 | ≥ 10 µm: 97 |
| | | ≥ 25 µm: 0 | ≥25 µm: 0 | ≥ 25 µm: 0 |

Diluent C12 was found to be stable when stored at +5 ± 3°C and at +25 ± 2°C for up to 3 months, as pH value, appearance of the liquid and sub-visible particles content were conform to the predefined specifications. No significant change was observed in terms of conductivity, osmolality, absorbance (A₂₈₀) value and Polysorbate 80 content.

Diluent C12 was found to be unstable when stored at +40 ± 2°C for up to 3 months as absorbance value significantly increased from the T0 value (0.037 AU to 0.125 AU). pH value, appearance of the liquid and sub-visible particles content are conform to the predefined specification.

### C14 - 3 months of storage at +5±3°C, at +25 ± 2°C and at +40 ± 2°C

**Table 20: C14 stability study: 3 months of storage at +5±3°C, at +25 ± 2°C and at +40 ± 2°C**

| **Stability Tests** | **Specification** | **Incubation time at +5±3°C** | | |
|---|---|---|---|---|
| | | **T0** | **T1M** | **T3M** |
| pH | pH at T0 ± 0.1 (i.e., ≥ 6.9 and ≤ 7.1) | 7.0 | 7.0 | 7.0 |
| Appearance | Colorless to slightly yellowish, clear to slightly opalescent liquid | Clear, colorless | Clear, colorless | Clear, colorless |
| Conductivity | Report results (in mS/cm) | 27.7 | 20.5 | 33.9 |
| A₂₈₀ | Report results (in AU) | 0.037 | 0.047 | 0.036 |
| Osmolality | Report results (in mOsm/kg) | 959 | 959 | 949 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 55 | ≥ 10 µm:5 | ≥ 10 µm: 180 |
| | | ≥ 25 µm: 10 | ≥ 25 µm: 0 | ≥ 25 µm: 0 |

| **Stability Tests** | **Specification** | **Incubation time at +25±2°C** | | |
|---|---|---|---|---|
| | | **T0** | **T1M** | **T3M** |
| pH | pH at T0 ± 0.1 (i.e., ≥ 6.9 and ≤ 7.1) | 7.0 | 7.0 | 7.0 |
| Appearance | Colorless to slightly yellowish, clear to slightly opalescent liquid | Clear, colorless | Clear, colorless | Clear, colorless |
| Conductivity | Report results (in mS/cm) | 27.7 | 17.6 | 32.6 |
| A₂₈₀ | Report results (in AU) | 0.037 | 0.046 | 0.045 |
| Osmolality | Report results (in mOsm/kg) | 959 | 961 | 946 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 55 | ≥ 10 µm :40 | ≥ 10 µm: 390 |
| | | ≥ 25 µm: 10 | ≥ 25 µm: 20 | ≥ 25 µm: 43 |

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.9 and ≤ 7.1) | 7.0 | 7.0 | 7.0 |
| Appearance | Colorless to slightly yellowish, clear to slightly opalescent liquid | Clear, colorless | Clear, colorless | Clear, colorless |
| Conductivity | Report results (in mS/cm) | 27.7 | 24.6 | 32.4 |
| A₂₈₀ | Report results (in AU) | 0.037 | 0.053 | 0.223 |
| Osmolality | Report results (in mOsm/kg) | 959 | 961 | 947 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 55 | ≥ 10 µm:15 | ≥ 10 µm: 193 |
| | | ≥ 25 µm: 10 | ≥ 25 µm: 5 | ≥ 25 µm: 13 |

Diluent C14 was found to be stable when stored at +5 ± 3°C and at +25 ± 2°C for up to 3 months, as pH value, appearance of the liquid and sub-visible particle content were conform to the predefined specifications. No significant change was observed in terms of osmolality, absorbance (A₂₈₀) value and Polysorbate 80 content. Diluent C14 was found to be unstable when stored at +40 ± 2°C for up to 3 months as absorbance value significantly increased from the T0 value (0.037 AU to 0.223 AU). All other testing (pH, appearance of the liquid, sub-visible particle content) are conform to the predefined specification. Osmolality, absorbance (A₂₈₀) value and Polysorbate 80 content showed no significant change.

### C16 - 3 months of storage at +5±3°C, at +25 ± 2°C and at +40 ± 2°C

**Table 21: C16 stability study: 3 months of storage at +5±3°C, at +25 ± 2°C and at +40 ± 2°C**

| **Stability Tests** | **Specification** | **Incubation time at +5±3°C** | | |
|---|---|---|---|---|
| | | **T0** | **T1M** | **T3M** |
| pH | pH at T0 ± 0.1 (i.e., ≥ 6.0 and ≤ 6.2) | 6.1 | 6.1 | 6.1 |
| Appearance | Colorless to slightly yellowish, clear to slightly opalescent liquid | Clear, colorless | Clear, colorless | Clear, colorless |
| Conductivity | Report results (in mS/cm) | 32.6 | 24.6 | 34.4 |
| A₂₈₀ | Report results (in AU) | 0.037 | 0.047 | 0.035 |
| Osmolality | Report results (in mOsm/kg) | 951 | 968 | 958 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 65 | ≥ 10 µm:20 | ≥ 10 µm: 110 |
| | | ≥ 25 µm: 0 | ≥ 25 µm: 5 | ≥ 25 µm: 7 |

| **Stability Tests** | **Specification** | **Incubation time at +25±2°C** | | |
|---|---|---|---|---|
| | | **T0** | **T1M** | **T3M** |
| pH | pH at T0 ± 0.1 (i.e., ≥ 6.0 and ≤ 6.2) | 6.1 | 6.1 | 6.1 |
| Appearance | Colorless to slightly yellowish, clear to slightly opalescent liquid | Clear, colorless | Clear, colorless | Clear, colorless |
| Conductivity | Report results (in mS/cm) | 32.6 | 21.0 | 33.0 |
| A₂₈₀ | Report results (in AU) | 0.037 | 0.044 | 0.037 |
| Osmolality | Report results (in mOsm/kg) | 951 | 967 | 949 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 65 | ≥ 10 µm:25 | ≥ 10 µm: 90 |
| | | ≥ 25 µm: 0 | ≥ 25 µm: 5 | ≥ 25 µm: 3 |

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.0 and ≤ 6.2) | 6.1 | 6.2 | 6.2 |
| Appearance | Colorless to slightly yellowish, clear to slightly opalescent liquid | Clear, colorless | Clear, colorless | Clear, colorless |
| Conductivity | Report results (in mS/cm) | 32.6 | 22.9 | 32.5 |
| A₂₈₀ | Report results (in AU) | 0.037 | 0.048 | 0.042 |
| Osmolality | Report results (in mOsm/kg) | 951 | 966 | 933 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 65 | ≥ 10 µm:30 | ≥ 10 µm: 290 |
| | | ≥ 25 µm: 0 | ≥ 25 µm: 5 | ≥ 25 µm: 0 |

Diluent C16 was found to be stable when stored at +5 ± 3°C, at +25 ± 2°C and at +40 ± 2°C for up to 3 months, as pH value, appearance of the liquid and sub-visible particle content were conform to the predefined specifications. No significant change was observed in terms of conductivity, osmolality, absorbance (A₂₈₀) value and Polysorbate 80 content.

### C17 - 3 months of storage at +5±3°C, at +25 ± 2°C and at +40 ± 2°C

**Table 22: C17 stability study: 3 months of storage at +5±3°C, at +25 ± 2°C and at +40 ± 2°C**

| **Stability Tests** | **Specification** | **Incubation time at +5±3°C** | | |
|---|---|---|---|---|
| | | **T0** | **T1M** | **T3M** |
| pH | pH at T0 ± 0.1 (i.e., ≥ 7.0 and ≤ 7.2) | 7.1 | 7.1 | 7.1 |
| Appearance | Colorless to slightly yellowish, clear to slightly opalescent liquid | Clear, colorless | Clear, colorless | Clear, colorless |
| Conductivity | Report results (in mS/cm) | 26.8 | 20.9 | 32.2 |
| A₂₈₀ | Report results (in AU) | 0.035 | 0.044 | 0.034 |
| Osmolality | Report results (in mOsm/kg) | 865 | 868 | 847 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 385 | ≥ 10 µm:40 | ≥ 10 µm: 160 |
| | | ≥ 25 µm: 35 | ≥ 25 µm: 5 | ≥ 25 µm: 10 |

| **Stability Tests** | **Specification** | **Incubation time at +25±2°C** | | |
|---|---|---|---|---|
| | | **T0** | **T1M** | **T3M** |
| pH | pH at T0 ± 0.1 (i.e., ≥ 7.0 and ≤ 7.2) | 7.1 | 7.1 | 7.1 |
| Appearance | Colorless to slightly yellowish, clear to slightly opalescent liquid | Clear, colorless | Clear, colorless | Clear, colorless |
| Conductivity | Report results (in mS/cm) | 26.8 | 17.1 | 30.6 |
| A₂₈₀ | Report results (in AU) | 0.035 | 0.056 | 0.035 |
| Osmolality | Report results (in mOsm/kg) | 865 | 867 | 858 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 385 | ≥ 10 µm:15 | ≥ 10 µm: 23 |
| | | ≥ 25 µm: 35 | ≥ 25 µm: 0 | ≥ 25 µm: 0 |

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 7.0 and ≤ 7.2) | 7.1 | 7.1 | 7.1 |
| Appearance | Colorless to slightly yellowish, clear to slightly opalescent liquid | Clear, colorless | Clear, colorless | Clear, colorless |
| Conductivity | Report results (in mS/cm) | 26.8 | 21.9 | 30.9 |
| A₂₈₀ | Report results (in AU) | 0.035 | 0.043 | 0.039 |
| Osmolality | Report results (in mOSm/kg) | 865 | 869 | 858 |
| Polysorbate 80 | Report result (in % w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 385 | ≥ 10 µm:20 | ≥ 10 µm: 143 |
| | | ≥ 25 µm: 35 | ≥ 25 µm: 0 | ≥ 25 µm: 7 |

Diluent C17 was found to be stable when stored at +5 ± 3°C, at +25 ± 2°C and at +40 ± 2°C for up to 3 months, as pH value, appearance of the liquid and sub-visible content were conform to the predefined specifications. No significant change was observed in terms of osmolality, absorbance (A₂₈₀) value and Polysorbate 80 content.

### C18 - 3 months storage at +5±3°C, at +25 ± 2°C and at +40 ± 2°C

**Table 23: C18 stability study: 3 months of storage at +5±3°C, at +25 ± 2°C and at +40 ± 2°C**

| **Stability Tests** | **Specification** | **Incubation time at +5±3°C** | | |
|---|---|---|---|---|
| | | **T0** | **T1M** | **T3M** |
| pH | pH at T0 ± 0.1 (i.e., ≥ 7.0 and ≤ 7.2) | 7.1 | 7.1 | 7.1 |
| Appearance | Colorless to slightly yellowish, clear to slightly opalescent liquid | Clear, colorless | Clear, colorless | Clear, colorless |
| Conductivity | Report results (in mS/cm) | 22.9 | 29.9 | 33.1 |
| A₂₈₀ | Report results (in AU) | 0.041 | 0.053 | 0.035 |
| Osmolality | Report results (in mOsm/kg) | 944 | 966 | 945 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm:40 | ≥ 10 µm:75 | ≥ 10 µm: 80 |
| | | ≥ 25 µm: 0 | ≥ 25 µm: 5 | ≥ 25 µm: 0 |

| Stability **Tests** | **Specification** | **Incubation time at +25±2°C** | | |
|---|---|---|---|---|
| | | **T0** | **T1M** | **T3M** |
| pH | pH at T0 ± 0.1 (i.e., ≥ 7.0 and ≤ 7.2) | 7.1 | 7.1 | 7.1 |
| Appearance | Colorless to slightly yellowish, clear to slightly opalescent liquid | Clear, colorless | Clear, colorless | Clear, colorless |
| Conductivity | Report results (in mS/cm) | 22.9 | 21.6 | 32.9 |
| A₂₈₀ | Report results (in AU) | 0.041 | 0.045 | 0.039 |
| Osmolality | Report results (in mOsm/kg) | 944 | 964 | 953 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm:40 | ≥ 10 µm:20 | ≥ 10 µm: 90 |
| | | ≥ 25 µm: 0 | ≥ 25 µm: 0 | ≥ 25 µm: 3 |

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 7.0 and ≤ 7.2) | 7.1 | 7.1 | 7.1 |
| Appearance | Colorless to slightly yellowish, clear to slightly opalescent liquid | Clear, colorless | Clear, colorless | Clear, colorless |
| Conductivity | Report results (in mS/cm) | 22.9 | 16.8 | 31.7 |
| A₂₈₀ | Report results (in AU) | 0.041 | 0.048 | 0.047 |
| Osmolality | Report results (in mOsm/kg) | 944 | 965 | 946 |
| Polysorbate 80 | Report result (in %w/v) | 0.04 | 0.04 | 0.04 |
| Sub-visible particles (SVPs) | ≤ 6000 particles per container of size ≥ 10 µm and ≤ 600 particles per container of size ≥ 25 µm | ≥ 10 µm:40 | ≥ 10 µm:25 | ≥ 10 µm: 117 |
| | | ≥ 25 µm: 0 | ≥ 25 µm: 0 | ≥ 25 µm: 3 |

Diluent C18 was found to be stable when stored at +5 ± 3°C, at +25 ± 2°C and at +40 ± 2°C for up to 3 months, as pH value, appearance of the liquid and sub-visible particles content were conform to the predefined specifications. No significant change was observed in terms of osmolality, absorbance (A₂₈₀) value and Polysorbate 80 content. In terms of conductivity, it seems that there is an increasing trend, with no relation with temperature, as it happened at all storage temperature conditions. Therefore, it is not related to an instability of the sample.

Based on the results above, following conclusions can be made:
1. Phosphate (C9, C11) based diluents with Arginine HCl and Polysorbate 80 were stable for up to 3 months across all temperatures. The Citrate (C3 and C4) based diluents with Arginine HCl and Polysorbate 80 were stable after 3M at all storage temperature. The Histidine based diluents (C7 and C8) with either of Lysine HCl/Arginine HCl and Polylobate 80 were unstable after 1M of storage under elevated temperatures at or above +25°C and unstable after 3M of storage at +5±3°C.
2. Diluents containing Lysine HCl (C8, C12 and C14) were more unstable at accelerated and stress temperature conditions than diluents with Arginine HCl (C9 and C11).
3. Usage of a dual buffer system (Citrate-Phosphate) dramatically improved the stability of diluents containing Lysine HCl (C14 vs. C18) as stress storage condition, as C18 was stable at all storage temperature after 3M and C14 was unstable at 3M only under stress condition.
4. Diluent containing Lysine HCl at pH 6.5 (C14) was found to be unstable after 3 months of storage under stress condition whereas diluent containing Arginine HCl at pH 6.5 (C12) was found to be stable after 3 months of storage under stress condition. Therefore, at pH 6.5, a buffer system combining Phosphate and Arginine HCl is preferable.

Correspondingly, the best combination was found to be of buffer with Phosphate and Arginine HCl with Polysorbate 80 (C9, C11) are best compositions. However, considering the potential of dual buffer systems (C16) in stabilizing Lysine HCl based system, it would be interesting to monitor their stability as well. Hence the final three diluents selected for long-term stability are C9, C11 and C16.

### C9 - 29 months of storage at +5±3°C

**Table 24: C9 stability study: 29 months of storage at +5±3°C**

| **Stability Tests** | **Specification** | **Incubation time at +5±3°C** | | | |
|---|---|---|---|---|---|
| | | **T0** | **T1M** | **T3M** | **T6M** |
| pH | pH at T0 ± 0.1 (i.e., ≥ 6.0 and ≤ 6.2) | 6.1 | 6.1 | 6.1 | 6.0 |
| Appearance | Colorless to slightly yellowish, clear to slightly opalescent liquid | Clear, colorless | Clear, colorless | Clear, colorless | Clear, colorless |
| Conductivity | Report results (in mS/cm) | 31.5 | 15.0 | 31.5 | 29.9 |
| A₂₈₀ | Report results (in AU) | 0.037 | 0.042 | 0.037 | 0.037 |
| Osmolality | Report results (in mOsm/kg) | 841 | 855 | 846 | 855 |
| Polysorbate 80 | Report result (in %w/v) | 0.04 | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 30 | ≥ 10 µm:10 | ≥ 10 µm:160 | ≥ 10 µm:140 |
| | | ≥ 25 µm: 0 | ≥ 25 µm: 0 | ≥ 25 µm: 0 | ≥ 25 µm: 20 |

| **Stability Tests** | **Specification** | **Incubation time at +5±3°C** | | | |
|---|---|---|---|---|---|
| | | **T9M** | **T12M** | **T18M** | **T29M** |
| pH | pH at T0 ± 0.1 (i.e., ≥ 6.0 and ≤ 6.2) | 6.0 | 6.0 | 6.0 | 6.1 |
| Appearance | Colorless to slightly yellowish, clear to slightly opalescent liquid | Clear, colorless | Clear, colorless | Clear, colorless | Clear, colorless |
| Conductivity | Report results (in mS/cm) | 30.1 | 30.4 | 30.1 | 28.0 |
| A₂₈₀ | Report results (in AU) | 0.033 | 0.034 | 0.032 | 0.031 |
| Osmolality | Report results (in mOsm/kg) | 850 | 851 | 850 | 866 |
| Polysorbate 80 | Report result (in %w/v) | 0.04 | 0.04 | 0.05 | 0.04 |
| Subvisible particles (SVPs) | ≤ 6000 particles per container of size ≥ 10 µm and ≤ 600 particles per container of size ≥ 25 µm | ≥ 10 µm:153 | ≥ 10 µm:20 | ≥ 10 µm:200 | ≥ 10 µm:3 |
| | | ≥ 25 µm: 10 | ≥ 25 µm: 7 | ≥ 25 µm: 33 | ≥ 25 µm: 0 |

### C9 - 12 months of storage at +25±2°C

**Table 25: C9 stability study: 12 months of storage at +25±2°C**

| **Stability Tests** | **Specification** | **Incubation time at +25±2°C** | | | | | |
|---|---|---|---|---|---|---|---|
| | | **T0** | **T1M** | **T3M** | **T6M** | **T9M** | **T12M** |
| pH | pH at T0 ± 0.1 (i.e., ≥ 6.0 and ≤ 6.2) | 6.1 | 6.1 | 6.1 | 6.0 | 6.0 | 6.0 |
| Appearance | Colorless to slightly yellowish, clear to slightly opalescent liquid | Clear, colorless | Clear, colorless | Clear, colorless | Clear, colorless | Clear, colorless | Clear, colorless |
| Conductivity | Report results (in mS/cm) | 31.5 | 18.9 | 31.0 | 30.0 | 30.3 | 30.8 |
| A₂₈₀ | Report results (in AU) | 0.037 | 0.042 | 0.035 | 0.037 | 0.030 | 0.032 |
| Osmolality | Report results (in mOsm/kg) | 841 | 856 | 854 | 849 | 859 | 847 |
| Polysorbate 80 | Report result (in %w/v) | 0.04 | 0.04 | 0.04 | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 30 | ≥ 10 µm:30 | ≥ 10 µm: 40 | ≥ 10 µm:80 | ≥ 10 µm:147 | ≥ 10 µm:57 |
| | | ≥ 25 µm: 0 | ≥ 25 µm: 0 | ≥ 25 µm: 0 | ≥ 25 µm: 10 | ≥ 25 µm: 1 | ≥ 25 µm: 0 |

### C9 - 3 months of storage at +40±2°C

**Table 26: C9 stability study: 3 months of storage at +40±2°C**

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.0 and ≤ 6.2) | 6.1 | 6.1 | 6.1 |
| Appearance | Colorless to slightly yellowish, clear to slightly opalescent liquid | Clear, colorless | Clear, colorless | Clear, colorless |
| Conductivity | Report results (in mS/cm) | 31.5 | 19.5 | 30.2 |
| A₂₈₀ | Report results (in AU) | 0.037 | 0.047 | 0.036 |
| Osmolality | Report results (in mOsm/kg) | 841 | 857 | 846 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 30 | ≥ 10 µm:10 | ≥ 10 µm: 110 |
| | | ≥ 25 µm: 0 | ≥ 25 µm: 0 | ≥ 25 µm: 10 |

### C11- 29 months of storage at +5±3°C

**Table 27: C11 stability study: 29 months of storage at +5±3°C**

| **Stability Tests** | **Specification** | **Incubation time at +5±3°C** | | | |
|---|---|---|---|---|---|
| | | **T0** | **T1M** | **T3M** | **T6M** |
| pH | pH at T0 ± 0.1 (i.e., ≥ 6.0 and ≤ 6.2) | 6.6 | 6.6 | 6.6 | 6.5 |
| Appearance | Colorless to slightly yellowish, clear to slightly opalescent liquid | Clear, colorless | Clear, colorless | Clear, colorless | Clear, colorless |
| Conductivity | Report results (in mS/cm) | 30.9 | 16.1 | 31.9 | 30.3 |
| A₂₈₀ | Report results (in AU) | 0.036 | 0.044 | 0.035 | 0.037 |
| Osmolality | Report results (in mOsm/kg) | 837 | 861 | 845 | 853 |
| Polysorbate 80 | Report result (in %w/v) | 0.04 | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 30 | ≥ 10 µm: 20 | ≥ 10 µm: 410 | ≥ 10 µm: 110 |
| | | ≥ 25 µm: 0 | ≥ 25 µm: 0 | ≥ 25 µm: 10 | ≥ 25 µm: 10 |

| Stability **Tests** | **Specification** | **Incubation time at +5±3°C** | | | |
|---|---|---|---|---|---|
| | | **T9M** | **T12M** | **T18M** | **T29M** |
| pH | pH at T0 ± 0.1 (i.e., ≥ 6.0 and ≤ 6.2) | 6.5 | 6.5 | 6.5 | 6.6 |
| Appearance | Colorless to slightly yellowish, clear to slightly opalescent liquid | Clear, colorless | Clear, colorless | Clear, colorless | Clear, colorless |
| Conductivity | Report results (in mS/cm) | 30.3 | 31.0 | 31.1 | 27.8 |
| A₂₈₀ | Report results (in AU) | 0.032 | 0.034 | 0.030 | 0.032 |
| Osmolality | Report results (in mOsm/kg) | 865 | 877 | 857 | 861 |
| Polysorbate 80 | Report result (in %w/v) | 0.04 | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 143 | ≥ 10 µm: 73 | ≥ 10 µm:333 | ≥ 10 µm:6 |
| | | ≥ 25 µm: 7 | ≥ 25 µm: 57 | ≥ 25 µm: 50 | ≥ 25 µm: 0 |

### C11- 12 months of storage at +25±2°C

**Table 28: C11 stability study: 12 months of storage at +25±2°C**

| **Stability Tests** | **Specification** | **Incubation time at +25±2°C** | | | | | |
|---|---|---|---|---|---|---|---|
| | | **T0** | **T1M** | **T3M** | **T6M** | **T9M** | **T12M** |
| pH | pH at T0 ± 0.1 (i.e., ≥ 6.5 and ≤ 6.7) | 6.6 | 6.6 | 6.6 | 6.5 | 6.5 | 6.5 |
| Appearance | Colorless to slightly yellowish, clear to slightly opalescent liquid | Clear, colorless | Clear, colorless | Clear, colorless | Clear, colorless | Clear, colorless | Clear, colorless |
| Conductivity | Report results (in mS/cm) | 30.9 | 19.8 | 31.2 | 30.3 | 30.5 | 30.8 |
| A₂₈₀ | Report results (in AU) | 0.036 | 0.044 | 0.034 | 0.033 | 0.031 | 0.033 |
| Osmolality | Report results (in mOSm/kg) | 837 | 856 | 861 | 858 | 861 | 850 |
| Polysorbate 80 | Report result (in % w/v) | 0.04 | 0.04 | 0.04 | 0.04 | 0.03 | 0.04 |
| Subvisible particles (SVPs) | ≤ 6000 particles per container of size ≥ 10 µm and ≤ 600 particles per container of size ≥ 25 µm | ≥ 10 µm: 30 | ≥ 10 µm:50 | ≥ 10 µm: 80 | ≥ 10 µm: 170 | ≥ 10 µm:183 | ≥ 10 µm: 43 |
| | | ≥ 25 µm: 0 | ≥ 25 µm: 20 | ≥ 25 µm: 0 | ≥ 25 µm: 40 | ≥ 25 µm: 3 | ≥ 25 µm: 0 |

### C11 - 3 months of storage at +40±2°C

**Table 29: C11 stability study: 3 months of storage at +40±2°C**

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.5 and ≤ 6.7) | 6.6 | 6.6 | 6.6 |
| Appearance | Colorless to slightly yellowish, clear to slightly opalescent liquid | Clear, colorless | Clear, colorless | Clear, colorless |
| Conductivity | Report results (in mS/cm) | 30.9 | 15.3 | 31.0 |
| A₂₈₀ | Report results (in AU) | 0.036 | 0.051 | 0.036 |
| Osmolality | Report results (in mOsm/kg) | 837 | 860 | 839 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 3 | ≥ 10 µm:3 | ≥ 10 µm: 17 |
| | | ≥ 25 µm: 0 | ≥ 25 µm: 1 | ≥ 25 µm: 2 |

### C16 - 29 months of storage at +5±3°C

**Table 30: C16 stability study: 29 months of storage at +5±3°C**

| **Stability Tests** | **Specification** | **Incubation time at +5±3°C** | | | |
|---|---|---|---|---|---|
| | | **T0** | **T1M** | **T3M** | **T6M** |
| pH | pH at T0 ± 0.1 (i.e., ≥ 6.0 and ≤ 6.2) | 6.1 | 6.1 | 6.1 | 6.0 |
| Appearance | Colorless to slightly yellowish, clear to slightly opalescent liquid | Clear, colorless | Clear, colorless | Clear, colorless | Clear, colorless |
| Conductivity | Report results (in mS/cm) | 32.6 | 24.6 | 34.4 | 32.3 |
| A₂₈₀ | Report results (in AU) | 0.037 | 0.047 | 0.035 | 0.040 |
| Osmolality | Report results (in mOsm/kg) | 951 | 968 | 958 | 983 |
| Polysorbate 80 | Report result (in %w/v) | 0.04 | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 70 | ≥ 10 µm:20 | ≥ 10 µm: 110 | ≥ 10 µm: 290 |
| | | ≥ 25 µm: 0 | ≥ 25 µm: 10 | ≥ 25 µm: 10 | ≥ 25 µm: 30 |

| **Stability Tests** | **Specification** | **Incubation time at +5±3°C** | | | |
|---|---|---|---|---|---|
| | | **T9M** | **T12M** | **T18M** | **T29M** |
| pH | pH at T0 ± 0.1 (i.e., ≥ 6.0 and ≤ 6.2) | 6.0 | 6.0 | 6.1 | 6.1 |
| Appearance | Colorless to slightly yellowish, clear to slightly opalescent liquid | Clear, colorless | Clear, colorless | Clear, colorless | Clear, colorless |
| Conductivity | Report results (in mS/cm) | 33.1 | 33.4 | 33.2 | 11.2 (2) |
| A₂₈₀ | Report results (in AU) | 0.037 | 0.036 | 0.030 | 0.032 |
| Osmolality | Report results (in mOsm/kg) | 971 | 984 | 963 | 965 |
| Polysorbate 80 | Report result (in %w/v) | 0.04 | 0.04 | 0.05 | 0.04 |
| Subvisible particles (SVPs) | ≤ 6000 particles per container of size ≥ 10 µm and ≤ 600 particles per container of size ≥ 25 µm | ≥ 10 µm: 160 | ≥ 10 µm: 27 | ≥ 10 µm:167 | ≥ 10 µm:2 |
| | | ≥ 25 µm: 7 | ≥ 25 µm: 0 | ≥ 25 µm: 0 | ≥ 25 µm: 0 |

### C16 - 12 months of storage at +25±2°C

**Table 31: C16 stability study: 12 months of storage at +5±3°C**

| **Stability Tests** | **Specification** | **Incubation time at +25±2°C** | | | | | |
|---|---|---|---|---|---|---|---|
| | | **T0** | **T1M** | **T3M** | **T6M** | **T9M** | **T12M** |
| pH | pH at T0 ± 0.1 (i.e., ≥ 6.0 and ≤ 6.2) | 6.1 | 6.1 | 6.1 | 6.0 | 6.1 | 6.0 |
| Appearance | Colorless to slightly yellowish, clear to slightly opalescent liquid | Clear, colorless | Clear, colorless | Clear, colorless | Clear, colorless | Clear, colorless | Clear, colorless |
| Conductivity | Report results (in mS/cm) | 32.6 | 21.0 | 33.0 | 32.9 | 32.5 | 32.6 |
| A₂₈₀ | Report results (in AU) | 0.037 | 0.044 | 0.037 | 0.042 | 0.039 | 0.038 |
| Osmolality | Report results (in mOsm/kg) | 951 | 967 | 949 | 977 | 978 | 973 |
| Polysorbate 80 | Report result (in %w/v) | 0.04 | 0.04 | 0.04 | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 70 | ≥ 10 µm:30 | ≥ 10 µm: 90 | ≥ 10 µm: 320 | ≥ 10 µm:183 | ≥ 10 µm: 40 |
| | | ≥ 25 µm: 0 | ≥ 25 µm: 10 | ≥ 25 µm: 0 | ≥ 25 µm: 20 | ≥ 25 µm: 3 | ≥ 25 µm: 3 |

### C16 - 3 months of storage at +40±2°C

**Table 32: C16 stability study: 3 months of storage at +40±2°C**

| **Stability Tests** | **Specification** | **Incubation time at +40±2°C** | | |
|---|---|---|---|---|
| | | **T0** | **T1M** | **T3M** |
| pH | pH at T0 ± 0.1 (i.e., ≥ 6.0 and ≤ 6.2) | 6.1 | 6.2 | 6.2 |
| Appearance | Colorless to slightly yellowish, clear to slightly opalescent liquid | Clear, colorless | Clear, colorless | Clear, colorless |
| Conductivity | Report results (in mS/cm) | 32.6 | 22.9 | 32.5 |
| A₂₈₀ | Report results (in AU) | 0.037 | 0.048 | 0.042 |
| Osmolality | Report results (in mOsm/kg) | 951 | 966 | 933 |
| Polysorbate 80 | Report result (in %w/v) | 0.04 | 0.04 | 0.04 |
| Subvisible particles (SVPs) | ≤ 6000 particles per container of size ≥ 10 µm and ≤ 600 particles per container of size ≥ 25 µm | ≥ 10 µm: 70 | ≥ 10 µm:30 | ≥ 10 µm: 290 |
| | | ≥ 25 µm: 0 | ≥ 25 µm: 10 | ≥ 25 µm: 0 |

In conclusion, based on the available data:
- C9 diluent is stable for up to 29 months when stored at +5±3°C, for up to 12 months when stored at +25±2°C, and for up to 3 months when stored at +40±2°C in 10-mL vials (VCDIN10R, from Schott), as it is within the predefined specifications in terms of pH, appearance of the liquid and in terms of sub-visible particles content. No significant change was observed in terms of conductivity, osmolality, absorbance (A₂₈₀) value and Polysorbate 80 content.
- C11 diluent is stable for up to 29 months when stored at +5±3°C, for up to 12 months when stored at +25±2°C, and for up to 3 months when stored at +40±2°C in 10-mL vials (VCDIN10R, from Schott), as it is within the predefined specifications in terms of pH, appearance of the liquid and in terms of sub-visible particles content. No significant change was observed in terms of conductivity, osmolality, absorbance (A₂₈₀) value and Polysorbate 80 content.
- C16 diluent is stable for up to 29 months when stored at +5±3°C, for up to 12 months when stored at +25±2°C, and for up to 3 months when stored at +40±2°C in 10-mL vials (VCDIN10R, from Schott), as it is within the predefined specifications in terms of pH, appearance of the liquid and in terms of sub-visible particles content. No significant change was observed in terms of conductivity, osmolality, absorbance (A₂₈₀) value and Polysorbate 80 content.

**Table 33: Results summary**

| **Diluent** | **Formulation composition** | **Temperature condition** | | |
|---|---|---|---|---|
| | | **+5 ± 3°C** | **+25 ± 2°C** | **+40 ± 2°C** |
| C3 | 25 mM Citrate, 500 mM L-Arginine monohydrochloride, 0.04% w/v Polysorbate 80 pH 6.5 | Stable up to 3M | Stable up to 3M | Stable up to 3M |
| C4 | 25 mM Citrate, 500 mM L-Arginine monohydrochloride, 0.04%w/v Polysorbate 80 pH 7.0 | Stable up to 3M | Stable up to 3M | Stable up to 3M |
| C7 | 25 mM Histidine, 500 mM L-Arginine monohydrochloride, 0.04%w/v Polysorbate 80 pH 6.5 | Not stable up to 3M | Not stable up to 1M | Not stable up to 1M |
| C8 | 25 mM Histidine, 500 mM L-Lysine monohydrochloride, 0.04% Polysorbate 80 pH 6.5 | Not stable up to 3M | Not stable up to 1M | Not stable up to 1M |
| C9 | 25 mM Phosphate, 500 mM L-Arginine monohydrochloride, 0.04%w/v Polysorbate 80 pH 6.0 | Stable up to 29M | Stable up to 12M | Stable up to 3M |
| C11 | 25 mM Phosphate, 500 mM L-Arginine monohydrochloride, 0.04%w/v Polysorbate 80 pH 6.5 | Stable up to 29M | Stable up to 12M | Stable up to 3M |
| C12 | 25 mM Phosphate, 500 mM L-Lysine monohydrochloride, 0.04%w/v Polysorbate 80 pH 6.5 | Stable up to 3M | Stable up to 3M | Not stable up to 3M |
| C14 | 25 mM Phosphate, 500 mM L-Lysine monohydrochloride, 0.04% Polysorbate 80 pH 7.0 | Stable up to 3M | Stable up to 3M | Not stable up to 3M |
| C16 | 25 mM Citrate-Phosphate, 500 mM L-Lysine monohydrochloride, 0.04%w/v Polysorbate 80 pH 6.0 | Stable up to 29M | Stable up to 12M | Stable up to 3M |
| C17 | 25 mM Citrate-Phosphate, 500 mM L-Arginine monohydrochloride, 0.04%w/v Polysorbate 80 pH 7.0 | Stable up to 3M | Stable up to 3M | Stable up to 3M |
| C18 | 25 mM Citrate-Phosphate, 500 mM L-Lysine monohydrochloride, 0.04%w/v Polysorbate 80 pH 7.0 | Stable up to 3M | Stable up to 3M | Stable up to 3M |

## Claims

1. A diluent for diluting a drug product wherein said diluent comprises a buffer and one or more stabilizing or tonicity agents, wherein said drug product comprises a multispecific hetero-dimeric antibody or hetero-dimeric antibody fragment thereof comprising a first and a second engineered CH3 domain, and wherein upon the dilution of said drug product with said diluent a drug product ready for administration to a patient by infusion is obtained, **characterized in that** said diluent has a pH between 5.5 and 7.5, said buffer is selected from the group comprising Histidine, Tris, Citrate, Phosphate and Citrate phosphate, and wherein said buffer is present within said diluent at a concentration equal to or less than 30 mM, said stabilizing or tonicity agent is selected from the group comprising polyols such as Polysorbate 20, Polysorbate 40, Polysorbate 80, amino acids such as histidine, arginine, glycine, methionine, proline, lysine, salts such as sodium chloride, and **in that** the loss of antibody material in an infusion system, measured by ELISA, is less than 30%.

2. The diluent of any one of claim 1, wherein said buffer is selected from the group comprising Histidine, Tris and Phosphate, present within said diluent at a concentration of 25 mM, and said stabilizing or tonicity agents comprise Arginine, present within said diluent at a concentration of 100 mM, and Polysorbate 80, present within said diluent at a concentration of 0.05% (w/v) or at a concentration of 0.004% (w/v), and wherein said diluent has a pH of 7 or of 7.4.

3. The diluent of any one of claims 1 to 2, wherein said buffer is Citrate, present within said diluent at a concentration of 1.1 mM, and said stabilizing or tonicity agents comprise Lysine-HCl, present within said diluent at a concentration of 62 mM, sodium chloride, present within said diluent at a concentration of 154 mM, and Polysorbate 80, present within said diluent at a concentration of 0.004% (w/v), and wherein said diluent has a pH from 6.6 to 7.

4. The diluent of any one of claims 1 to 2, wherein said diluent comprises a buffer selected from the group comprising Histidine, Phosphate, Citrate and Citrate-Phosphate, present within said diluent at a concentration of 25 mM, and said stabilizing or tonicity agents comprise Arginine-HCl or Lysine-HCl, present within said diluent at a concentration of 500 mM, and Polysorbate 80, present within said diluent at a concentration of 0.04% (w/v), and wherein said diluent has a pH selected from the group comprising 6, 6.5 and 7.

5. The diluent of claim 4, wherein said buffer is selected from the group comprising Phosphate, Citrate and Citrate-Phosphate, present within said diluent at a concentration of 25 mM, and said stabilizing or tonicity agents comprise Arginine-HCl or Lysine-HCl, present within said diluent at a concentration of 500 mM, and Polysorbate 80, present within said diluent at a concentration of 0.04% (w/v), and wherein said diluent has a pH of 6.5 or of 7 and it is stable at 5°C and/or 25°C and/or at 40°C for at least 3 months.

6. The diluent of claim 4, wherein said buffer is Phosphate or Citrate-Phosphate, present within said diluent at a concentration of 25 mM, and said stabilizing or tonicity agents comprise Arginine-HCl or Lysine-HCl, present within said diluent at a concentration of 500 mM, and Polysorbate 80, present within said diluent at a concentration of 0.04% (w/v), and wherein said diluent has a pH of 6 or of 6.5 and it is stable at 5°C for at least 29 months, at 25°C for at least 12 months, and at 40°C for at least 3 months.

7. The diluent of claim 6, wherein said buffer is Phosphate present within said diluent at a concentration of 25 mM, said stabilizing or tonicity agents are Arginine-HCl, present within said diluent at a concentration of 500 mM, and Polysorbate 80, present within said diluent at a concentration of 0.04% (w/v), and wherein said diluent has a pH of 6 and it is stable at 5°C for at least 29 months, at 25°C for at least 12 months, and at 40°C for at least 3 months.

8. The diluent of any one of the preceding claims, wherein an isotonic solution selected from the group comprising saline solution, dextrose solution, and combination of saline and dextrose solution is mixed with said diluent at a ratio of 1:10.

9. The diluent of any one of the preceding claims wherein said multispecific hetero-dimeric antibody or hetero-dimeric antibody fragment thereof is present within said drug product ready for administration at a concentration between 5 ng/mL and 20 ng/mL.

10. The diluent of claim 9 wherein said multispecific hetero-dimeric antibody or hetero-dimeric antibody fragment thereof is present within said drug product ready for administration at a concentration of 10 ng/mL.

11. The diluent of claim 1, wherein said first engineered CH3 domain comprises the substitutions of the group consisting of: S20K, T22V, K26T, K79Y, F85.1S, Y86V, K88W, T90N, and said second engineered CH3 domain comprises the substitutions of the group consisting of: Q3E, Y5A, L7F, S20T, T22V, K26T, T81D, V84L, D84.2E, F85.1A, Y86S, K88R, T90R, and wherein said multispecific hetero-dimeric immunoglobulin or hetero-dimeric fragment heterodimerize through said first and second engineered CH3 domains.

12. The diluent of any one of the preceding claims wherein said multispecific hetero-dimeric antibody or hetero-dimeric antibody fragment thereof binds to a first target selected from the group comprising CD3, HER2, CD38, EGFR, CD20, OX40, CD19, CD47, IL1RAP, BMCA, NKp30 and CD16, to a second target different from the first target selected from the group comprising CD3, HER2, CD38, EGFR, CD20, OX40, CD19, CD47, IL1RAP, BMCA, NKp30 and CD16, and to a third target different from the first target or different from the first and the second target selected from the group comprising CD3, HER2, CD38, EGFR, CD20, OX40, CD19, CD47, IL1RAP, BMCA, NKp30 and CD16.

13. The diluent of any one of the preceding claims wherein said multispecific hetero-dimeric antibody or hetero-dimeric antibody fragment thereof binds to a first target selected from the group comprising CD3, HER2, CD38, EGFR, CD20, OX40, CD19, CD47, IL1RAP, BMCA, NKp30 and CD16, and to a second target different from the first target selected from the group comprising CD3, HER2, CD38, EGFR, CD20, OX40, CD19, CD47, IL1RAP, BMCA, NKp30 and CD16.

14. The diluent of any one of the preceding claims wherein said multispecific hetero-dimeric antibody or hetero-dimeric antibody fragment thereof is a bispecific hetero-dimeric antibody or hetero-dimeric antibody fragment thereof comprising the amino acid sequences of SEQ ID NOs: 1, 2 and 3, or the amino acid sequences of SEQ ID NOs: 4, 5 and 6 or comprises the amino acid sequences of SEQ ID NOs: 7, 8 and 9, or amino acid sequences with at least 80% homology to the amino acid sequences of SEQ ID NOs: 1 to 9.

15. A drug product ready for administration to a patient for use in treating cancer obtained by the steps comprising:
(i) Purifying a multispecific hetero-dimeric antibody or hetero-dimeric antibody fragment thereof to obtain a drug substance;
(ii) Add excipients to the drug substance to obtain a stable pharmaceutical formulation, wherein said stable pharmaceutical formulation can be liquid, or lyophilized or reconstituted;
(iii) Add to the stable pharmaceutical formulation the diluent of any one of claims 2 to 14;
wherein said drug product ready for administration is administered to a patient by infusion and said diluent is such that loss of antibody material in an infusion system, measured by ELISA, is less than 30%.

## Patentansprüche

1. Verdünnungsmittel zum Verdünnen eines Arzneimittelprodukts, wobei das Verdünnungsmittel einen Puffer und ein oder mehrere Stabilisierungs- oder Tonizitätsmittel umfasst, wobei das Arzneimittelprodukt einen multispezifischen hetero-dimeren Antikörper oder ein hetero-dimeres Antikörperfragment davon umfasst, umfassend eine erste und eine zweite manipulierte CH3-Domäne, und wobei nach dem Verdünnen des Arzneimittelprodukts mit dem Verdünnungsmittel ein Arzneimittelprodukt erhalten wird, das zur Verabreichung an einen Patienten durch Infusion bereit ist, **dadurch gekennzeichnet, dass** das Verdünnungsmittel einen pH-Wert zwischen 5,5 und 7,5 aufweist, der Puffer aus der Gruppe ausgewählt ist, umfassend Histidin, Tris, Citrat, Phosphat und Citratphosphat, und wobei der Puffer in dem Verdünnungsmittel in einer Konzentration von 30 mM oder weniger vorhanden ist, das Stabilisierungs- oder Tonizitätsmittel aus der Gruppe ausgewählt ist, umfassend Polyole wie Polysorbat 20, Polysorbat 40, Polysorbat 80, Aminosäuren wie Histidin, Arginin, Glycin, Methionin, Prolin, Lysin, Salze wie Natriumchlorid, und dass der Verlust von Antikörpermaterial in einem Infusionssystem, gemessen durch ELISA, weniger als 30 % beträgt.

2. Verdünnungsmittel nach einem der Ansprüche 1, wobei der Puffer aus der Gruppe ausgewählt ist, umfassend Histidin, Tris und Phosphat, die in dem Verdünnungsmittel in einer Konzentration von 25 mM vorhanden sind, und die Stabilisierungs- oder Tonizitätsmittel Arginin umfassen, das in dem Verdünnungsmittel in einer Konzentration von 100 mM vorhanden ist, und Polysorbat 80, das in dem Verdünnungsmittel in einer Konzentration von 0,05 % (Gew./Vol.) oder in einer Konzentration von 0,004 % (Gew./Vol.) vorhanden ist, und wobei das Verdünnungsmittel einen pH-Wert von 7 oder von 7,4 aufweist.

3. Verdünnungsmittel nach einem der Ansprüche 1 bis 2, wobei der Puffer Citrat ist, das in dem Verdünnungsmittel in einer Konzentration von 1,1 mM vorhanden ist, und die Stabilisierungs- oder Tonizitätsmittel Lysin-HCl, das in dem Verdünnungsmittel in einer Konzentration von 62 mM vorhanden ist, Natriumchlorid, das in dem Verdünnungsmittel in einer Konzentration von 154 mM vorhanden ist, und Polysorbat 80, das in dem Verdünnungsmittel in einer Konzentration von 0,004 % (Gew./Vol.) vorhanden ist, umfassen, und wobei das Verdünnungsmittel einen pH-Wert von 6,6 bis 7 aufweist.

4. Verdünnungsmittel nach einem der Ansprüche 1 bis 2, wobei das Verdünnungsmittel einen Puffer umfasst, der aus der Gruppe ausgewählt ist, umfassend Histidin, Phosphat, Citrat und Citrat-Phosphat, der in dem Verdünnungsmittel in einer Konzentration von 25 mM vorhanden ist, und die Stabilisierungs- oder Tonizitätsmittel Arginin-HCl oder Lysin-HCl umfassen, die in dem Verdünnungsmittel in einer Konzentration von 500 mM vorhanden sind, und Polysorbat 80, das in dem Verdünnungsmittel in einer Konzentration von 0,04 % (Gew./Vol.) vorhanden ist, und wobei das Verdünnungsmittel einen pH-Wert aufweist, der aus der Gruppe ausgewählt ist, umfassend 6, 6,5 und 7.

5. Verdünnungsmittel nach Anspruch 4, wobei der Puffer aus der Gruppe ausgewählt ist, umfassend Phosphat, Citrat und Citrat-Phosphat, die in dem Verdünnungsmittel in einer Konzentration von 25 mM vorhanden sind, und die Stabilisierungs- oder Tonizitätsmittel Arginin-HCl oder Lysin-HCl umfassen, die in dem Verdünnungsmittel in einer Konzentration von 500 mM vorhanden sind, und Polysorbat 80, das in dem Verdünnungsmittel in einer Konzentration von 0,04 % (Gew./Vol.) vorhanden ist, und wobei das Verdünnungsmittel einen pH-Wert von 6,5 oder 7 aufweist und bei 5 °C und/oder 25 °C und/oder bei 40 °C für mindestens 3 Monate stabil ist.

6. Verdünnungsmittel nach Anspruch 4, wobei der Puffer Phosphat oder Citrat-Phosphat ist, das in dem Verdünnungsmittel in einer Konzentration von 25 mM vorhanden ist, und die Stabilisierungs- oder Tonizitätsmittel Arginin-HCl oder Lysin-HCl umfassen, die in dem Verdünnungsmittel in einer Konzentration von 500 mM vorhanden sind, und Polysorbat 80, das in dem Verdünnungsmittel in einer Konzentration von 0,04 % (Gew./Vol.) vorhanden ist, und wobei das Verdünnungsmittel einen pH-Wert von 6 oder von 6,5 aufweist und bei 5 °C für mindestens 29 Monate, bei 25 °C für mindestens 12 Monate und bei 40 °C für mindestens 3 Monate stabil ist.

7. Verdünnungsmittel nach Anspruch 6, wobei der Puffer Phosphat ist, das in dem Verdünnungsmittel in einer Konzentration von 25 mM vorhanden ist, die Stabilisierungs- oder Tonizitätsmittel Arginin-HCl sind, das in dem Verdünnungsmittel in einer Konzentration von 500 mM vorhanden ist, und Polysorbat 80, das in dem Verdünnungsmittel in einer Konzentration von 0,04 % (Gew./Vol.) vorhanden ist, und wobei das Verdünnungsmittel einen pH-Wert von 6 aufweist und es bei 5 °C für mindestens 29 Monate, bei 25 °C für mindestens 12 Monate und bei 40 °C für mindestens 3 Monate stabil ist.

8. Verdünnungsmittel nach einem der vorstehenden Ansprüche, wobei eine isotonische Lösung, ausgewählt aus der Gruppe, umfassend Kochsalzlösung, Dextroselösung und Kombination aus Kochsalz- und Dextroselösung, mit dem Verdünnungsmittel in einem Verhältnis von 1:10 gemischt wird.

9. Verdünnungsmittel nach einem der vorstehenden Ansprüche, wobei der multispezifische hetero-dimere Antikörper oder das hetero-dimere Antikörperfragment davon in dem zur Verabreichung bereiten Arzneimittel in einer Konzentration zwischen 5 ng/ml und 20 ng/ml vorhanden ist.

10. Verdünnungsmittel nach Anspruch 9, wobei der multispezifische hetero-dimere Antikörper oder das hetero-dimere Antikörperfragment davon in dem zur Verabreichung bereiten Arzneimittel in einer Konzentration von 10 ng/ml vorhanden ist.

11. Verdünnungsmittel nach Anspruch 1, wobei die erste manipulierte CH3-Domäne die Substitutionen aus der Gruppe umfasst, bestehend aus: S20K, T22V, K26T, K79Y, F85.1S, Y86V, K88W, T90N, und die zweite manipulierte CH3-Domäne umfasst die Substitutionen aus der Gruppe, bestehend aus: Q3E, Y5A, L7F, S20T, T22V, K26T, T81D, V84L, D84.2E, F85.1A, Y86S, K88R, T90R, und wobei das multispezifische hetero-dimere Immunglobulin oder hetero-dimere Fragment durch die erste und zweite manipulierte CH3-Domäne heterodimerisieren.

12. Verdünnungsmittel nach einem der vorstehenden Ansprüche, wobei der multispezifische hetero-dimere Antikörper oder das hetero-dimere Antikörperfragment davon an ein erstes Ziel bindet, das aus der Gruppe ausgewählt ist, umfassend CD3, HER2, CD38, EGFR, CD20, OX40, CD19, CD47, IL1RAP, BMCA, NKp30 und CD16, an ein zweites Ziel, das von dem ersten Ziel unterschiedlich ist und aus der Gruppe ausgewählt ist, umfassend CD3, HER2, CD38, EGFR, CD20, OX40, CD19, CD47, IL1RAP, BMCA, NKp30 und CD16, und zu einem dritten Ziel, das sich von dem ersten Ziel unterscheidet oder sich von dem ersten und dem zweiten Ziel unterscheidet, ausgewählt aus der Gruppe, umfassend CD3, HER2, CD38, EGFR, CD20, OX40, CD19, CD47, IL1RAP, BMCA, NKp30 und CD16.

13. Verdünnungsmittel nach einem der vorstehenden Ansprüche, wobei der multispezifische hetero-dimere Antikörper oder das hetero-dimere Antikörperfragment davon an ein erstes Ziel bindet, das aus der Gruppe ausgewählt ist, umfassend CD3, HER2, CD38, EGFR, CD20, OX40, CD19, CD47, IL1RAP, BMCA, NKp30 und CD16, und an ein zweites Ziel, das sich von dem ersten Ziel unterscheidet und aus der Gruppe ausgewählt ist, umfassend CD3, HER2, CD38, EGFR, CD20, OX40, CD19, CD47, IL1RAP, BMCA, NKp30 und CD16.

14. Verdünnungsmittel nach einem der vorstehenden Ansprüche, wobei der multispezifische hetero-dimere Antikörper oder das hetero-dimere Antikörperfragment davon ein bispezifischer hetero-dimerer Antikörper oder ein hetero-dimeres Antikörperfragment davon ist, umfassend die Aminosäuresequenzen von SEQ ID NO: 1, 2 und 3, oder die Aminosäuresequenzen von SEQ ID NO: 4, 5 und 6, oder die Aminosäuresequenzen von SEQ ID NO: 7, 8 und 9, oder Aminosäuresequenzen mit mindestens 80 % Homologie zu den Aminosäuresequenzen von SEQ ID NO: 1 bis 9.

15. Arzneimittelprodukt, das zur Verabreichung an einen Patienten zur Verwendung bei der Behandlung von Krebs geeignet ist, erhalten durch die folgenden Schritte, umfassend:
(i) Reinigen eines multispezifischen hetero-dimeren Antikörpers oder eines hetero-dimeren Antikörperfragments davon, um eine Arzneimittelsubstanz zu erhalten;
(ii) Hinzufügen von Hilfsstoffen zu der Arzneimittelsubstanz, um eine stabile pharmazeutische Formulierung zu erhalten, wobei die stabile pharmazeutische Formulierung flüssig, lyophilisiert oder rekonstituiert sein kann;
(iii) Hinzufügen des Verdünnungsmittels nach einem der Ansprüche 2 bis 14 zu der stabilen pharmazeutischen Formulierung;
wobei das Arzneimittel, das zur Verabreichung bereit ist, einem Patienten durch Infusion verabreicht wird und das Verdünnungsmittel so beschaffen ist, dass der Verlust an Antikörpermaterial in einem Infusionssystem, gemessen durch ELISA, weniger als 30 % beträgt.

## Revendications

1. Diluant permettant de diluer un produit médicamenteux, dans lequel ledit diluant comprend un tampon et un ou plusieurs agents de stabilisation ou de tonicité, dans lequel ledit produit médicamenteux comprend un anticorps hétérodimérique multispécifique ou un fragment d'anticorps hétérodimérique de celui-ci comprenant un premier et un second domaine CH3 modifié, et dans lequel, lors de la dilution dudit produit médicamenteux avec ledit diluant, un produit médicamenteux prêt à être administré à un patient par perfusion est obtenu, **caractérisé en ce que** ledit diluant a un pH compris entre 5,5 et 7,5, ledit tampon est choisi dans le groupe comprenant l'histidine, le tris, le citrate, le phosphate et le citrate-phosphate, et dans lequel ledit tampon est présent dans ledit diluant à une concentration égale ou inférieure à 30 mM, ledit agent de stabilisation ou de tonicité est choisi dans le groupe comprenant des polyols tels que le polysorbate 20, le polysorbate 40, le polysorbate 80, des acides aminés tels que l'histidine, l'arginine, la glycine, la méthionine, la proline, la lysine, des sels tels que le chlorure de sodium, et **en ce que** la perte de matériau d'anticorps dans un système de perfusion, mesurée par ELISA, est inférieure à 30 %.

2. Diluant selon l'une quelconque de la revendication 1, dans lequel ledit tampon est choisi dans le groupe comprenant l'histidine, le tris et le phosphate, présents dans ledit diluant à une concentration de 25 mM, et lesdits agents de stabilisation ou de tonicité comprennent l'arginine, présente dans ledit diluant à une concentration de 100 mM, et le polysorbate 80, présent dans ledit diluant à une concentration de 0,05 % (p/v) ou à une concentration de 0,004 % (p/v), et dans lequel ledit diluant a un pH de 7 ou de 7,4.

3. Diluant selon l'une quelconque des revendications 1 à 2, dans lequel ledit tampon est le citrate, présent dans ledit diluant à une concentration de 1,1 mM, et lesdits agents de stabilisation ou de tonicité comprennent la lysine-HCl, présente dans ledit diluant à une concentration de 62 mM, le chlorure de sodium, présent dans ledit diluant à une concentration de 154 mM, et le polysorbate 80, présent dans ledit diluant à une concentration de 0,004 % (p/v), et dans lequel ledit diluant a un pH allant de 6,6 à 7.

4. Diluant selon l'une quelconque des revendications 1 à 2, dans lequel ledit diluant comprend un tampon choisi dans le groupe comprenant l'histidine, le phosphate, le citrate et le citrate-phosphate, présents dans ledit diluant à une concentration de 25 mM, et lesdits agents de stabilisation ou de tonicité comprennent l'arginine-HCl ou la lysine-HCl, présente dans ledit diluant à une concentration de 500 mM, et le polysorbate 80, présent dans ledit diluant à une concentration de 0,04 % (p/v), et dans lequel ledit diluant a un pH choisi dans le groupe comprenant 6, 6,5 et 7.

5. Diluant selon la revendication 4, dans lequel ledit tampon est choisi dans le groupe comprenant le phosphate, le citrate et le citrate-phosphate, présents dans ledit diluant à une concentration de 25 mM, et lesdits agents de stabilisation ou de tonicité comprennent l'arginine-HCl ou la lysine-HCl, présentes dans ledit diluant à une concentration de 500 mM, et le polysorbate 80, présent dans ledit diluant à une concentration de 0,04 % (p/v), et dans lequel ledit diluant a un pH de 6,5 ou de 7 et est stable à 5 °C et/ou 25 °C et/ou 40 °C pendant au moins 3 mois.

6. Diluant selon la revendication 4, dans lequel ledit tampon est le phosphate ou le citrate-phosphate, présent dans ledit diluant à une concentration de 25 mM, et lesdits agents de stabilisation ou de tonicité comprennent l'arginine-HCl ou la lysine-HCl, présentes dans ledit diluant à une concentration de 500 mM, et le polysorbate 80, présent dans ledit diluant à une concentration de 0,04 % (p/v), et dans lequel ledit diluant a un pH de 6 ou de 6,5 et est stable à 5 °C pendant au moins 29 mois, à 25 °C pendant au moins 12 mois, et à 40 °C pendant au moins 3 mois.

7. Diluant selon la revendication 6, dans lequel ledit tampon est le phosphate, présent dans ledit diluant à une concentration de 25 mM, lesdits agents de stabilisation ou de tonicité sont l'arginine-HCl, présente dans ledit diluant à une concentration de 500 mM, et le polysorbate 80, présent dans ledit diluant à une concentration de 0,04 % (p/v), et dans lequel ledit diluant a un pH de 6 et est stable à 5 °C pendant au moins 29 mois, à 25 °C pendant au moins 12 mois, et à 40 °C pendant au moins 3 mois.

8. Diluant selon l'une quelconque des revendications précédentes, dans lequel une solution isotonique choisie dans le groupe comprenant une solution saline, une solution de dextrose, et une combinaison de solution saline et de dextrose est mélangée avec ledit diluant dans un rapport de 1:10.

9. Diluant selon l'une quelconque des revendications précédentes, dans lequel ledit anticorps hétérodimérique multispécifique ou fragment d'anticorps hétérodimérique de celui-ci est présent dans ledit produit médicamenteux prêt à être administré à une concentration comprise entre 5 ng/mL et 20 ng/mL.

10. Diluant selon la revendication 9, dans lequel ledit anticorps hétérodimérique multispécifique ou fragment d'anticorps hétérodimérique de celui-ci est présent dans ledit produit médicamenteux prêt à être administré à une concentration de 10 ng/ml.

11. Diluant selon la revendication 1, dans lequel ledit premier domaine CH3 modifié comprend les substitutions du groupe constitué de : S20K, T22V, K26T, K79Y, F85.1S, Y86V, K88W, T90N, et ledit second domaine CH3 modifié comprend les substitutions du groupe constitué de : Q3E, Y5A, L7F, S20T, T22V, K26T, T81D, V84L, D84.2E, F85.1A, Y86S, K88R, T90R, et dans lequel ladite immunoglobuline hétérodimérique multispécifique ou ledit fragment hétérodimérique s'hétérodimérisent par l'intermédiaire desdits premier et second domaines CH3 modifiés.

12. Diluant selon l'une quelconque des revendications précédentes, dans lequel ledit anticorps hétérodimérique multispécifique ou fragment d'anticorps hétérodimérique de celui-ci se lie à une première cible choisie dans le groupe comprenant CD3, HER2, CD38, EGFR, CD20, OX40, CD19, CD47, IL1RAP, BMCA, NKp30 et CD16, à une deuxième cible différente de la première cible choisie dans le groupe comprenant CD3, HER2, CD38, EGFR, CD20, OX40, CD19, CD47, IL1RAP, BMCA, NKp30 et CD16, et à une troisième cible différente de la première cible ou différente de la première et de la deuxième cible choisie dans le groupe comprenant CD3, HER2, CD38, EGFR, CD20, OX40, CD19, CD47, IL1RAP, BMCA, NKp30 et CD16.

13. Diluant selon l'une quelconque des revendications précédentes, dans lequel ledit anticorps hétérodimérique multispécifique ou fragment d'anticorps hétérodimérique de celui-ci se lie à une première cible choisie dans le groupe comprenant CD3, HER2, CD38, EGFR, CD20, OX40, CD19, CD47, IL1RAP, BMCA, NKp30 et CD16, et à une deuxième cible différente de la première cible choisie dans le groupe comprenant CD3, HER2, CD38, EGFR, CD20, OX40, CD19, CD47, IL1RAP, BMCA, NKp30 et CD16.

14. Diluant selon l'une quelconque des revendications précédentes, dans lequel ledit anticorps hétérodimérique multispécifique ou fragment d'anticorps hétérodimérique de celui-ci est un anticorps hétérodimérique bispécifique ou un fragment d'anticorps hétérodimérique de celui-ci comprenant les séquences d'acides aminés SEQ ID NO : 1, 2 et 3, ou les séquences d'acides aminés SEQ ID NO : 4, 5 et 6 ou comprend les séquences d'acides aminés SEQ ID NO : 7, 8 et 9, ou des séquences d'acides aminés présentant une homologie d'au moins 80 % avec les séquences d'acides aminés SEQ ID NO : 1 à 9.

15. Produit médicamenteux prêt à être administré à un patient pour utilisation dans le traitement du cancer, obtenu par les étapes comprenant :
(i) la purification d'un anticorps hétérodimérique multispécifique ou fragment d'anticorps hétérodimérique de celui-ci afin d'obtenir une substance médicamenteuse ;
(ii) l'ajout d'excipients à la substance médicamenteuse afin d'obtenir une formulation pharmaceutique stable, dans lequel ladite formulation pharmaceutique stable peut être liquide, ou lyophilisée ou reconstituée ;
(iii) l'ajout à la formulation pharmaceutique stable du diluant selon l'une quelconque des revendications 2 à 14 ;
dans lequel ledit produit médicamenteux prêt à être administré est administré à un patient par perfusion et ledit diluant est tel que la perte de matériau d'anticorps dans un système de perfusion, mesurée par ELISA, est inférieure à 30 %.
